# EUROPEAN PATENT SPECIFICATION

(11) **EP 3 369 427 B1**
(45) Date of publication and mention of the grant of the patent: **13.10.2021**
(21) Application number: 18157575.4
(22) Date of filing: 24.12.2008
(51) Int. Cl.: A61P 33/10, A61P 43/00, A61K 36/00, A61K 36/53, A61K 36/63, A61P 33/00

(54) **SYNERGISTIC ANTIPARASITIC COMPOSITIONS AND SCREENING METHODS**
SYNERGISTISCHE ANTIPARASITÄRE ZUSAMMENSETZUNGEN UND SCREENING-VERFAHREN
COMPOSITIONS ANTIPARASITAIRES SYNERGIQUES ET PROCÉDÉS DE CRIBLAGE

(30) Priority: 27.12.2007 US 17145 P; 27.12.2007 US 17157 P; 03.01.2008 US 18879 P
(43) Date of publication of application: 05.09.2018
(62) Divisional of application: 08866562.5
(73) Proprietor: TyraTech, Inc., Melbourne, FL 32901 (US)
(72) Inventor: ENAN, Essam, Davis, CA 95618 (US)
(74) Representative: Høiberg P/S

(56) References cited:
- WO-A-2004/100971
- WO-A1-89/12626
- WO-A2-2008/003007
- KUROWSKA A ET AL: "[Analysis of essential oils and their insecticidal properties. IV. Garden thyme (Thymus vulgaris L.)]", PESTYCYDY = PESTICIDES : QUARTERLY OF THE INSTITUTE OF INDUSTRIAL ORGANIC CHEMISTRY, INSTYTUT PRZEMYS?U ORGANICZNEGO <WARSCHAU>, no. 2, 1 January 1991 (1991-01-01), pages 25-29, XP009506686, ISSN: 0208-8703
- BOOKWALTER G N ET AL: "Tricalcium Phosphate-Soybean Oil in Fortified Processed Cereals to Suppress insects, Dusting, and Separation", JOURNAL OF FOOD SCIENCE,, vol. 50, no. 1, 1 January 1985 (1985-01-01), pages 245-248, XP001350134,

## Description

### FIELD OF THE INVENTION

The presently-disclosed subject matter relates to antiparasitic compositions for use in treating specific parasitic infections.

### BACKGROUND

Parasitic infections of plants, humans, and other animals pose a worldwide problem. For example, more than 650 million people are at risk for gastrointestinal parasitic infection, and about 200 million are actually infected. Various conditions contribute to the development and spread of parasitic infections, including poor sanitary conditions; low host resistance; population expansion; and inadequate control of vectors and infection reservoirs.

Such parasitic infections present an abundance of medical and social problems. For example, parasitic infection can undermine child development, educational achievement, reproductive health, and social and economic development. Indeed, some parasitic infections can cause morbidity and mortality. Notwithstanding the severe impact that parasitic infections can have, relatively few treatment options are available.

Available treatments are limited, and treatments for some parasitic infections are non-existent. In the 1960s, niclosamide (also known as yomesan) was identified for use in treating certain helminthic parasitic infections; however, niclosamide has certain drawbacks. For example, in many cases a single dose of niclosamide does not provide a curative effect, rather, a relapse ensues because the compound has difficulty accessing cysticercoids buried deeply within the mucosal villi. As such, satisfactory results require an extended treatment with niclosamide for approximately 7 days. *See* Davis, Drug treatment of intestinal helminthiasis, World Health Organization (WHO), Geneva, 1973.

Another drug that has been used to treat helminthic parasitic infections is Praziquantel (2-(cyclohexylcarbonyl)-1,2,3,6,7,11b-hexahydro-4H-pyrazino(2,1-a)isoquinolin-4-one; also known as Biltracide). *See* Pearson and Gurrant, Praziquantel: a major advance in anthelminthic therapy. Annals of Internal Medicine, 99:195-198, 1983. Praziquantel can be administered in a single dose; however, treatment strategies making use of Praziquantel are at risk because of the possibility of the development of resistance to Praziquantel. Accordingly, there remains a need in the art for non-harmful compositions that are effective for treating parasitic infections. WO 2004/100971 discloses compositions comprising one or more plant essential oils and an insect control agent for controlling insects.

### SUMMARY

The presently-disclosed subject matter meets some or all of the above-identified needs, as will become evident to those of ordinary skill in the art after a study of the information provided in this document. The references to methods of treatment in the subsequent paragraphs of this description are to be interpreted as references to the antiparasitic compositions of the present invention for use in a method for treatment of the human (or animal) body by therapy.

This Summary describes several embodiments of the presently-disclosed subject matter, and in many cases lists variations and permutations of these embodiments. This Summary is merely exemplary of the numerous and varied embodiments. Disclosure of one or more representative features of a given embodiment is likewise exemplary. Such an embodiment can typically exist with or without the feature(s) mentioned; likewise, those features can be applied to other embodiments of the presently-disclosed subject matter, whether listed in this Summary or not. To avoid excessive repetition, this Summary does not list or suggest all possible combinations of such features.

The presently-disclosed subject matter includes compositions and methods for treating parasitic infections, and methods of screening for and selecting compositions useful for treating a parasitic infection.

In some embodiments of the present disclosure, the parasitic infections are caused by parasites classified as endoparasites, ectoparasites, human parasites, animal parasites, or agricultural parasites.

In some embodiments of the present disclosure, the composition for treating a parasitic infection in a subject includes two or more compounds selected from: trans-anethole, para-cymene, linalool, α-pinene, and thymol.

In some embodiments of the present disclosure, the composition includes two more compounds selected from: para-cymene, linalool, α-pinene, and thymol. In some embodiments of the present disclosure, the composition includes three or more compounds selected from: para-cymene, linalool, α-pinene, and thymol. In some embodiments of the present disclosure, the composition includes para-cymene, linalool, α-pinene, and thymol. In some embodiments of the present disclosure, the composition further includes soy bean oil.

In some embodiments of the present disclosure, the composition includes 25-35 by weight para-cymene, 1-10% by weight linalool, 1-10% by weight α-pinene, 35-45% by weight thymol, and 20-30% by weight soy bean oil. In some embodiments of the present disclosure, the composition includes 28.39% by weight para-cymene, 6.6% by weight linalool, 3.8% by weight α-pinene, 37.2% by weight thymol, and 24% by weight soy bean oil.

In some embodiments of the present disclosure, the composition includes 25-35% by volume para-cymene, 1-10% by volume linalool, 1-10% by volume α-pinene, 35-45% by volume thymol and 20-30% by volume soy bean oil. In some embodiments of the present disclosure, the composition includes 30% by volume para-cymene, 7% by volume linalool, 4%> by volume α-pinene, 35% by volume thymol, and 24% by volume soy bean oil.

In some embodiments of the present disclosure, the composition includes three or more compounds selected from: trans-anethole, para-cymene, linalool, α-pinene, and thymol. In some embodiments of the present disclosure, the composition includes four or more compounds selected from: trans-anethole, para-cymene, linalool, α-pinene, and thymol. In some embodiments of the present disclosure, the composition includes trans-anethole, para-cymene, linalool, α-pinene, and thymol.

In some embodiments of the present disclosure, the composition includes 15-25% by weight trans-anethole, 30-40% by weight para-cymene, 1-10% by weight linalool, 1-10% by weight α-pinene, and 35-45% by weight thymol. In some embodiments of the present disclosure, the composition includes 18.2% by weight trans-anethole, 34.4% by weight para-cymene, 4.7% by weight linalool, 1.9% by weight α-pinene, and 40.8% by weight thymol.

In some embodiments of the present disclosure, the composition includes 10-20% by volume trans-anethole, 30-40% by volume para-cymene, 1-10% by volume linalool, 1-10% by volume α-pinene, and 35-45% by volume thymol. In some embodiments of the present disclosure, the composition includes 17% by volume trans-anethole, 37% by volume para-cymene, 5% by volume linalool, 2% by volume α-pinene, and 39% by volume thymol.

In some embodiments of the present disclosure, the composition includes 15-25% by weight trans-anethole, 1-10% by weight para-cymene, 35-45% by weight linalool, 1-10% by weight α-pinene, and 30-40% by weight thymol. In some embodiments of the present disclosure, the composition includes 18.2% by weight trans-anethole, 1.9% by weight para-cymene, 40.8% by weight linalool, 4.7% by weight α-pinene, and 34.4% by weight thymol.

In some embodiments of the present disclosure, the composition includes 15-25% by volume trans-anethole, 1-10% by volume para-cymene, 35-45% by volume linalool, 1-10% by volume α-pinene, and 30-40% by volume thymol. In some embodiments of the present disclosure, the composition includes 17% by volume trans-anethole, 2% by volume para-cymene, 39% by volume linalool, 5% by volume α-pinene, and 37% by volume thymol.

In some embodiments of the present disclosure, the compounds of the composition together demonstrate a synergistic anti-parasitic effect. In some embodiments of the present disclosure, the actual percent effect of the composition is greater than the expected percent effect of the composition. In some embodiments of the present disclosure the coefficient of synergy relative to a component of the composition is greater than 5, 10, 25, 50, 75, or 100.

In some embodiments of the present disclosure, the parasitic infection is by a protozoan parasite. In some embodiments of the present disclosure, the parasite is selected from intestinal protozoa, tissue protozoa, and blood protozoa. In some embodiments of the present disclosure, the parasite is selected from: *Entamoeba hystolytica, Giardia lamblia, Cryptosporidium muris, Cryptosporidium parvum, Trypanosomatida gambiense, Trypanosomatida rhodesiense, Trypanosomatida crusi, Leishmania mexicana, Leishmania braziliensis, Leishmania tropica, Leishmania donovani, Toxoplasma gondii, Plasmodium vivax, Plasmodium ovale, Plasmodium malariae, Plasmodium falciparum, Trichomonas vaginalis, and Histomonas meleagridis.*

In some embodiments of the present disclosure, the parasitic infection is by a helminthic parasite. In some embodiments of the present disclosure, the parasite is selected from nematodes. In some embodiments of the present disclosure, the parasite is selected from *Adenophorea.* In some embodiments of the present disclosure, the parasite is selected from *Secementea.* In some embodiments of the present disclosure, the parasite is selected from: *Trichuris trichiura, Ascaris lumbricoides, Enterobius vermicularis, Ancylostoma duodenale, Necator americanus, Strongyloides stercoralis, Wuchereria bancrofti, Dracunculus medinensis.* In some embodiments of the present disclosure, the parasite is selected from trematodes. In some embodiments of the present disclosure, the parasite is selected from: blood flukes, liver flukes, intestinal flukes, and lung flukes. In some embodiments of the present disclosure, the parasite is selected from: *Schistosoma mansoni, Schistosoma haematobium, Schistosoma japonicum, Fasciola hepatica, Fasciola gigantica, Heterophyes heterophyes, Paragonimus westermani,* and *Opishorchis sinensis.*

In some embodiments of the present disclosure, the parasite is selected from cestodes. In some embodiments of the present disclosure, the parasite is selected from *Taenia solium, Taenia saginata, Hymenolepis nana, Echinococcus granulosus,* and *Diplyidium caninum.*

In some embodiments of the present disclosure, the composition is provided in a formulation. The formulation can include the composition and a carrier, such as a food product. In some embodiments of the present disclosure the formulation includes the composition encapsulated or microencapsulated with an outer shell material.

The presently-disclosed subject matter includes a method of treating a parasitic infection in a subject. In some embodiments of the present disclosure, the method includes administering to the subject an effective amount of a composition as described herein.

The presently-disclosed subject matter includes a method for selecting a composition for use in treating a parasitic infection. In some embodiments of the present disclosure, the method includes: providing a cell expressing a tyramine receptor; contacting test compounds to the cell; measuring the receptor binding affinity of the compounds; measuring at least one parameter selected from, (i) intracellular cAMP level, and (ii) intracellular Ca²⁺ level; identifying a first compound for the composition that is capable of altering at least one of said parameters, and which has a high receptor binding affinity for the tyramine receptor; identifying a second compound for the composition that is capable of altering at least one of said parameters, and which has a low receptor binding affinity for the tyramine receptor; and selecting a composition including the first and second compounds. In some embodiments of the present disclosure, the selected composition demonstrates an anti-parasitic effect that exceeds the anti-parasitic effect of any of the compounds when used alone.

An embodiment of the present disclosure provides an antiparasitic composition, comprising a synergistic combination of two or more compounds from a blend listed in Table E.

An embodiment of the present disclosure provides an antiparasitic composition, comprising a synergistic combination of three or more compounds from a blend listed in Table E.

An embodiment of the present disclosure provides an antiparasitic composition, comprising a synergistic combination of four or more compounds from a blend listed in Table E.

An embodiment of the present disclosure provides an antiparasitic composition, comprising a synergistic combination of all compounds from a blend listed in Table E.

An embodiment of the present disclosure provides an antiparasitic composition wherein the amount of each compound is within a range obtained by multiplying the amount in Table E by Factor 1.

An embodiment of the present disclosure provides an antiparasitic composition, wherein the amount of each compound is within a range obtained by multiplying the amount in Table E by Factor 2.

An embodiment of the present disclosure provides an antiparasitic composition, wherein the amount of each compound is within a range obtained by multiplying the amount in Table E by Factor 3.

An embodiment of the present disclosure provides an antiparasitic composition, wherein the amount of each compound is within a range obtained by multiplying the amount in Table E by Factor 4.

An embodiment of the present disclosure provides an antiparasitic composition, wherein each compound is present in the amount stated in Table E.

An embodiment of the present disclosure provides an antiparasitic composition, wherein a coefficient of synergy relative to a component of the composition is greater than 5, 10, 25, 50, 75, or 100.

An embodiment of the present disclosure provides an antiparasitic composition, wherein the composition exhibits synergistic effects on a parasite selected from the group consisting of: a protozoan parasite, a helminthic parasite, a pest of the subclass Acari, a louse, a flea, or a fly.

An embodiment of the present disclosure provides an antiparasitic composition, wherein the composition exhibits synergistic effects on a parasite having a host selected from the group consisting of: canola, cat, dog, goat, horse, man, maize, mouse, ox, pig, poultry, rabbit, rice, sheep, soybean, tobacco, and wheat.

An embodiment of the present disclosure provides any of the above antiparasitic compositions, additionally comprising an ingredient selected from the group consisting of a surfactant and a fixed oil.

An embodiment of the present disclosure provides an antiparasitic composition, comprising a synergistic combination of two or more compounds listed in any of Tables B, B1, C, D, or E.

An embodiment of the present disclosure provides a formulation comprising the composition of any of the above antiparasitic compositions and a carrier.

An embodiment of the present disclosure provides the above formulation, wherein the carrier is a food product.

An embodiment of the present disclosure provides any of the above antiparasitic compositions as a medicament for the treatment or prevention of parasitic disease or infestation.

An embodiment of the present disclosure relates to the any of the above antiparasitic compositions as an antiparasitic agent for the treatment or prevention of parasitic disease or infestation.

An embodiment of the present disclosure relates to a method of treating a parasitic infection in a subject, comprising administering an effective amount of any of the above antiparasitic compositions to the subject.

An embodiment of the present disclosure relates to the above method, where the parasitic infection is caused by a parasite in a classification selected from the group consisting of endoparasites, ectoparasites, human parasites, animal parasites, or agricultural parasites.

An embodiment of the present disclosure relates to a method of selecting a composition for use in treating a parasitic infection, comprising: providing a cell expressing a receptor selected from the group consisting of a tyramine receptor and a receptor of the olfactory cascade; contacting test compounds to the cell; measuring the receptor binding affinity of the compounds; measuring at least one parameter selected from (i) intracellular cAMP level; and (ii) intracellular Ca²+ level; identifying a first compound for the composition that is capable of altering at least one of said parameters, and which has a high receptor binding affinity for the receptor; and identifying a second compound for the composition that is capable of altering at least one of said parameters, and which has a low receptor binding affinity for the receptor; and selecting a composition including the first and second compounds.

An embodiment of the present disclosure relates to a method of selecting a composition for use in treating a parasitic infection, comprising: providing a cell expressing a receptor selected from the group consisting of the receptors listed in Table F; contacting test compounds to the cell; measuring the receptor binding affinity of the compounds; measuring at least one parameter selected from (i) intracellular cAMP level; and (ii) intracellular Ca²+ level; identifying a first compound for the composition that is capable of altering at least one of said parameters, and which has a high receptor binding affinity for the receptor; and identifying a second compound for the composition that is capable of altering at least one of said parameters, and which has a low receptor binding affinity for the receptor; and selecting a composition including the first and second compounds.

An embodiment of the present disclosure relates to a method of selecting a composition for use in treating a parasitic infection, comprising: providing a cell comprising a molecular target selected from the group consisting of the molecular targets listed in Table G; contacting test compounds to the cell; measuring the binding affinity of the compounds for the molecular target; measuring at least one parameter selected from (i) intracellular cAMP level; and (ii) intracellular Ca²+ level; identifying a first compound for the composition that is capable of altering at least one of said parameters, and which has a high binding affinity for the molecular target; and identifying a second compound for the composition that is capable of altering at least one of said parameters, and which has a low binding affinity for the molecular target; and selecting a composition including the first and second compounds.

### BRIEF DESCRIPTION OF THE DRAWINGS

Figure 1 is a bar graph demonstrating cure rates of animals infected with *H*. *nana* and treated with compounds disclosed herein.
Figure 2 is a series of line graphs demonstrating effective killing of *S. mansoni in vitro* by differing concentrations of compounds disclosed herein. LT100 = lethal time required to induce 100% mortality among treated worms, ppm = mg (weight) in 1 L (volume). For example 100 ppm equal 100 mg (weight) in 1L (volume) saline.
Figure 3 is a bar graph demonstrating effective killing of *S. mansoni in vitro* by 100 ppm concentration of compounds disclosed herein, either alone or in combination with one another. LT100 = lethal time required to induce 100% mortality among treated worms.
Figure 4 is a series of line graphs demonstrating effective killing of *H*. *meleagridis in vitro* by differing concentrations of compounds disclosed herein.
Figure 5 is a series of line graphs demonstrating effective killing of *H*. *meleagridis in vitro* by differing concentrations of compounds disclosed herein.
Figures 6-14 show photographs and graphs depicting examples related to testing performed on *T. spiralis* and *A. lumbricoides.*

### DETAILED DESCRIPTION

The invention is defined by the appended claims. Any embodiment not falling under the scope of the appended claims does not form part of the invention.

The presently-disclosed subject matter includes compositions and methods for treating parasitic infections, and methods of screening for and selecting compositions useful for treating a parasitic infection.

As used herein, the term "parasitic infection" refers to the infection of a plant or animal host by a parasite, such as a successful invasion of a host by an endoparasite, including for example a protozoan parasite or a helminthic parasite.

As used herein, the term "parasite" includes parasites, such as but not limited to, protozoa, including intestinal protozoa, tissue protozoa, and blood protozoa. Examples of intestinal protozoa include, but are not limited to: *Entamoeba hystolytica, Giardia lamblia, Cryptosporidium muris,* and *Cryptosporidium parvum.* Examples of tissue protozoa include, but are not limited to: *Trypanosomatida gambiense, Trypanosomatida rhodesiense, Trypanosomatida crusi, Leishmania mexicana, Leishmania braziliensis, Leishmania tropica, Leishmania donovani, Toxoplasma gondii,* and *Trichomonas vaginalis.* Examples of blood protozoa include, but are not limited to *Plasmodium vivax, Plasmodium ovale, Plasmodium malariae,* and *Plasmodium falciparum. Histomonas meleagridis* is yet another example of a protozoan parasite.

As used herein, the term "parasite" further includes, but is not limited to: helminthes or parasitic worms, including nematodes (round worms) and platyhelminthes (flat worms). Examples of nematodes include, but are not limited to: animal and plant nematodes of the adenophorea class, such as the intestinal nematode *Trichuris trichiura* (whipworm) and the plant nematode *Trichodorus obtusus* (stubby-root nematode); intestinal nematodes of the secementea class, such as *Ascaris lumbricoides, Enterobius vermicularis* (pinworm), *Ancylostoma duodenale* (hookworm), *Necator americanus* (hookworm), and *Strongyloides stercoralis;* and tissue nematodes of the *secementea* class, such as *Wuchereria bancrofti* (*Filaria bancrofti*) and *Dracunculus medinensis* (Guinea worm). Examples of plathyeminthes include, but are not limited to: Trematodes (flukes), including blood flukes, such as *Schistosoma mansoni* (intestinal Schistosomiasis), *Schistosoma haematobium,* and *Schistosoma japonicum;* liver flukes, such as *Fasciola hepatica,* and *Fasciola gigantica;* intestinal flukes, such as *Heterophyes heterophyes;* and lung flukes such as *Paragonimus westermani.* Examples of platheminthes further include, but are not limited to: Cestodes (tapeworms), including *Taenia solium, Taenia saginata, Hymenolepis nana,* and *Echinococcus granulosus.*

Furthermore, the term "parasite" further includes, but is not limited to those organisms and classes of organisms listed in the following Table A:

**Protozoa (sub-groups: rhizopods, flagellates, ciliate, sporozoans)**

| Parasite (*Genus*) | (*Species*) | Context |
|---|---|---|
| *Entamoeba* | *coli* *dispar* *histolytica* *gingivalis* | Example of gut rhizopod that can switch from commensal to parasite depending on circumstances. Several species are found in humans. *E. histolytica* is the pathogen responsible for amoebiasis (which includes amoebic dysentery and amoebic liver abscesses). |
| *Balantidium* | *coli* | Example of parasitic ciliate and zoonosis |
| *Giardia* | *intenstinalis* *lamblia* | Example of water-borne flagellate and zoonosis |
| *Trichomonas* | *vaginalis* | Example of gut flagellate in birds. Venereally transmitted flagellate causing abortion & infertility |
| *Histomonas* | *meleagridis* | Example of a parasite transmitted by another parasite - Heterakis |
| *Trypanosoma* | *avium brucei cruzi equiperdum evansi vivax* | Example of a venerally transmitted flagellate |
| *Eimeria* | *acervulina* *brunetti* *jemezi* *maxima* *nextrix* *tenella* *stiedae* *meleagridis* | Apicomplexan parasite responsible for the poultry disease coccidiosis. Used to illustrate the basic characteristics of the coccidian direct lifecycle. Ovine, bovine & rabbit coccidiosis mentioned but not by species. |
| *Isospora* | *belli* *felis* *canis* | Mentioned as the dog/cat/pig equivalent of *Eimeria* |
| *Cyclospora* | *cayetanensis* | Traveler's Diarrhea. |
| *Cryptosporidium* | *parvum* *hominis* | Of the Phylum Apicomplexa and causes a diarrheal illness called |
| | *canis* *felis* *hominis* *meleagridis* *muris* | cryptosporidiosis. Example of an important water borne zoonosis. |
| *Sarcocystis* | *cruzi* *hominis* *muris* | Used to illustrate the basic characteristics of the coccidian indirect lifecycle. Can proliferate when undercooked meat is ingested. Symptoms include diarrhea, which can be mild and transient or severe and life threatening. |
| *Toxoplasma* | *gondii* | The definitive host is the cat, but the parasite can be carried by the vast majority of warm-blooded animals, including humans. The causative agent of toxoplasmosis. |
| *Neospora* | *caninum* | Important pathogen in cattle and dogs. Highly transmissible with some herds having up to 90% prevalence. Causes abortions. |
| *Babesia* | *major* *microti* *divergens* *duncani* *gibsoni* | Example of tick-borne protozoa, responsible for causing Texas Fever. |
| *Plasmodium* | *falciparum* *vivax* *ovale* *malariae* *knowlesi* *gigliolii* | Example of an endemic insect borne protozoan. Causative agent of malaria. |
| *Leishmania* | *aethiopica* *donovani* *major* *mexicana* *tropica* *braziliensis* | Example of insect borne protozoan that lives inside host macrophages |

**Trematodes**

| Parasite (*Genus*) | (*Species*) | Context |
|---|---|---|
| *Fasciola* | *hepatica* *magna* *gigantica* *jacksoni* | Also known as the **common liver fluke** it is a parasitic flatworm of phylum Platyhelminthes that infects liver of a various mammals, including man. The disease caused by the fluke is called fascioliasis (also known as fasciolosis). *F. hepatica* is worldwide distributed and causes great economic losses in sheep and cattle. |
| *Dicrocoelium* | *dendriticum* | The Lancet liver fluke is a parasite fluke that tends to live in cattle or other grazing mammals. |
| *Schistosoma* | *mansoni* *japonicum* *mekongi* *intercalatum* *haematobium* | Commonly known as **blood-flukes** and **bilharzia,** cause the most significant infection of humans by flatworms. Considered by the World Health Organization as second in importance only to malaria. |

**Cestodes**

| Parasite (*Genus*) | (*Species*) | Context |
|---|---|---|
| *Taenia* | *crassiceps* *pisiformis* *saginata* *solium* | Example of tapeworms with humans as natural definite hosts but with implications for zoonoses and meat inspection |
| *Dipylidium* | *caninum* | Also called the **cucumber tapeworm** or the **double-pore** tapeworm, it infects organisms afflicted with fleas, including canids, felids, and pet-owners, especially children. |
| *Echinococcus* | *granulosus* *multilocularis* *shiquicus* | Includes six species of cyclophyllid tapeworms Infection with *Echinococcus* results in hydatid disease, also known as *echinococcosis.* |

**Nematodes**

| Parasite (*Genus*) | (*Species*) | Context |
|---|---|---|
| *Aphelenchoides* | *fragariae* *ritzemabosi* *besseyi.* | Foliar nematodes are plant parasitic roundworms which are a widespread problem for the ornamental and nursery industries. |
| *Heterodera* | | Soybean cyst nematode. |
| *Globodera* | *solanacearum* *virginiae* *tabacum* | **Potato cyst nematode.** |
| *Nacobbus* | *dorsalis* | **False Root-knot.** |
| *Pratylenchus* | *brachurus* *penetrans* | **Brown root rot.** |
| *Ditylenchus* | *dipsaci* | Plant pathogenic nematode which infects the bud and stem. |
| *Xiphinema* | *americanum* | **American dagger nematode;** plant pathogen. |
| *Longidorus* | *svlphus* | Attacks mint. |
| *Paratrichodorus* | *minor* | **Christie's stubby root nematode.** |
| *Dioctophyma* | *renale* | **Giant kidney worm;** common parasital worm found in carnivorous animals. |
| *Meloidogyne* | *hapla* *incognita* *javanica* | **Root-knot** nematodes infect plant roots and are one of the three most economically damaging genera of nematodes on horticultural and field crops. |
| *Trichostrongylus* | *tenius* | Used as a basic nematode lifecycle |
| *Ostertagia* or *Teladorsagia* | | Highlights impact of larval development in abomasum wall, differences between type I & II, example of seasonally-induced hypobiosis |
| *Nematodirus* | | Example of nematode developing in the gut lumen, example of nematode with critical hatching conditions |
| *Haemonchus* | | Example of blood-feeding nematode |
| *Cooperia* | | Distinctive coiled nematode of ruminants |
| *Trichuris* | | Distinctive whip-like nematode of ruminants |
| *Ascaris* | | Example of hepato-trachael migratory nematode |
| *Parascaris* | | Important equine nematode |
| *Oxyuris* | | Distinctive pin-worm of equines |
| *Toxascaris* | | Example of non-migratory ascarid of dogs & cats referred forward to the migratory *Toxocara sp.* |
| *Toxocara* | | Example of complex migratory nematode with hypobiotic larval stages, complex biochemical interactions between host & parasite, congenital infections, vertical transmission, zoonosis, reproductive-related hypobiosis. Comparison with T.catti, refs back to non-migratory Toxascaris |
| *Trichinella* | | Example of hypobiotic larvae, no external stages, zoonosis |
| *Oesophagostomum* | | Example of strongyle of ruminants with extensive cuticular ornamentation and nodule formation on gut wall |
| *Chabertia* | | Example of strongyle of ruminants with large buccal capsule as adaptation to tissue feeding |
| *Cyathostomes* or *Trichonemes* | | Horse colic. |
| *Strongylus* | *vulgaris* | **Blood worm;** common horse parasite. |
| *Bunostomum* | | Example of hookworm of ruminants |
| *Uncinaria* | | Example of canine/feline "northern" hookworm |
| *Ancylostoma* | | Example of potential emerging hookworm related to climate change/behaviour |
| *Dictyocaulus* | | Basic lungworm direct lifecycle, vaccination using irradiated larvae |
| *Metastrongylus* | | Lungworm with indirect lifecycle, used to reinforce concepts of transport, paratenic & intermediate host using earthworm as example |
| *Parafilaria* | | Example of filarial worm, example of insect-borne parasite that does not involve a blood-feeding vector |
| *Dirofialria* | | Example of filarial worm transmitted by blood-feeding vector, distribution limited by that of vector, potential impact of climate change on distribution |

**Fungi**

| Parasite (*Genus*) | (*Species*) | Context |
|---|---|---|
| *Cercospora* | *zeae-maydis* | Etiological agent of **grey leaf spot** in cereal plants. |
| *Ustilago* | *maydis* | Etiological agent of **corn smut** disease of maize. |
| *Magnaporthe* | *grisea* | Most significant disease affecting rice cultivation; **rice blast.** |
| *Bipolaris* | *oryzae* | **Brown spot** can infect both seedlings and mature plants. |

**Acarina - Mites And Ticks**

| Parasite (*Genus*) | Context |
|---|---|
| Psoropftic mites - *Psoroptes ovis, Chorioptes* | Sheep scab aetiology and control. Topology of infestation in relation to skin histology. |
| Sarcoptic mites - *Sarcoptes, Knemidocoptes* | Causation of mange, hypersensitivity and pruritus. Topology of infestation in relation to skin histology. |
| Demodectic mites - *Demodex, Trombicula, Cheyletiella* | Causation of demodecosis. Topology of infestation in relation to histology of skin. Aesthetic and zoonotic problems with *Cheyletiella.* |
| Dermanyssid mites - *Dermanyssus, Ornithonyssus* | Nature of infestation as micro-predator. Importance to poultry industry. Control by hygiene and pesticides. |
| *Ixodes ricinus* | Vector of agents of babesiosis, tick borne fever, louping ill and Lyme disease. |

**Lice and Fleas**

| Parasite (*Genus*) | Context |
|---|---|
| *Linognathus* and *Haematopinus sp.* | Example of sessile ectoparasites with incomplete metamorphosis causing stress and hide damage. Example of blood feeding anopluran lice. |
| *Trichodectes* and *Felicola* | Lice problems in small companion animals caused by chewing lice. Role as intermediate host of *Dipylidium* tapeworm. |
| *Lipeurus, Cuclotogaster, Menopon* | Two families of chewing lice on birds. All bird lice are chewing lice causing irritation and production losses. |
| *Ctenocephalides felis* and *C. canis* | Cat/Dog flea; one of the most abundant and widespread fleas in the world. |
| *Ceratophyllus* and *Echidnophaga* | Parasitizes mainly rodents and birds. |

**Flies**

| Parasite (*Genus*) | Context |
|---|---|
| Muscid flies | Importance of flies with sponging mouthparts a nuisance leading to production losses in dairy cattle and as mechanical vectors of pathogens such as Moraxella bacteria. |
| *Haematobia* and *Stomoxys* | **Horn fly;** *H. irritans* is a bloodsucking fly dangerous to livestock. |
| Tabanid flies | Examples of biting stress caused by flies with complex slashing and sponging blood feeding mouthparts. Example of life cycle of flies with complete metamorphosis. |
| Melophagus ovinus | **Louse flies** or **keds;** obligate parasite of mammals and birds - can serve as the vector of pigeon malaria. |
| Culicoides midges | Example of how flies act as vectors. |
| Mosquitoes | Vectors of viral, protozoal and nematode pathogens. |
| Phlebotomus sand flies | Vector of *Leishmania* protozoa. |
| Lucilia cuprina blowfly | Example of facultative myiasis - blowfly strike. |
| Hypoderma bovis | Example of obligate myiasis - warble fly. Example of low reproduction / high survival system. |
| Gasterophilus and Oestrus bots | Illustration of these forms of myiasis. |

**Parasite list by host**

| **Canola** | |
|---|---|
| (*Brassica rapa*) | |

| | **Fungal Diseases** |
|---|---|
| Alternaria black spot | *Alternaria brassicae* |
| = Dark pod spot (UK) | *Alternaria brassicicola* |
| | *Alternaria japonica* |
| | = Alternaria raphani |
| Anthracnose | *Colletotrichum gloeosporioides* |
| | *Glomerella cingulata* [teleomorph] |
| | *Colletotrichum higginsianum* |
| Black leg = stem canker (UK) | *Leptosphaeria maculans* |
| | *Phoma lingam* [anamorph] |
| Black mold rot | *Rhizopus stolonifer* |
| Black root | *Aphanomyces raphani* |
| Brown girdling root rot | *Rhizoctonia solani Thanatephorus cucumeris* [teleomorph] |
| Cercospora leaf spot | *Cercospora brassicicola* |
| Clubroot | *Plasmodiophora brassicae* |
| Downy mildew | *Peronospora parasitica* |
| Fusarium wilt | *Fusarium oxysporum f.sp. conglutinans* |
| Gray mold | *Botrytis cinerea* |
| | *Botryotinia fuckeliana* [teleomorph] |
| Head rot | *Rhizoctonia solani* |
| | *Thanatephorus cucumeris* [teleomorph] |
| Leaf spot | *Alternaria alternata* |
| | *Ascochyta spp.* |
| | *Pyrenopeziza brassicae* |
| Light leaf spot | *Cylindrosporium concentricum* [anamorph] |
| Pod rot | *Alternaria alternata* |
| | *Cladosporium spp.* |
| Powdery mildew | *Erysiphe polygoni* |
| | *Erysiphe cruciferarum* |
| | *Mycosphaerella brassicicola* |
| Ring spot | *Asteromella brassicae* [anamorph] |
| | *Alternaria alternata* |
| Root rot | *Fusarium spp.* |
| | *Macrophomina phaseolina* |
| | *Phymatotrichopsis omnivora* |
| | *Phytophthora megasperma* |
| | *Pythium debaryanum* |
| | *Pythium irregulare* |
| | *Rhizoctonia solani* |
| | *Thanatephorus cucumeris* [teleomorph] |
| | *Sclerotium rolfsii* |
| | *Athelia rolfsii* [teleomorph] |
| Sclerotinia stem rot | *Sclerotinia sclerotiorum* |
| Seed rot, damping-off | *Alternaria spp.* |
| | *Fusarium spp.* |
| | *Gliocladium roseum* |
| | *Nectria ochroleuca* [teleomorph] |
| | *Pythium spp.* |
| | *Rhizoctonia solani* |
| | *Thanatephorus cucumeris* [teleomorph] |
| | *Rhizopus stolonifer* |
| | *Sclerotium rolfsii* |
| Root gall smut | *Urocystis brassicae* |
| Southern blight (leaf, root and seed rot) | *Sclerotium rolfsii* |
| Verticillium wilt | *Verticillium longisporum* |
| White blight | *Rhizoctonia solani* |
| | *Thanatephorus cucumeris* [teleomorph] |
| White leaf spot = grey stem (Canada) | *Pseudocercosporella capsellae* = *Cercosporella brassicae* |
| | *Mycosphaerella capsellae* [teleomorph] |
| White rust = staghead | *Albugo candida* = *Albugo cruciferarum (Peronospora* sp commonly present in staghead phase) |
| Yellows | *Fusarium oxysporum* |

| **Cat** (*Felis catus*) | |
|---|---|
| Apicomplexa : | |
| *Besnoitia* sp. (*oocysts*) | |
| *Isospora, felis* | |
| *Isospora rivolta* | |
| *Sarcocystis gigantea* (*sporocysts*) | |
| *Sarcocystis hirsuta* (*sporocysts*) | |
| *Sarcocystis medusijormis* (*sporocysts*) | |
| *Sarcocystis muris* (*sporocysts*) | |
| *Sarcocystis* sp. (*sporocysts*) | |
| *Toxoplasma gondii* (*cysts*) | |
| *Toxoplasma gondii* (*oocysts*) | |

| Sarcomastigophora: | |
|---|---|
| *Giardia intestinalis* | |

| **Dog** | |
|---|---|
| (*Canis familiaris*) | |
| Apicomplexa : | |
| *Hammondia heydorni* (*oocysts*) | |
| *Isospora canis* | |
| *Isospora ohioensis* | |
| *Neospora caninum* | |
| *Sarcocystis arieticanis* (*sporocysts*) | |
| *Sarcocystis capracanis* (*sporocysts*) | |
| *Sarcocystis cruzi* (*sporocysts*) | |
| *Sarcocystis tenella* (*sporocysts*) | |
| *Sarcocystis sp.* (*sporocysts*) | |
| *Toxoplasma gondii* (*cysts*) | |

| Sarcomastigophora: | |
|---|---|
| *Giardia intestinalis* | |

| **Goat** | |
|---|---|
| (*Capra hircus*) | |

| Apicomplexa : | |
|---|---|
| *Cryptosporidium sp.* | |
| *Eimeria alijevi* | |
| *Eimeria apsheronica* | |
| *Eimeria arloingi* | |
| *Eimeria capralis* | |
| *Eimeria caprina* | |
| *Eimeria caprovina* | |
| *Eimeria charlestoni* | |
| *Eimeria christenseni* | |
| *Eimeria hirci* | |
| *Eimeria jolchejevi* | |
| *Eimeria masseyensis* | |
| *Eimeria ninakohlyakimovae* | |
| *Eimeria punctata* | |
| *Eimeria tunisiensis* | |
| *Sarcocystis capracanis* (*cysts*) | |
| *Toxoplasma gondii* (*cysts*) | |

| Sarcomastigophora: | |
|---|---|
| *Giardia sp.* | |

| **Horse** | |
|---|---|
| (*Equus caballus*) | |

| Apicomplexa : | |
|---|---|
| *Eimeria leuckarti* | |
| *Klossiella equi* | |
| *Sarcocystis* sp. (cysts) | |

| **Man** | |
|---|---|
| (*Homo sapiens*) | |

| Apicomplexa : | |
|---|---|
| *Ciyptosporidium* sp. | |
| *Isospora hominis* * | |
| *Plasmodium* sp. * | |
| *Toxoplasma gondii* (cysts) | |

| Sarcomastisophora : | |
|---|---|
| *Chilomastix mesnili* | |
| *Dientamoeba fragilis* | |
| *Endolimax nana* | |
| *Entamoeba coli* | |
| *Entamoeba hartmanni* | |
| *Entamoeba histolytica* | |
| *Giardia intestinalis* | |
| *Iodamoeba buetschlii* | |
| *Leishmania donovani* * | |
| *Trichomonas hominis* | |
| *Trichomonas vaginalis* | |

| **Maize** | |
|---|---|
| (*Zea mays*) | |

| | **Fungal Diseases** |
|---|---|
| Anthracnose leaf blight | *Colletotrichum graminicola* |
| Anthracnose stalk rot | *Glomerella graminicola* |
| | *Glomerella tucumanensis* |
| | *Glomerella falcatum* |
| Aspergillus ear and kernel rot | *Aspergillus flavus* |
| Banded leaf and sheath spot | *Rhizoctonia solani* = *Rhizoctonia microsclerotia Thanatephorus cucumeris* |
| Black bundle disease | *Acremonium strictum* = *Cephalosporium acremonium* |
| Black kernel rot | *Lasiodiplodia theobromae* = *Botryodiplodia theobromae* |
| Borde blanco | *Marasmiellus sp.* |
| Brown spot | *Physoderma maydis* |
| Black spot | |
| Stalk rot | |
| Cephalosporium kernel rot | *Acremonium strictum* = *Cephalosporium acremonium* |
| Charcoal rot | *Macrophomina phaseolina* |
| Corticium ear rot | *Thanatephorus cucumeris* = *Corticium sasakii* |
| Curvularia leaf spot | *Curvularia clavata* |
| | *C. eragrostidis* = *C. maculans* |
| | *Cochliobolus eragrostidis* |
| | *Curvularia inaequalis* |
| | *C. intermedia* |
| | *Cochliobolus intermedius* |
| | *Curvularia lunata* |
| | *Cochliobolus lunatus* |
| | *Curvularia pallescens Cochliobolus pallescens* |
| | *Curvularia senegalensis* |
| | *C. tuberculate* |
| | *Cochliobolus tuberculatus* |
| Didymella leaf spot | *Didymella exitalis* |
| Diplodia ear rot and stalk rot | *Diplodia frumenti* |
| | *Botryosphaeria festucae* |
| Diplodia ear rot Stalk rot Seed rot Seedling blight | *Diplodia maydis* |
| Diplodia leaf spot or leaf streak | *Stenocarpella macrospora* = *Diplodia macrospora* |

| | **Downy mildews** |
|---|---|
| Brown stripe downy mildew | *Sclerophthora rayssiae* |
| Crazy top downy mildew | *Sclerophthora macrospora* = *Sclerospora macrospora* |
| Green ear downy mildew | |
| Graminicola downy mildew | *Sclerospora graminicola* |
| Java downy mildew | *Peronosclerospora maydis* = *Sclerospora maydis* |
| Philippine downy mildew | *Peronosclerospora philippinensis* = *Sclerospora philippinensis* |
| Sorghum downy mildew | *Peronosclerospora sorghi* = *Sclerospora sorghi* |
| Spontaneum downy mildew | *Peronosclerospora spontanea* = *Sclerospora spontanea* |
| Sugarcane downy mildew | *Peronosclerospora sacchari* = *Sclerospora sacchari* |
| Dry ear rot | *Nigrospora oryzae* |
| Cob, kernel and stalk rot | *Khuskia oryzae* |
| Ear rots, minor | *Alternaria alternate* = *A. tenuis* |
| | *Aspergillus glaucus* |
| | *A. niger* |
| | *Aspergillus spp.* |
| | *Botrytis cinerea* |
| | *Botryotinia fuckeliana* |
| | *Cunninghamella* sp. |
| | *Curvularia pallescens* |
| | *Doratomyces stemonitis* = *Cephalotrichum stemonitis* |
| | *Fusarium culmorum* |
| | *Gonatobotrys simplex* |
| | *Pithomyces maydicus* |
| | *Rhizopus microsporus* |
| | *R. stolonifer* = *R. nigricans* |
| | *Scopulariopsis brumptii* |
| Ergot | *Claviceps gigantea* |
| Horse's tooth | *Sphacelia sp.* |
| Eyespot | *Aureobasidium zeae* = *Kabatiella zeae* |
| Fusarium ear and stalk rot | *Fusarium subglutinans* = *F. moniliforme* |
| Fusarium kernel, root and stalk rot, seed rot and seedling blight | *Fusarium moniliforme* |
| | *Gibberella fujikuroi* |
| Fusarium stalk rot | *Fusarium avenaceum* |
| Seedling root rot | *Gibberella avenacea* |
| Gibberella ear and stalk rot | *Gibberella zeae Fusarium graminearum* |
| Gray ear rot | *Botryosphaeria zeae* = *Physalospora zeae Macrophoma zeae* |
| Gray leaf spot | *Cercospora sorghi* = *C. sorghi* |
| Cercospora leaf spot | *C. zeae-maydis* |
| Helminthosporium root rot | *Exserohilum pedicellatum* = *Helminthosporium pedicellatum* |
| | *Setosphaeria pedicellata* |
| Hormodendrum ear rot | *Cladosporium cladosporioides* = |
| Cladosporium rot | *Hormodendrum cladosporioides* |
| | *C. herbarum* |
| | *Mycosphaerella tassiana* |
| Hyalothyridium leaf spot | *Hyalothyridium maydis* |
| Late wilt | *Cephalosporium maydis* |
| Leaf spots, minor | *Alternaria alternate* |
| | *[[Ascochyta maydis]]* |
| | *A. tritici* |
| | *A. zeicola* |
| | *Bipolaris victoriae* = *Helminthosporium victo riae* |
| | *Cochliobolus victoriae C. sativus* |
| | *Bipolaris sorokiniana* = *H. sorokinianum* = *H*. *sativum* |
| | *Epicoccum nigrum* |
| | *Exserohilum prolatum* = *Drechslera prolata Setosphaeria prolata* |
| | *Graphium penicillioides* |
| | *Leptosphaeria maydis* |
| | *Leptothyrium zeae* |
| | *Ophiosphaerella herpotricha* |
| | *Scolecosporiella sp.* |
| | *Paraphaeosphaeria michotii* |
| | *Phoma sp.* |
| | *Septoria zeae* |
| | *S. zeicola* |
| | *S. zeina* |
| Northern corn leaf blight | *Setosphaeria turcica* |
| White blast Crown stalk rot | *Exserohilum turcicum* = *Helminthosporium turcicum* |
| Stripe | |
| Northern corn leaf spot | *Cochliobolus carbonum* |
| Helminthosporium ear rot (race 1) | *Bipolaris zeicola* = *Helminthosporium carbonum* |
| Penicillium ear rot | *Penicillium spp.* |
| Blue eye | *P. chrysogenum* |
| Blue mold | *P. expansum* |
| | *P. oxalicum* |
| Phaeocytostroma stalk rot and root rot | *Phaeocytostroma ambiguum* = *Phaeocytosporella zeae* |
| Phaeosphaeria leaf spot | *Phaeosphaeria maydis* = *Sphaerulina maydis* |
| Physalospora ear rot | *Botryosphaeria festucae* = *Physalospora zeicola* |
| Botryosphaeria ear rot | *Diplodia frumenti* |
| Purple leaf sheath | Hemiparasitic bacteria and fungi |
| Pyrenochaeta stalk rot and root rot | *Phoma terrestris* = *Pyrenochaeta terrestris* |
| Pythium root rot | *Pythium spp. P. arrhenomanes P. graminicola* |
| Pythium stalk rot | *Pythium aphanidermatum* = *P. butleri* |
| Red kernel disease | *Epicoccum nigrum* |
| Ear mold, leaf and seed rot | |
| Rhizoctonia ear rot | *Rhizoctonia zeae* |
| Sclerotial rot | *Waitea circinata* |
| Rhizoctonia root rot and stalk rot | *Rhizoctonia solani* |
| | *R. zeae* |
| Root rots, minor | *Alternaria alternata* |
| | *Cercospora sorghi* |
| | *Dictochaeta fertilis* |
| | *Fusarium acuminatum Gibberella acuminate* |
| | *F. equiseti* |
| | *G. intricans* |
| | *F. oxysporum* |
| | *F. pallidoroseum* |
| | *F. poae* |
| | *F. roseum* |
| | *G. cyanogena* |
| | *F. sulphureum* |
| | *Microdochium bolleyi* |
| | *Mucor* sp. |
| | *Periconia circinata* |
| | *Phytophthora cactorum* |
| | *P. drechsleri* |
| | *P. nicotianae* |
| | *Rhizopus arrhizus* |
| Rostratum leaf spot | *Setosphaeria rostrata* = *Helminthosporium* |
| Helminthosporium leaf disease, ear and stalk rot | *rostratum* |
| Rust, common corn | *Puccinia sorghi* |
| Rust, southern corn | *Puccinia polysora* |
| Rust, tropical corn | *Physopella pallescens* |
| | *P. zeae* = *Angiopsora zeae* |
| Sclerotium ear rot | *Sclerotium rolfsii* |
| Southern blight | *Athelia rolfsii* |
| Seed rot-seedling blight | *Bipolaris sorokiniana* |
| | *B. zeicola* = *Helminthosporium carbonum* |
| | *Diplodia maydis* |
| | *Exserohilum pedicillatum* |
| | *Exserohilum turcicum* = *Helminthosporium* |
| | *turcicum* |
| | *Fusarium avenaceum* |
| | *F. culmorum* |
| | *F. moniliforme* |
| | *Gibberella zeae* |
| | *F. graminearum* |
| | *Macrophomina phaseolina* |
| | *Penicillium spp.* |
| | *Phomopsis spp.* |
| | *Pythium spp.* |
| | *Rhizoctonia solani* |
| | *[Rhizoctonia zeae\R. zeae* |
| | *Sclerotium rolfsii* |
| | *Spicaria spp.* |
| Selenophoma leaf spot | *Selenophoma* sp. |
| Sheath rot | *Gaeumannomyces graminis* |
| Shuck rot | *Myrothecium gramineum* |
| Silage mold | *Monascus purpureus* |
| | *M. ruber* |
| Smut, common | *Ustilaso zeae* = *U. maydis* |
| Smut, false | *Ustilasinoidea vixens* |
| Smut, head | *Sphacelotheca reiliana* = *Sporisorium holci-sorghi* |
| Southern corn leaf blight and stalk rot | *Cochliobolus heterostrophus Bipolaris maydis* = *Helminthosporium maydis* |
| Southern leaf spot | *Stenocarpella macrospora* = *Diplodia macrospora* |
| Stalk rots, minor | *Cercospora sorghi* |
| | *Fusarium episphaeria* |
| | *F. merismoides* |
| | *F. oxysporum* |
| | *F. poae* |
| | *F. roseum* |
| | *F. solani* |
| | *Nectria haematococca* |
| | *F. tricinctum* |
| | *Mariannaea elegans* |
| | *Mucor spp.* |
| | *Rhopographus zeae* |
| | *Spicaria spp.* |
| Storage rots | *Aspergillus spp. Penicillium spp.* and other fungi |
| Tar spot | *Phyllachora maydis* |
| Trichoderma ear rot and root rot | *Trichoderma viride* = *T. lignorum Hypocrea* sp. |
| White ear rot, root and stalk rot | *Stenocarpella maydis* = *Diplodia zeae* |
| Yellow leaf blight | *Ascochyta ischaemi* |
| Stalk rots, minor | *F. solani* |
| | *Nectria haematococca* |
| | *F. tricinctum* |
| | *Mariannaea elegans* |
| | *Mucor spp.* |
| | *Rhopographus zeae* |
| | *Spicaria spp.* |
| Storage rots | *Aspergillus spp. Penicillium spp. and other fungi* |
| Tar spot | *Phyllachora maydis* |
| Trichoderma ear rot and root rot | *Trichoderma viride* = *T lignorum Hypocrea sp.* |
| White ear rot, root and stalk rot | *Stenocarpella maydis* = *Diplodia zeae* |
| Yellow leaf blight | *Ascochyta ischaemi Phyllosticta maydis Mycosphaerella zeae-maydis* |
| Zonate leaf spot | *Gloeocercospora sorghi* |

| | **Nematodes** |
|---|---|
| Awl | *Dolichodorus spp.* |
| | *D. heterocephalus* |
| Bulb and stem | *Ditylenchus dipsaci* |
| Burrowing | *Radopholus similis* |
| Cyst | *Heterodera avenae* |
| | *H. zeae* |
| | *Punctodera chalcoensis* |
| Dagger | *Xiphinema spp.* |
| | *X. Americanum X. mediterraneum* |
| False root-knot | *Nacobbus dorsalis* |
| Lance, Columbia | *Hoplolaimus Columbus* |
| Lance | *Hoplolaimus spp. H. galeatus* |
| Lesion | *Pratylenchus spp.* |
| | *P. brachyurus* |
| | *P. crenatus* |
| | *P. hexincisus* |
| | *P. neglectus* |
| | *P. penetrans* |
| | *P. scribneri* |
| | *P. thornei* |
| | *P. zeae* |
| Needle | *Longidorus spp.* |
| | *L. breviannulatus* |
| Ring | *Criconemella spp.* |
| | *C. ornata* |
| Root-knot | *Meloidogyne spp.* |
| | *M. chitwoodi* |
| | *M. incognita* |
| | *M. javanica* |
| Spiral | *Helicotylenchus spp.* |
| Sting | *Belonolaimus spp.* |
| | *B. longicaudatus* |
| Stubby-root | *Paratrichodorus spp.* |
| | *P. christiei* |
| | *P. minor* |
| | *Quinisulcius acutus* |
| | *Trichodorus spp.* |
| Stunt | *Tylenchorhynchus dubius* |

| **Mouse** | |
|---|---|
| *(Mus musculus)* | |

| Apicomplexa : | |
|---|---|
| *Hepatozoon musculi* | |
| *Sarcocystis muris* (cysts) | |

| Sarcomastigophora: | |
|---|---|
| *Giardia intestinalis* | |
| *Giardia muris* | |

| **Ox** | |
|---|---|
| (*Bos tarus*) | |

| *Apicomplexa:* | |
|---|---|
| Cryptosporidium *sp.* | |
| Eimeria alabamensis | |
| Eimeria auburnensis | |
| Eimeria bovis | |
| Eimeria brasiliensis | |
| Eimeria bukidnonensis | |
| Eimeria canadensis | |
| Eimeria cylindrica | |
| Eimeria ellipsoidalis | |
| Eimeria subspherica | |
| Eimeria wyomingensis | |
| Eimeria zurnii | |
| Isospora *sp.* | |
| *Neospora caninum* | |
| *Sarcocystis cruzi* (cysts) | |
| *Sarcocystis hirsuta* (cysts) | |
| *Theileria orientalis* | |

| Sarcomastigophora: | |
|---|---|
| *Tritrichomonas foetus* | |

| Ciliophora: | |
|---|---|
| *Balantidium coli* | |

| **Pig** | |
|---|---|
| (*Sus scrofa*) | |

| Apicomplexa: | |
|---|---|
| *Cryptosporidium* sp. | |
| *Eimeria cerdonis* | |
| *Eimeria debliecki* | |
| *Eimeria neodebliecki* | |
| *Eimeria porci* | |
| *Eimeria scabra* | |
| *Eimeria suis* | |
| *Isospora suis* | |
| *Sarcocystis* sp. (cysts) | |
| *Toxoplasma gondii* (cysts) | |

| Ciliophora: | |
|---|---|
| *Balantidium coli* | |

| **Poultry** | |
|---|---|
| (*Gallus gallus*) | |

| Endoparasites: | |
|---|---|
| Protozoa: | |
| *Histomonas meleagridis* | |
| *Hexamita meleagridis* | |
| *Eimeria spp.* | |
| Helminths: | |
| *Ascaridia galli* | |
| *Ascaridia dissimilis* | |
| *Ascardidia columbae* | |
| *Capillaria contorta* | |
| *Capillaria obsingata* | |
| *Capillaria caudinflata* | |
| *Heterakis gallinarum* | |
| *Heterakis isolonche* | |
| *Syngamus trachea* | |

| Ectoparasites: | |
|---|---|
| Mites: | |
| *Cnemidocoptes mutans* | |
| *Cnemidocoptes gallinae* | |
| *Dermanyssus gallinae* | |
| *Lamiosioptes cysticola* | |
| *Ornithonyssus slyvarium* | |

| Fleas: | |
|---|---|
| *Ceratophyllus gallinae Echindnophaga gallinacea* | |

| Lice: | |
|---|---|
| *Menacanthus stramineus* | |

| **Rabbit** | |
|---|---|
| (*Otyctolagus cuniculus*) | |
| Apicomplexa: | |
| *Eimeria flavescens* | |
| *Eimeria irresidua* | |
| *Eimeria media* | |
| *Eimeria petforans* | |
| *Eimeria pyriformis* | |
| *Eimeria stiedae* | |
| *Hepatozoon cuniculi* | |
| *Sarcocystis sp.* (cysts) | |
| *Toxoplasma gondii* (cysts) | |

| **Rice** | |
|---|---|
| (*Oryza sativa*) | |

| | **Fungal diseases** |
|---|---|
| Aggregate sheath spot | *Ceratobasidium oryzae-sativae Rhizoctonia oryzae-sativae* |
| Black kernel | *Curvularia lunata Cochliobolus lunatus* |
| Blast (leaf, neck [rotten neck], nodal and collar) | *Pyricularia grisea* = *Pyricularia oryzae Magnaporthe grisea* |
| Brown spot | *Cochliobolus miyabeanus Bipolaris oryzae* |
| Crown sheath rot | *Gaeumannomyces graminis* |
| Downy mildew | *Sclerophthora macrospora* |
| Eyespot | *Drechslera gigantea* |
| False smut | *Ustilaginoidea virens* |
| Kernel smut | *Tilletia barclayana* = *Neovossia horrida* |
| Leaf smut | *Entyloma oryzae* |
| Leaf scald | *Microdochium oyvzae* = *Rhynchosporium oryzae* |
| Narrow brown leaf spot | *Cercospora janseana* = *Cercospora oryzae Sphaerulina oryzina* |
| Pecky rice (kernel spotting) | Damage by many fungi including |
| | *Cochliobolus miyabeanus* |
| | *Curvularia spp.* |
| | *Fusarium spp.* |
| | *Microdochium oryzae* |
| | *Sarocladium oryzae* and other fungi. |
| Root rots | *Fusarium spp.* |
| | *Pythium spp.* |
| | *Pythium dissotocum* |
| | *Pythium spinosum* |
| Seedling blight | *Cochliobolus miyabeanus* |
| | *Curvularia spp.* |
| | *Fusarium spp.* |
| | *Rhizoctonia solani* |
| | *Sclerotium rolfsii* |
| | *Athelia rolfsii* |
| Sheath blight | *Thanatephorus cucumeris* |
| | *Rhizoctonia solani* |
| Sheath rot | *Sarocladium oryzae* = *Acrocylindrium oryzae* |
| Sheath spot | *Rhizoctonia oryzae* |
| Stackburn (Alternaria leaf spot) | *Alternaria padwickii* |
| Stem rot | *Magnaporthe salvinii* |
| | *Sclerotium oryzae* |
| Water-mold (seed-rot and seedling disease) | *Achlya conspicua* |
| | *Achlya klebsiana* |
| | *Fusarium spp.* |
| | *Pythium spp.* |
| | *Pythium dissotocum* |
| | *Pythium spinosum* |

| | **Nematodes, parasitic** |
|---|---|
| Crimp nematode, summer | *Aphelenchoides besseyi* |
| Root-knot | *Meloidogyne spp.* |
| Root nematode, rice | *Hirschmanniella oryzae* |
| Stem nematode, rice | *Ditylenchus angustus* |

| **Sheep** | |
|---|---|
| (*Ovis aries*) | |

| Apicomplexa : | |
|---|---|
| *Ctyptosporidium* sp. | |
| *Eimeria ahsata* | |
| *Eimeria crandallis* | |
| *Eimeria faurei* | |
| *Eimeria granulosa* | |
| *Eimeria intricata* | |
| *Eimeria ovinoidalis* | |
| *Eimeria ovis* | |
| *Eimeria pallida* | |
| *Eimeria pama* | |
| *Eimeria punctata* | |
| *Eimeria weybridgensis* | |
| *Sarcocystis arieticanis* (cysts) | |
| *Sarcocystis gigantea* (cysts) | |
| *Sarcocystis medusiformis* (cysts) | |
| *Sarcocystis tenella* (cysts) | |
| *Toxoplasma gondii* (cysts) | |

| **Soybean** | |
|---|---|
| (*Glycine max*) | |

| | **Fungal diseases** |
|---|---|
| Alternaria leaf spot | *Alternaria spp.* |
| Anthracnose | *Collelotrichum runcatum* |
| | *Collelotrichum demalium f. truncatum* |
| | *Glomerella glycines* |
| | *Colletotrichum destructivum* |
| Black leaf blight | *Arkoola nigra* |
| Black root rot | *Thielaviopsis basicola Chalara elegans* [synanamorph] |
| Brown spot | *Septoria glycines Mycosphaerella usoenskajae* |
| Brown stem rot | *Phialophora gregata* = *Cephalosporium gregatum* |
| Charcoal rot | *Macrophomina phaseolina* |
| Choanephora leaf blight | *Choanephora infundibulifera Choanephora trispora* |
| Damping-off | *Rhizoctonia solani* |
| | *Thanatephorus cucumeris* |
| | *Pythium aphanidermatum* |
| | *Pythium debaryanum* |
| | *Pythium irregulare* |
| | *Pythium myriotylum* |
| | *Pythium ultimum* |
| Downy mildew | *Peronospora manshurica* |
| Drechslera blight | *Drechslera glycines* |
| Frogeye leaf spot | *Cercosvora sojina* |
| Fusarium root rot | *Fusarium spp.* |
| Leptosphaerulina leaf spot | *Leptosphaerulina trifolii* |
| Mycoleptodiscus root rot | *Mycoleptodiscus terrestris* |
| Neocosmospora stem rot | *Neocosmospora vasinfecta Acremonium spp.* |
| Phomopsis seed decay | *Phomopsis spp.* |
| Phytophthora root and stem rot | *Phytophthora sojae* |
| Phyllosticta leaf spot | *Phyllosticta sojaecola* |
| Phymatotrichum root rot = cotton root rot | *Phymatotrichopsis omnivora* = *Phymatotrichum omnivorum* |
| Pod and stem blight | *Diaporthe phaseolorum Phomopsis sojae* |
| Powdery mildew | *Microsphaera diffusa* |
| Purple seed stain | *Cercospora kikuchii* |
| Pyrenochaeta leaf spot | *Pyrenochaeta glycines* |
| Pythium rot | *Pythium aphanidermatum Pythium debaryanum* |
| | *Pythium irregulare* |
| | *Pythium myriotylum* |
| | *Pythium ultimum* |
| Red crown rot | *Cylindrocladium crotalariae Calonectria crotalariae* |
| Red leaf blotch = Dactuliophora leaf spot | *Dactuliochaeta glycines* = *Pyrenochaeta glycines Dactuliophora glycines* [synanamorph] |
| Rhizoctonia aerial blight | *Rhizoctonia solani Thanatephorus cucumeris* |
| Rhizoctonia root and stem rot | *Rhizoctonia solani* |
| Rust | *Phakopsora pachyrhizi* |
| Scab | *Spaceloma glycines* |
| Sclerotinia stem rot | *Sclerotinia sclerotiorum* |
| Southern blight (damping-off and stem rot) = Sclerotium blight | *Sclerotium rolfsii* |
| | *Athelia rolfsii* |
| Stem canker | *Diaporthe phaseolorum* |
| | *Diaporthe phaseolorum var. caulivora* |
| | *Phomopsis phaseoli* |
| Stemphylium leaf blight | *Stemphylium botryosum Pleospora tarda* |
| Sudden death syndrome | *Fusarium solani f. sp. glycines* |
| Target spot | *Corynespora cassiicola* |
| Yeast spot | *Nematospora coryli* |

| | **Nematodes, parasitic** |
|---|---|
| Lance nematode | *Hoplolaimus columbus* |
| | *Hoplolaimus galeatus* |
| | *Hoplolaimus magnistylus* |
| Lesion nematode | *Pratylenchus spp.* |
| Pin nematode | *Paratylenchus projectus* |
| | *Paratylenchus tenuicaudatus* |
| Reniform nematode | *Rotylenchulus reniformis* |
| Ring nematode | *Criconemella ornata* |
| Root-knot nematode | *Meloidogyne arenaria* |
| | *Meloidogyne hapla* |
| | *Meloidogyne incognita* |
| | *Meloidogyne javanica* |
| Sheath nematode | *Hemicycliophora spp.* |
| Soybean cyst nematode | *Heterodera glycines* |
| Spiral nematode | *Helicotylenchus spp.* |
| Sting nematode | *Belonolainus gracilis Belonolainus longicaudatus* |
| Stubby root nematode | *Paratrichodorus minor* |
| Stunt nematode | *Quinisulcius acutus lylenchorhynchus spp.* |

| **Tobacco** | |
|---|---|
| (*Nicotiana tabacum*) | |

| | **Fungal diseases** |
|---|---|
| Anthracnose | *Collelotrichum destructivum Glomerella glycines* |
| Barn spot | *Cercospora nicotianae* |
| Barn rot | *Several fungi and bacteria* |
| Black root rot | *Thielaviopsis basicola* |
| Black shank | *Phytophthora nicotianae* |
| Blue mold (downy mildew) | *Peronospora tabacina* = *Peronospora hyoscyami f.sp. tabacina* |
| Brown spot | *Alternaria alternata* |
| Charcoal root | *Macrophomina phaseolina* |
| Collar rot | *Sclerotinia sclerotiorum* |
| Damping-off, Pythium | *Pythium spp.* |
| | *Pythium aphanidermatum* |
| | *Pythium ultimum* |
| Frogeye leaf spot | *Cercospora nicotianae* |
| Fusarium wilt | *Fusarium oxysporum* |
| Gray mold | *Botrytis cinerea Botryotinia fuckeliana* |
| Mycosphaerella leaf spot | *Mycosphaerella nicotianae* |
| Olpidium seedling blight | *Olpidium brassicae* |
| Phyllosticta leaf spot | *Phyllosticta nicotiana* |
| Powdery mildew | *Erysiphe cichoracearum* |
| Ragged leaf spot | *Phoma exigua var. exigua* = *Ascochyta phaseolorum* |
| Scab | *Hymenula affinis* = *Fusarium affine* |
| Sore shin and damping-off | *Rhizoctonia solani Thanatephorus cucumeris* |
| Southern stem rot | *Sclerotium rolfsii* |
| Southern blight | *Athelia rolfsii* |
| Stem rot of tranplants | *Pythium spp.* |
| Target spot | *Rhizoctonia solani* |
| Verticillium wilt | *Verticillium albo-atrum* |
| | *Verticillium dahliae* |

| | **Nematodes, parasitic** |
|---|---|
| Bulb and stem (stem break) | *Ditylenchus dipsaci* |
| Cyst | *Globodera solanacearum* = *Globodera virginiae Globodera tabacum* |
| Dagger, American | *Xiphinema americanum* |
| Foliar | *Aphelenchoides ritzemabosi* |
| Lesion | *Pratylenchus brachyurus Pratylenchus penetrans Pratylenchus spp.* |
| Reniform | *Rotylenchulus reniformis* |
| Root-knot | *Meloidogvne arenaria* |
| | *Meloidogvne hapla* |
| | *Meloidogvne incognita* |
| | *Meloidogvne javanica* |
| Spiral | *Helicotylenchus spp.* |
| Stubby-root | *Paratrichodorus spp. Trichodorus spp.* |
| Stunt | *Merlinius spp.* |
| | *Tylenchorhynchus spp.* |

| **Wheat** | |
|---|---|
| *(Triticum spp.)* | |

| | **Fungal diseases** |
|---|---|
| Alternaria leaf blight | *Alternaria triticina* |
| Anthracnose | *Colletotrichum graminicola Glomerella graminicola* |
| Ascochyta leaf spot | *Ascochyta tritici* |
| Aureobasidium decay | *Microdochium bolleyi* =*Aureobasidium bolleyi* |
| Black head molds = sooty molds | *Alternaria spp.* |
| | *Cladosporium spp.* |
| | *Epicoccum spp.* |
| | *Sporobolomyces spp.* |
| | *Stemphylium spp.* and other genera |
| Cephalosporium stripe | *Hymenula cerealis* = *Cephalosporium gramineum* |
| Common bunt = stinking smut | *Tilletia tritici* = *Tilletia caries* |
| | *Tilletia laevis* = *Tilletia foetida* |
| Common root rot | *Cochliobolus sativus Bipolaris sorokiniana* = *Helminthosporium sativum* |
| Cottony snow mold | *Coprinus psychromorbidus* |
| Crown rot = foot rot, seedling blight, | *Fusarium spp.* |
| dryland root rot | *Fusarium pseudograminearum* |
| | *Gibberella zeae* |
| | *Fusarium graminearum* Group II |
| | *Gibberella avenacea* |
| | *Fusarium avenaceum* |
| | *Fusarium culmorum* |
| Dilophospora leaf spot = twist | *Dilophospora alopecuri* |
| Downy mildew = crazy top | *Sclerophthora macrospora* |
| Dwarf bunt | *Tilletia controversa* |
| Ergot | *Claviceps purpurea* |
| | *Sphacelia segetum* |
| Eyespot = foot rot. strawbreaker | *Tapesia vallundae* |
| | *Ramulispora herpotrichoides* = *Pseudocercosporella herpotrichoides W-pathotype* |
| | *T acuformis* |
| | *Ramulispora acuformis* = *Pseudocercosporella herpotrichoides var. acuformis R-pathotype* |
| False eyespot | *Gibellina cerealis* |
| Flag smut | *Urocystis agropyri* |
| Foot rot = dryland foot rot | *Fusarium spp.* |
| Halo spot | *Pseudoseptoria donacis* = *Selenophoma donacis* |
| Karnal bunt = partial bunt | *Tilletia indica* = *Neovossia indica* |
| Leaf rust = brown rust | *Puccinia triticina Puccinia recondite f.sp. tritici Puccinia tritici-duri* |
| Leptosphaeria leaf spot | *Phaeosphaeria herpotrichoides* = *Leptosphaeria herpotrichoides Stagonospora* sp. |
| Loose smut | *Ustilaso tritici* = *Ustilaeo segetum var. tritici Ustilaso segetum var. nuda Ustilaso segetum var. avenae* |
| Microscopica leaf spot | *Phaeosphaeria microscopica* = *Leptosphaeria microscopica* |
| Phoma spot | *Phoma spp. Phoma glomerata Phoma sorghina* = *Phoma insidiosa* |
| Pink snow mold = Fusarium patch | *Microdochium nivale* = *Fusarium nivale Monographella nivalis* |
| Platyspora leaf spot | *Clathrospora pentamera* = *Platyspora pentamera* |
| Powdery mildew | *Erysiphe graminis f.sp. tritici Blumeria graminis* = *Erysiphe graminis Oidium monilioides* |
| Pythium root rot | *Pythium aphanidermalum* |
| | *Pythium arrhenomanes* |
| | *Pythium graminicola* |
| | *Pythium myriotylum* |
| | *Pythium volutum* |
| Rhizoctonia root rot | *Rhizoctonia solani Thanalephorus cucumeris* |
| Ring spot = Wirrega blotch | *Pyrenophora seminiperda* = *Drechslera campanulata Drechslera wirreganensis* |
| Scab = head blight | *Fusarium spp. Gibberella zeae* |
| | *Fusarium graminearum* Group II |
| | *Gibberella avenacea* |
| | *Fusarium avenaceum* |
| | *Fusarium culmorum* |
| | *Microdochium nivale* = *Fusarium nivale* |
| | *Monographella nivalis* |
| Sclerotinia snow mold = snow scald | *Myriosclerotinia borealis* = *Sclerotinia borealis* |
| Sclerotium wilt (see Southern blight) | *Sclerotium rolfsii* |
| | *Athelia rolfsii* |
| Septoria blotch | *Septoria tritici* |
| | *Mycosphaerella graminicola* |
| Sharp eyespot | *Rhizoctonia cerealis* |
| | *Ceratobasidium cereale* |
| Snow rot | *Pythium spp.* |
| | *Pythium aristosporum* |
| | *Pythium iwayamae* |
| | *Pythium okanoganense* |
| Southern blight = Sclerotium base rot | *Sclerotium rolfsii* |
| | *Athelia rolfsii* |
| Speckled snow mold = gray snow | *Typhula idahoensis* |
| mold or Typhula blight | *Typhula incarnata* |
| | *Typhula ishikariensis* |
| | *Typhula ishikariensis var. canadensis* |
| Spot blotch | *Cochliobolus sativus Bipolaris sorokiniana* = *Helminthosporium sativum* |
| Stagonospora blotch | *Phaeosphaeria avenaria f.sp. triticae* |
| | *Stasonospora avenge f.sp. triticae* = *Septoria avenge f.sp. triticea* |
| | *Phaeosphaeria nodorum* |
| | *Slagonospora nodorum* = *Septoria nodorum* |
| Stem rust = black rust | *Puccinia graminis* = *Puccinia graminis f. sp. tritici* |
| Storage molds | *Aspersillus spp.* |
| | *Penicillium spp.* |
| | and others |
| Stripe rust = yellow rust | *Puccinia striiformis* |
| | *Uredo glumarum* |
| Take-all | *Gaeumannomyces graminis var. tritici* |
| | *Gaeumannomyces graminis var. avenae* |
| Tan spot = yellow leaf spot, red | *Pyrenophora tritici-repentis* |
| smudge | *Drechslera tritici-repentis* |
| Tar spot | *Phyllachora graminis* |
| | *Linochora graminis* |
| Wheat Blast | *Masnaporthe grisea* |
| Zoosporic root rot | *Lagena radicicola* |
| | *Ligniera pilorum* |
| | *Olpidium brassicae* |
| | *Rhizophydium graminis* |

Compositions of the disclosure can be used to treat parasitic infections. In some embodiments of the present disclosure, the compositions can include compounds that are generally regarded as safe (GRAS compounds). In some embodiments of the present disclosure, the compositions can include compounds of a plant origin, such as plant essential oils or monoterpenoids of plant essential oils. In some embodiments of the present disclosure, the compositions include two or more compounds. In some embodiments of the present disclosure, the compositions can include any of the following oils, or mixtures thereof:

| **TABLE B** | | | |
|---|---|---|---|
| t-anethole | allyl sulfide | allyl trisulfide | allyl-disulfide |
| artemisia alcohol acetate | benzaldehyde | benzoic acid | benzyl acetate |
| benzyl alcohol | bergamotene | β-bisabolene | bisabolene oxide |
| α-bisabolol | bisabolol oxide | bisabolol oxide B | bornyl acetate |
| β-bourbonene | black seed oil (BSO) | α-cadinol | camphene |
| α-campholene | α-campholene aldehyde | camphor | carvacrol |
| d-carvone | 1-carvone | caryophyllene oxide | trans-caryophyllene |
| castor oil | cedar oil | chamazulene | 1,8-cineole |
| cinnamaldehyde | cinnamyl alcohol | cinnamon oil | citral A |
| citral B | isopropyl citrate | citronellal | citronella oil |
| citronellol | citronellyl acetate | citronellyl formate | clove oil |
| α-copaene | cornmint oil | corn oil | β-costol |
| cryptone | cumin oil | curzerenone | *p*-cymene |
| davanone | diallyl tetrasulfide | diethyl phthalate | dihydropyrocurzeren one |
| dihydrotagentone | β-elemene | gamma-elemene | Elmol |
| Estragole | 2-ethyl-2-hexen-1-ol | eugenol | eugenol acetate |
| α-farnesene | (Z,E)-α-farnesene | E-β-farnesene | fenchone |
| Furanodiene furanoeudesma-1,3-diene | furanoeudesma-1,4-diene | furano germacra 1,10(15)-diene-6-one | furanosesquiterpene |
| garlic oil | geraniol | geraniol acetate | germacrene D |
| germacrene B | grapefruit oil | α-gurjunene | α-humulene |
| α-ionone | β-ionone | isoborneol | isofuranogermacrene |
| iso-menthone | iso-pulegone | jasmone | lecithin |
| lemon oil | lemon grass oil | lilac flower oil (LFO) | lime oil |
| d-limonene | linalool | linalyl acetate | linalyl anthranilate |
| lindestrene | lindenol | linseed oil | methyl-allyl-trisulfide |
| menthone | 2-methoxy furanodiene | menthyl acetate | menthol |
| menthone | 2-methoxy \|furanodiene | menthyl acetate | methyl cinnamate |
| methyl citrate | methyl di-hydrojasmonate | menthyl salicylate | mineral oil |
| musk ambrette | myrcene | myrtenal | neraldimethyl acetate |
| nerolidol | nonanone | gamma-nonalactone | oil of pennyroyal |
| olive oil | orange sweet oil | 1-octanol | E ocimenone |
| Z ocimenone | 3-octanone | ocimene | octyl acetate |
| peanut oil | perillyl alcohol | peppermint oil | α-phellandrene |
| β-phellandrene | phenethyl proprionate | phenyl acetaldehyde | α-pinene |
| β-pinene | pine oil | trans-pinocarveol | piperonal |
| piperonyl | piperonyl acetate | piperonyl alcohol | piperonyl amine |
| prenal | pulegone | quinine | rosemary oil |
| sabinene | sabinyl acetate | safflower oil | α-santalene |
| santalol | sativen | δ-selinene | sesame oil |
| β-sesquphelandrene | silicone fluid | sodium lauryl sulfate | soybean oil |
| spathulenol | tagetone | tangerine oil | α-terpinene |
| terpinene 900 | α-terpineol | a-terpinolene | gamma-terpineol |
| α-terpinyl acetate | 2-tert-butyl-*p-*quinone | α-thujone | thyme oil |
| thymol | thymyl methyl ether | gamma-undecalactone | valeric anhydride |
| vanillin | trans-verbenol | cis-verbenol | verbenone |
| white mineral oil | yomogi alcohol | zingiberene | |

In other embodiments of the present disclosure, methods can be used to assess or screen the anti-parasitic effect of a particular small molecule other than the essential oils described above. These small molecules can include, for example, any of the following small molecules, or the like, or any other small molecules that include these groups, or different groups of the like. In the following table, the bolded designations indicate generic terms for small molecules sharing particular characteristics, while non-bolded terms following the bolded generic terms indicate individual small molecules within the genus described by the bolded term.

**TABLE B1**

| | | |
|---|---|---|
| **Cumulenes:** | pyridine | **Polycyclic heteroarenes:** |
| butatriene | | |
| **Allenes:** | pyrimidine | isoquinoline |
| buta-1-2-diene | | |
| **Pseudohalogens:** | thiophene | 1H-indole |
| oxalonitrile | | |
| thiocyanogen | selenophene | quinoline |
| selenocyanogen | selenophene | pteridine |
| **Monocyclic heterarnes:** | tellurophene | oxanthrene |
| pyrazole | pyrazine | 2H-isoindole |
| pyridazine | **Functional Classes:** | isochromenylium |
| 1 H-pyrrole | imides | acridine |
| 3H-pyrrole | imines | phthalazine |
| 2H-pyrrole | ethers | cinnoline |
| furan | oximes | quinazoline |
| isoxazole | thiols | quinolizinylium |
| isothiazole | amines | phenazine |
| 1H-arsole | Carboxylic acids | Benzo[g]pyrazine |
| 2H-arsole | Hydroxamic acids | 1-benzazpine |
| 3H-arsole | esters | benzotriazine |
| triazine | quinones | 1H-benzimidazole |
| thiazole | thioketones | **Heteroaryl groups:** |
| imidazole | | 2-thienyl group |
| 3-thienyl group | octaphenylene | ethylbenzene |
| **Arynes:** | acene | *p*-cymene |
| 1-methoxycyclohexa-1,3-dien-5-yne | dibenz[a,h]anthracene | 1-ethyl-2-methlybenzene |
| 2-methoxycyclohexa-1,3-dien-5-yne | helicene | 3-ethyltoluene |
| **Polycyclic Arenes:** | dibenzannulene | cumene |
| fluorene | picene | heptaphene |
| phenanthrene | pentaphene | hexaphenylene |
| biphenylene | tetraphenylene | nonaphene |
| triphenylene | tetranaphthylene | octaphene |
| chrysene | hexaphene | nonaphene |
| tetraphene | trinaphtylene | Teteranphthylene |
| octaphene | dibenzo[a,1]pyrene | phenyl group |
| **Organic Hetro Monocyclic Compounds** | pyrene | biphenyl-4-yl group |
| | benzo [b] fluranthene | Aryl β-D-glucosides: |
| aziridines | | |
| | Monocyclic Arenes: | salcin |
| diazoles | | |
| | benzene | phlorizin |
| pyrrolines | | |
| | diflurobenze | syringin |
| furan | | |
| | thiazole | Alicyclic compounds |
| perylene | | |
| | azetidene | cyclic olefins |
| coronene | | |
| | triazinane | cyclic acetylenes |
| acenaphtylene | | |
| | pentathiepane | benzynes |
| phenalene | | |
| | pentathiepane | alicyclic ketones |
| fluoranthene | | |
| | sec-butylbenzene | penarns |
| acephenanthrylene | | |
| | methylbenzene | cephams |
| pleiadene | | |
| | isobutylbenzene | indolizines |
| ovalene | | |
| | butylbenzene | quinazolines |
| rubicene | | |
| | hexaflurobenzene | pyrazolopyrimidines |
| pyranthrene | | |
| | Aryl groups: | |
| 3-methycholanthrene | | |
| piperazine | arsolane | pyrrolopyrimidines |
| piperidine | tetrazocane | oxazolopyridines |
| pyran | axocane | phthalazine |
| pyridine | diazepane | indazoles |
| pyrrole | diazepane | **Heteroaryl Groups:** |
| oxolane | **Organic Heterobicyclic Compounds:** | 2-thienyl group |
| selenophene | | |
| thiazolidine | benzimidazole | 3-thienyl group |
| tetrazole | benzodiazepine | **Monocyclic Heteroarenes:** |
| triazole | benzopyran | pyrazole |
| oxazole | benzopyrrole | imidazole |
| triazine | isoquinolines | pyridine |
| pyrrolidine | pteridines | thiophene |
| diazolidine | quinolines | selenophene |
| diazine | quinclidines | tellurophene |
| thiazine | quinuclidines | pyrazine |
| oxazolidine | benzofurans | pyridazine |
| arsole | benzazepines | pyrrole |
| tellurophene | imidazopyrimidines | furan |
| isoxazole | Alkanes: | butane |
| oxazole | heptadecane | pentane |
| tetrazole | methane | tetradecane |
| pyrylium | octane | decane |
| arsole | propanes | ethane |
| triazine | 9-crown-3 | 1,4,8,11-tetraazacyclotetradecane |
| thiazoles | 15-crown-5 | |
| triazole | dibenzo-18-crown-6 | 1,4,7-triazonane |
| **Crown Compounds:** | benzo-15-crown-5 | **Alicyclic Compounds:** |
| 18-crown-6 | 1,4,7,10-tetraazacyclododecane | Cycloakanes |
| 12-crowh-4 | | cyclic olefins |
| cyclic | heptacosane | **Hydrocarbylidene** |
| acetylenes | tridecane | **Groups:** |
| benzynes | dotriacontane | alkenylidenes |
| **Organoheteryl** | | allenylidene groups |
| | Carboacyl groups | |
| **Groups:** | | alkylidene groups |
| | **Arylene groups** | |
| Alkyamino groups | | Nucleosidyl groups |
| Alkyloxy groups | Phenylene group | Carboxyl groups |
| Ureido group | **Organyl Groups** | **Carbonyl Group** |
| Oxaloamino group | | Glycoloyl group |
| | Hydrocarbyl groups | |
| dodecane | | **Alkenylidene Groups** |
| | heptane | |
| petadecane | | Oaxalooxy group |
| | icosane | |
| hexane | | Allylic groups |
| | hexadecane | |
| neopentane | | Ethenylidene group |
| | docosane | |
| isopentane | | Oxaloamino group |
| | undecane | |
| isobutanetriacontane | | Benzylic groups |
| | hentriacontane | |
| propane | nonacosane | Allylidene group |
| nonane | | Oxalosulfanyl group |
| | tritriacontane | |
| octadecane | | Organic heterocyclyl |
| | **Elemental Carbon:** | |
| nonadacane | | Acyl **groups** |
| | fullerenes | |
| henicosane | | Silyl groups |
| | monatomic carbon | |
| tricosane | | Vinylic groups |
| | diatomic carbon | |
| teteracosane | | |
| | **Hydrocarbylidyne Group:** | |
| pentacosane | | |
| | methylidyne | |
| Hexacosane farnesane | | |

In some embodiments of the present disclosure, compositions include two or more compounds selected from the following compounds:

| **TABLE C** | |
|---|---|
| **Compounds** | CAS **Registry No.** |
| trans-anethole | 41080-23-8 |
| tert-butyl-*p*-benzoquinone | 3602-55-9 |
| black seed oil | 977017-84-7 |
| borneol | 507-70-0 |
| camphene | 79-92-5 |
| β-caryophyllene | 87-44-5 |
| cineol | 470-82-6 |
| triethyl citrate | 77-93-0 |
| para-cymene | 99-87-6 |
| geraniol | 106-24-1 |
| hedion | 24851-98-7 |
| heliotropine | 120-57-0 |
| hercolyn D | 8050-15-5 |
| lilac flower oil | |
| lime oil | |
| d-limonene | 5989-27-5 |
| linalool | 78-70-6 |
| ethyl linalool | 10339-55-6 |
| tetrahydrolinalool | 78-69-3 |
| methyl salicylate | 119-36-8 |
| α-pinene | 80-56-8 |
| β-Pinene | 127-91-3 |
| α-Terpinene | 99-86-5 |
| α-Thujene | 2867-05-2 |
| thyme oil | 8007-46-3 |
| thymol | 89-83-8 |
| wintergreen oil | 68-917-75-9 |

In some embodiments of the compositions of the present disclosure that include lilac flower oil, one or more of the following compounds can be substituted for the lilac flower oil: tetrahydrolinalool; ethyl linalool; heliotropine; hedion; hercolyn D; and triethyl citrate.

In some embodiments of the compositions of the present disclosure that include black seed oil, one or more of the following compounds can be substituted for the black seed oil: α-thujene, α-pinene, β-pinene, *p*-cymene, limonene, and tert-butyl-p-benzoquinone.

In some embodiments of the compositions of the present disclosure that include thyme oil, one or more of the following compounds can be substituted for the thyme oil: thymol, α-thujone; α-pinene, camphene, β-pinene, *p*-cymene, α-terpinene, linalool, borneol, and β-caryophyllene. In some embodiments of the compositions of the present disclosure that include thymol, thyme oil can be substituted. In some embodiments of the compositions of the present disclosure that include thyme oil, it can be desirable to include a specific type of thyme oil. In this regard, thyme oil (white) is preferred to thyme oil (red) because the latter has been found to cause negative side effects for the subject or host.

Compounds used to prepare embodiments of the compositions of the present disclosure can be obtained, for example, from the following sources: Millennium Chemicals, Inc. (Jacksonville, FL), Ungerer Company (Lincoln Park, NJ), SAFC (Milwaukee, WI), IFF Inc. (Hazlet, NJ); Sigma Chemical Co. (St. Louis, MO); and The Lebermuth Company, Inc. (Southbend, IN).

In some embodiments of the compositions of the present disclosure, it can be desirable to include a naturally-occurring version or a synthetic version of a compound. For example, in certain embodiments it can be desirable to include Lime Oil 410, a synthetic lime oil that can be obtained, for example, from Millennium Chemicals, Inc. In certain exemplary compositions, it can be desirable to include a compound that is designated as meeting Food Chemical Codex (FCC), for example, geraniol Fine FCC or Tetrahydrolinalool FCC, which compounds can be obtained, for example, from Millennium Chemicals, Inc.

In some embodiments of the compositions of the present disclosure, it can be desirable to include a compound having a specific purity. In some embodiments of the compositions of the present disclosure, it can be desirable to include compounds each having a purity of at least about 80%, 81%, 82%, 83%, 84%, 85%, 86%, 87%, 88%, 89%, 90%, 91%, 92%, 93%, 94%, 95%, 96%, 97%, 98%, or 99%. For example, in some embodiments of the compositions of the present disclosure including α-pinene, an α-pinene that is at least about 98% pure can be selected. For another example, in embodiments of the compositions of the present disclosure including linalool, a linalool that is at least about 97-99% pure (e.g., linalool Coeur) can be selected.

In some embodiments of the compositions of the present disclosure, it can be desirable to include compounds each having a purity of about 60%, 65%, 70%, 75%, 80%, 85%, 90%, or 95%. For example, in some embodiments of the compositions of the present disclosure that include geraniol, it can be desirable to include a geraniol that is at least about 60%, 85% or 95% pure. In some embodiments of the present disclosure, it can be desirable to include a specific type of geraniol. For example, in some embodiments of the present disclosure, the compositions can include: geraniol 60, geraniol 85, or geraniol 95. When geraniol is obtained as geraniol 60, geraniol 85, or geraniol 95, then forty percent, fifteen percent, or five percent of the oil can be Nerol. Nerol is a monoterpene (C₁₀H₁₈O), which can be extracted from attar of roses, oil of orange blossoms, and oil of lavender.

In some embodiments of the present disclosure, compositions include two or more compounds selected from the following compounds: linalool, thymol, α-pinene, para-cymene, and trans-anethole. In some embodiments of the present disclosure, compositions include three or more compounds selected from the following compounds: linalool, thymol, α-pinene, para-cymene, and Trans-Anethole. In some embodiments of the present disclosure, compositions include four or more compounds selected from the following compounds: linalool, thymol, α-pinene, para-cymene, and Trans-Anethole. In some embodiments of the present disclosure, compositions include: linalool, thymol, α-pinene, para-cymene, and Trans-Anethole. In some embodiments of the present disclosure, it is preferred that an α-pinene that is at least about 98% pure is used. In some embodiments of the present disclosure, it is preferred that a linalool that is a linalool coeur is used. In some embodiments of the present disclosure, the composition can further include soy bean oil.

In some embodiments of the present disclosure, compositions include two or more compounds selected from the following compounds: linalool, thymol, α-pinene, and para-cymene. In some embodiments of the present disclosure, compositions include three or more compounds selected from the following compounds: linalool, thymol, α-pinene, and para-cymene. In some embodiments of the present disclosure, compositions include: linalool, thymol, α-pinene, and para-cymene. In some embodiments of the present disclosure, it is preferred that an α-pinene that is at least about 98% pure is used. In some embodiments of the present disclosure, it is preferred that a linalool that is a linalool coeur is used. In some embodiments of the present disclosure, the composition can further include soy bean oil.

In some embodiments of the present disclosure, each compound can make up between about 1% to about 99%, by weight (wt/wt %) or by volume (vol/vol %), of the composition. For example, composition can comprises about 1% α-pinene and about 99% thymol. As used herein, % amounts, by weight or by volume, of compounds are to be understood as referring to relative amounts of the compounds. As such, for example, a composition including 7% linalool, 35% thymol, 4% α-pinene, 30% para-cymene, and 24% soy bean oil (vol/vol %) can be said to include a ratio of 7 to 35 to 4 to 30 to 24 linalool, thymol, α-pinene, para-cymene, and soy bean oil, respectively (by volume). As such, if one compound is removed from the composition, or additional compounds or other ingredients are added to the composition, it is contemplated that the remaining compounds can be provided in the same relative amounts. For example, if soy bean oil was removed from the exemplary composition, the resulting composition would include 7 to 35 to 4 to 40 linalool, thymol, α-pinene, and para-cymene, respectively (by volume). This resulting composition would include 9.21% linalool, 46.05% thymol, 5.26% α-pinene, and 39.48% para-cymene (vol/vol %). For another example, if safflower oil was added to the original composition to yield a final composition containing 40% (vol/vol) safflower oil, then the resulting composition would include 4.2% linalool, 21% thymol, 2.4% α-pinene, 18% para-cymene, 14.4% soy bean oil, and 40% safflower oil (vol/vol %).

In some embodiments of the present disclosure, the composition includes about 1-5%, about 5-10%, about 10-15%, about 15-20%, about 20-25%, about 25-30%, about 30-35%, about 35-40%, about 40- 45%, about 45-50%, about 50-60%, about 60-75%, or about 75-99% linalool, as measured by volume (vol/vol %). In some embodiments of the present disclosure, the composition includes about 4.5 -5.5% linalool, as measured by volume. In some embodiments of the present disclosure, the composition includes about 5% linalool, as measured by volume. In some embodiments of the present disclosure, the composition includes about 6.5-7.5% linalool, as measured by volume. In some embodiments of the present disclosure, the composition includes about 7% linalool, as measured by volume. In some embodiments of the present disclosure, the composition includes about 38-40% linalool, as measured by volume. In some embodiments of the present disclosure, the composition includes about 39% linalool, as measured by volume.

In some embodiments of the present disclosure, the composition includes about 1-5%, about 5-10%, about 10-15%, about 15-20%, about 20-25%, about 25-30%, about 30-35%, about 35-40%, about 40- 45%, about 45-50%, about 50-60%, about 60-75%, or about 75-99% linalool, as measured by weight (wt/wt %). In some embodiments of the present disclosure, the composition includes about 4.2-5.2% linalool, as measured by weight. In some embodiments of the present disclosure, the composition includes about 4.7% linalool, as measured by weight. In some embodiments of the present disclosure, the composition includes about 6.1-7.1% linalool, as measured by weight. In some embodiments of the present disclosure, the composition includes about 6.6% linalool, as measured by weight. In some embodiments of the present disclosure, the composition includes about 40.3-41.3% linalool, as measured by weight. In some embodiments of the present disclosure, the composition includes about 40.8% linalool, as measured by weight.

In some embodiments of the present disclosure, the composition includes about 1-5%, about 5-10%, about 10-15%, about 15-20%, about 20-25%, about 25-30%, about 30-35%, about 35-40%, about 40- 45%, about 45-50%, about 50-60%, about 60-75%, or about 75-99% thymol, as measured by volume (vol/vol %). In some embodiments of the present disclosure, the composition includes about 38-40% thymol, as measured by volume. In some embodiments of the present disclosure, the composition includes about 39% thymol, as measured by volume. In some embodiments of the present disclosure, the composition includes about 36-38% thymol, as measured by volume. In some embodiments of the present disclosure, the composition includes about 37% thymol, as measured by volume. In some embodiments of the present disclosure, the composition includes about 34-36% thymol, as measured by volume. In some embodiments of the present disclosure, the composition includes about 35% thymol, as measured by volume.

In some embodiments of the present disclosure, the composition includes about 1-5%, about 5-10%, about 10-15%, about 15-20%, about 20-25%, about 25-30%, about 30-35%, about 35-40%, about 40- 45%, about 45-50%, about 50-60%, about 60-75%, or about 75-99% thymol, as measured by weight (wt/wt %). In some embodiments of the present disclosure, the composition includes about 40.3-41.3% thymol, as measured by weight. In some embodiments of the present disclosure, the composition includes about 40.8% thymol, as measured by weight. In some embodiments of the present disclosure, the composition includes about 33.9-34.9% thymol, as measured by weight. In some embodiments of the present disclosure, the composition includes about 34.4% thymol, as measured by weight. In some embodiments, the composition includes about 36.7-37.7% thymol, as measured by weight. In some embodiments of the present disclosure, the composition includes about 37.2% thymol, as measured by weight.

In some embodiments of the present disclosure, the composition includes about 1-5%, about 5-10%, about 10-15%, about 15-20%, about 20-25%, about 25-30%, about 30-35%, about 35-40%, about 40- 45%, about 45-50%, about 50-60%, about 60-75%, or about 75-99% α-pinene, as measured by volume (vol/vol %). In some embodiments of the present disclosure, the composition includes about 1.5-2.5% α-pinene, as measured by volume. In some embodiments of the present disclosure, the composition includes about 2% α-pinene, as measured by volume. In some embodiments of the present disclosure, the composition includes about 4.5-5.5% α-pinene, as measured by volume. In some embodiments of the present disclosure, the composition includes about 5% α-pinene, as measured by volume. In some embodiments of the present disclosure, the composition includes about 3.5-4.5% α-pinene, as measured by volume. In some embodiments of the present disclosure, the composition includes about 4% α-pinene, as measured by volume.

In some embodiments of the present disclosure, the composition includes about 1-5%, about 5-10%, about 10-15%, about 15-20%, about 20-25%, about 25-30%, about 30-35%, about 35-40%, about 40- 45%, about 45-50%, about 50-60%, about 60-75%, or about 75-99% α-pinene, as measured by weight (wt/wt %). In some embodiments of the present disclosure, the composition includes about 1.4-2.4% α-pinene, as measured by weight. In some embodiments of the present disclosure, the composition includes about 1.9% α-pinene, as measured by weight. In some embodiments of the present disclosure, the composition includes about 4.2-5.2% α-pinene, as measured by weight. In some embodiments of the present disclosure, the composition includes about 4.7% α-pinene, as measured by weight. In some embodiments of the present disclosure, the composition includes about 3.3-4.3% α-pinene, as measured by weight. In some embodiments of the present disclosure, the composition includes about 3.8% α-pinene, as measured by weight.

In some embodiments of the present disclosure, the composition includes about 1-5%, about 5-10%, about 10-15%, about 15-20%, about 20-25%, about 25-30%, about 30-35%, about 35-40%, about 40-45%, about 45-50%, about 50-60%, about 60-75%, or about 75-99% para-cymene, as measured by volume (vol/vol %). In some embodiments of the present disclosure, the composition includes about 36.5-37.5% para-cymene, as measured by volume. In some embodiments of the present disclosure, the composition includes about 37% para-cymene, as measured by volume. In some embodiments of the present disclosure, the composition includes about 29.5- 30.5% para-cymene, as measured by volume. In some embodiments of the present disclosure, the composition includes about 30% para-cymene, as measured by volume. In some embodiments of the present disclosure, the composition includes about 1.5-2.5% para-cymene, as measured by volume. In some embodiments of the present disclosure, the composition includes about 2% para-cymene, as measured by volume.

In some embodiments of the present disclosure, the composition includes about 1-5%, about 5-10%, about 10-15%, about 15-20%, about 20-25%, about 25-30%, about 30-35%, about 35-40%, about 40- 45%, about 45-50%, about 50-60%, about 60-75%, or about 75-99% para-cymene, as measured by weight (wt/wt %). In some embodiments of the present disclosure, the composition includes about 33.9-34.9% para-cymene, as measured by weight. In some embodiments of the present disclosure, the composition includes about 34.4% para-cymene, as measured by weight. In some embodiments of the present disclosure, the composition includes about 1.4- 2.4% para-cymene, as measured by weight. In some embodiments of the present disclosure, the composition includes about 1.9% para-cymene, as measured by weight. In some embodiments of the present disclosure, the composition includes about 27.9-28.9% para-cymene, as measured by weight. In some embodiments of the present disclosure, the composition includes about 28.4% para-cymene, as measured by weight.

In some embodiments of the present disclosure, the composition includes about 1-5%, about 5-10%, about 10-15%, about 15-20%, about 20-25%, about 25-30%, about 30-35%, about 35-40%, about 40- 45%, about 45-50%, about 50-60%, about 60-75%, or about 75-99% trans-anethole, as measured by volume (vol/vol %). In some embodiments of the present disclosure, the composition includes about 16.5-17.5% trans-anethole, as measured by volume. In some embodiments of the present disclosure, the composition includes about 17% trans-anethole, as measured by volume.

In some embodiments of the present disclosure, the composition includes about 1-5%, about 5-10%, about 10-15%, about 15-20%, about 20-25%, about 25-30%, about 30-35%, about 35-40%, about 40- 45%, about 45-50%, about 50-60%, about 60-75%, or about 75-99% trans-anethole, as measured by weight (wt/wt %). In some embodiments of the present disclosure, the composition includes about 17.7-18.7% trans-anethole, as measured by weight. In some embodiments of the present disclosure, the composition includes about 18.2% trans-anethole, as measured by weight.

In some embodiments of the present disclosure, the composition includes the following compounds in the following relative amounts, where the relative amounts of the compounds are expressed as % wt/wt: 15-25% trans-anethole, 30-40% para-cymene, 1-10% linalool, 1-10% α-pinene, and 35-45% thymol. In some embodiments of the present disclosure, the composition includes the following compounds in the following relative amounts, where the relative amounts of the compounds are expressed as % wt/wt: 18.2% trans-anethole, 34.4% para-cymene, 4.7% linalool, 1.9% α-pinene, and 40.8% thymol.

In some embodiments of the present disclosure, the composition includes the following compounds in the following relative amounts, where the relative amounts of the compounds are expressed as % vol/vol: 10-20% trans-anethole, 30-40% para-cymene, 1-10% linalool, 1-10% α-pinene, and 35-45% thymol. In some embodiments of the present disclosure, the composition includes the following compounds in the following relative amounts, where the relative amounts of the compounds are expressed as % vol/vol: 17% trans-anethole, 37% para-cymene, 5% linalool, 2% α-pinene, and 39% thymol.

In some embodiments of the present disclosure, the composition includes the following compounds in the following relative amounts, where the relative amounts of the compounds are expressed as % wt/wt: 15-25% trans-anethole, 1-10% para-cymene, 35-45% linalool, 1-10% α-pinene, and 30-40% thymol. In some embodiments of the present disclosure, the composition includes the following compounds in the following relative amounts, where the relative amounts of the compounds are expressed as % wt/wt: 18.2% trans-anethole, 1.9% para-cymene, 40.8% linalool, 4.7% α-pinene, and 34.4% thymol.

In some embodiments of the present disclosure, the composition includes the following compounds in the following relative amounts, where the relative amounts of the compounds are expressed as % vol/vol: 15-25% trans-anethole, 1-10% para-cymene, 35-45% linalool, 1-10% α-pinene, and 30-40% thymol. In some embodiments of the present disclosure, the composition includes the following compounds in the following relative amounts, where the relative amounts of the compounds are expressed as % vol/vol: 17% trans-anethole, 2% para-cymene, 39% linalool, 5% α-pinene, and 37% thymol.

In some embodiments of the present disclosure, the composition includes the following compounds in the following relative amounts, where the relative amounts of the compounds are expressed as % wt/wt: 25-35% para-cymene, 1-10% linalool, 1-10% α-pinene, 20-30% soy bean oil, and 35-45% thymol. In some embodiments of the present disclosure, the composition includes the following compounds in the following relative amounts, where the relative amounts of the compounds are expressed as % wt/wt: 28.39% para-cymene, 6.6% linalool, 3.8% α-pinene, 24% soy bean oil, and 37.2% thymol.

In some embodiments of the present disclosure, the composition includes the following compounds in the following relative amounts, where the relative amounts of the compounds are expressed as % vol/vol: 25-35% para-cymene, 1-10% linalool, 1-10% α-pinene, 20-30% soy bean oil, and 35-45% thymol. In some embodiments of the present disclosure, the composition includes the following compounds in the following relative amounts, where the relative amounts of the compounds are expressed as % vol/vol: 30% para-cymene, 7% linalool, 4% α-pinene, 24% soy bean oil, and 35% thymol.

In some embodiments of the present disclosure, the composition can include, for example, any of the following compounds from Table D, or active components of any of the compositions listed as "blends" in Table E, or the like:

**TABLE D: COMPOUNDS**

| | | | | |
|---|---|---|---|---|
| t-anethole | allyl sulfide | allyl trisulfide | allyl-disulfide | artemisia alcohol acetate |
| benzaldehyde | benzoic acid | benzyl acetate | benzyl alcohol | bergamotene |
| 3-bisabolene | bisabolene oxide | a-bisabolol | bisabolol oxide | bisobolol oxide 3 |
| bornyl acetate | 3-bourbonene | black seed oil (BSO) | a-cadinol | camphene |
| a-campholene | a-campholene aldehyde | camphor | carvacrol | d-carvone |
| 1-carvone | caryophyllene oxide | trans-caryophyllene | corn oil | 3-costol |
| cryptone | cumin oil | curzerenone | *p*-cymene | davanone |
| diallyl tetrasulfide | diethyl phthalate | dihydropyrocurzere none | dihydrotagentone | β-elemene |
| gamma-elemene | Elmol | Estragole | 2-ethyl-2-hexen-1-ol | eugenol |
| eugenol acetate | a-farnesene | (Z,E)-a-farnesene | E-*p*-farnesene | fenchone |
| furanodiene | furanoeudesma- | 1,3-diene | furanoeudesma-1,4-diene | furano germacra |
| lilac flower oil (LFO) | lime oil | d-limonene | linalool | 1,10(15)-diene-6-linalyl acetate |
| linalyl anthranilate | lindestrene | lindenol | linseed oil | methyl-allyl-trisulfide |
| menthol | menthone | 2-methoxy furanodiene | menthyl acetate | methyl cinnamate |
| methyl citrate | methyl di-hydrojasmonat e | menthyl salicylate | mineral oil | musk ambrette |
| myrcene | myrtenal | neraldimethyl acetate | nerolidol | nonanone |
| gamma-nonalactone | piperonal | piperonyl | piperonyl acetate | Piperonyl alcohol |
| piperonyl amine | prenal pulegone | quinine rosemary | oil sabinene | sabinyl acetate |
| safflower oil | a-santalene | santalol sativen | 5-selinene | sesame oil |
| *P-*sesquphelandrene | silicone fluid | sodium lauryl sulfate | soybean oil | spathulenol |
| tagetone | tangerine oil | a-terpinene | terpinene 900 | a-terpineol |
| a-terpinolene | anise oil | *p*-cymene | amyl butyrate | eucalyptus oil |
| geraniol oil | castor oil cedar | oil chamazulene | 1,8-cineole | cinnamaldehy de |
| cinnamyl alcohol | cinnamon oil | citral A citral B | isopropyl citrate | citronellal |
| citronella oil | citronellol | citronellyl acetate | citronellyl formate | clove oil |
| a-copaene | cornmint oil | germacrene D | furanosesquiterpe ne | garlic oil |
| geraniol | geraniol acetate | a-ionone | germacrene B | grapefruit oil |
| a-gurjunene | a-humulene | iso-pulegone | β-ionone | isoborneol |
| isofuranogermacre ne | iso-menthone | oil of pennyroyal | jasmone | lecithin |
| lemon oil | lemon grass oil | Z ocimenone | olive oil | orange sweet oil |
| 1 -octanol | E ocimenone | perillyl alcohol | 3-octanone | ocimene |
| octyl acetate | peanut oil | phenyl acetaldehyde | peppermint oil | a-phellandrene |
| *P*-phellandrene | phenethyl proprionate | gamma-terpineol | a-pinene | *P*-pinene |
| pine oil | trans-pinocarveol | thymol | a-terpinyl acetate | 2-tert-butyl-*p-*quinone |
| a-thujone | thyme oil | trans-verbenol | thymyl methyl ether | gamma-undecalactone |
| valeric anhydride | vanillin | cis-verbenol | verbenone | white mineral oil |
| yomogi alcohol | zingiberene | | | |

| **TABLE E: BLENDS** | | | |
|---|---|---|---|
| | **Compounds** | CAS **Registry Number** | **Wt/Wt** |
| **Blend 1** | Lilac Flower Oil (LFO) | | 4.40% |
| | D-Limonene | 5989-27-5 | 82.30% |
| | Thyme Oil White | 8007-46-3 | 3.30% |
| | Blend 105 | | 10.00% |
| | | | |
| **Blend 2** | D-Limonene | 5989-27-5 | 82.52% |
| | Thyme Oil White | 8007-46-3 | 3.28% |
| | Linalool Coeur | 78-70-6 | 0.57% |
| | Tetrahydrolinalool | 78-69-3 | 0.78% |
| | Vanillin | 121-33-5 | 0.05% |
| | Isopropyl myristate | 110-27-0 | 0.80% |
| | Piperonal (aldehyde) [Heliotropine] | 120-57-0 | 0.80% |
| | Blend 106 | | 9.99% |
| | Geraniol Fine FCC | 106-24-1 | 0.41% |
| | Triethyl Citrate | 77-93-0 | 0.80% |
| | | | |
| **Blend 3** | D-Limonene | 5989-27-5 | 82.44% |
| | Thyme Oil White | 8007-46-3 | 3.28% |
| | Blend 106 | | 10.07% |
| | Blend 103 | | 4.21% |
| | | | |
| **Blend 4** | LFO | | 79.50% |
| | BSO | 977017-84-7 | 21.50% |
| | | | |
| | | | |
| **Blend 5** | BSO | 977017-84-7 | 21.50% |
| | Linalool Coeur | 78-70-6 | 15.90% |
| | Tetrahydrolinalool | 78-69-3 | 19.00% |
| | Vanillin | 121-33-5 | 1.80% |
| | Isopropyl myristate | 110-27-0 | 23.50% |
| | Piperonal (aldehyde) [Heliotropine] | 120-57-0 | 7.80% |
| | Geraniol Fine FCC | 106-24-1 | 10.50% |
| | | | |
| **Blend 6** | D-Limonene | 5989-27-5 | 8.80% |
| | BSO | 977017-84-7 | 26.20% |
| | Linalool Coeur | 78-70-6 | 6.40% |
| | Tetrahydrolinalool | 78-69-3 | 7.80% |
| | Vanillin | 121-33-5 | 0.80% |
| | Isopropyl myristate | 110-27-0 | 9.50% |
| | Piperonal (aldehyde) [Heliotropine] | 120-57-0 | 3.20% |
| | Geraniol Fine FCC | 106-24-1 | 4.30% |
| | Methyl Salicylate 98% Nat | 119-36-8 | 33.00% |
| | | | |
| **Blend 7** | Thyme Oil White | 8007-46-3 | 20.50% |
| | Wintergreen Oil | 68917-75-9 | 45.00% |
| | Vanillin | 121-33-5 | 1.10% |
| | Isopropyl myristate | 110-27-0 | 33.40% |
| | | | |
| **Blend 8** | D-Limonene | 5989-27-5 | 56.30% |
| | Thyme Oil White | 8007-46-3 | 12.38% |
| | Wintergreen Oil | 68917-75-9 | 31.32% |
| | | | |
| **Blend 9** | D-Limonene | 5989-27-5 | 56.30% |
| | Thyme Oil White | 8007-46-3 | 12.38% |
| | Wintergreen Oil | | 31.32% |
| | | | |
| **Blend 10** | LFO | | 12.94% |
| | D-Limonene | 5989-27-5 | 8.72% |
| | Thyme Oil White | 8007-46-3 | 9.58% |
| | Blend 105 | | 68.76% |
| | | | |
| **Blend 11** | LFO | | 12.94% |
| | D-Limonene | 5989-27-5 | 42.12% |
| | Thyme Oil White | 8007-46-3 | 9.58% |
| | Linalool Coeur | 78-70-6 | 0.84% |
| | Citral | 5392-40-5 | 7.02% |
| | gamma-terpinene | 99-85-4 | 7.23% |
| | A-Pinene, 98% | 80-56-8 | 1.33% |
| | α-Terpineol | 98-55-5 | 4.68% |
| | Terpinolene | 586-62-9 | 4.33% |
| | Para-Cymene | 99-87-6 | 1.11% |
| | Linalyl Acetate | 115-95-7 | 1.79% |
| | B Pinene | 127-91-3 | 1.93% |
| | Camphor Dextro | 464-49-3 | 0.09% |
| | Terpinene 4 OL | 562-74-3 | 0.08% |
| | A Terpinene | 99-86-5 | 1.93% |
| | Borneol L | 507-70-0 | 0.89% |
| | Camphene | 79-92-5 | 0.37% |
| | Decanal | 112-31-2 | 0.12% |
| | Dodecanal | 112-54-9 | 0.10% |
| | Fenchol A | 512-13-0 | 0.01% |
| | Geranyl Acetate | 105-87-3 | 0.12% |
| | Isoborneol | 124-76-5 | 0.28% |
| | 2-Methyl 1,3-cyclohexadiene | 30640-46-1, 1888-90-0 | 0.26% |
| | Myrcene | 123-35-3 | 0.78% |
| | Nonanal | 124-19-6 | 0.02% |
| | Octanal | 124-13-0 | 0.04% |
| | Tocopherol Gamma (TENOX®) | 54-28-4 | 0.02% |
| | | | |
| **Blend 12** | D-Limonene | 5989-27-5 | 9.70% |
| | Thyme Oil White | 8007-46-3 | 8.54% |
| | Blend 105 | | 69.41% |
| | Linalool Coeur | 78-70-6 | 1.66% |
| | Tetrahydrolinalool | 78-69-3 | 2.29% |
| | Vanillin | 121-33-5 | 0.15% |
| | Isopropyl myristate | 110-27-0 | 2.35% |
| | Piperonal (aldehyde) [Heliotropine] | 120-57-0 | 2.35% |
| | Geraniol Fine FCC | 106-24-1 | 1.21% |
| | Triethyl Citrate | 77-93-0 | 2.35% |
| | | | |
| **Blend 13** | LFO | | 80.09% |
| | BSO | 977017-84-7 | 19.91% |
| | | | |
| | | | |
| **Blend 14** | LFO | | 50.13% |
| | BSO | 977017-84-7 | 49.87% |
| **Blend 15** | Thyme Oil White | 8007-46-3 | 4.60% |
| | Wintergreen Oil | 68917-75-9 | 57.80% |
| | Isopropyl myristate | 110-27-0 | 37.60% |
| | | | |
| **Blend 16** | D-Limonene | 5989-27-5 | 28.24% |
| | Thyme Oil White | 8007-46-3 | 4.44% |
| | Wintergreen Oil | 68917-75-9 | 67.32% |
| | | | |
| **Blend 17** | D-Limonene | 5989-27-5 | 9.90% |
| | Linalool Coeur | 78-70-6 | 14.14% |
| | Tetrahydrolinalool | 78-69-3 | 24.29% |
| | Vanillin | 121-33-5 | 2.48% |
| | Isopropyl myristate | 110-27-0 | 28.92% |
| | Piperonal (aldehyde)[Heliotropine] | 120-57-0 | 9.97% |
| | Geraniol Fine FCC | 106-24-1 | 10.30% |
| | | | |
| **Blend 18** | D-Limonene | 5989-27-5 | 9.90% |
| | Linalool Coeur | 78-70-6 | 14.14% |
| | Tetrahydrolinalool | 78-69-3 | 24.29% |
| | Vanillin | 121-33-5 | 2.48% |
| | Isopropyl myristate | 110-27-0 | 28.92% |
| | Piperonal (aldehyde)[Heliotropine] | 120-57-0 | 9.97% |
| | Geraniol Fine FCC | 106-24-1 | 10.30% |
| | | | |
| **Blend 19** | D-Limonene | 5989-27-5 | 9.90% |
| | Geraniol Fine FCC | 106-24-1 | 10.30% |
| | Blend 101 | | 79.80% |
| | | | |
| **Blend 20** | D-Limonene | 5989-27-5 | 9.89% |
| | Blend 112 | | 90.11% |
| | | | |
| | | | |
| **Blend 21** | D-Limonene | 5989-27-5 | 9.89% |
| | Linalool Coeur | 78-70-6 | 17.35% |
| | Tetrahydrolinalool | 78-69-3 | 20.89% |
| | Vanillin | 121-33-5 | 1.12% |
| | Isopropyl myristate | 110-27-0 | 20.64% |
| | Piperonal (aldehyde)[Heliotropine] | 120-57-0 | 21.45% |
| | Piperonyl Alcohol | 495-76-1 | 8.66% |
| | | | |
| **Blend 22** | D-Limonene | 5989-27-5 | 9.30% |
| | BSO | 977017-84-7 | 31.92% |
| | Linalool Coeur | 78-70-6 | 9.48% |
| | Tetrahydrolinalool | 78-69-3 | 11.40% |
| | Vanillin | 121-33-5 | 1.16% |
| | Isopropyl myristate | 110-27-0 | 14.04% |
| | Piperonal (aldehyde)[Heliotropine] | 120-57-0 | 4.68% |
| | Geraniol Fine FCC | 106-24-1 | 6.29% |
| | Methyl Salicylate 98% Nat | 119-36-8 | 11.72% |
| | | | |
| **Blend 23** | D-Limonene | 5989-27-5 | 9.63% |
| | BSO | 977017-84-7 | 26.66% |
| | Linalool Coeur | 78-70-6 | 9.82% |
| | Tetrahydrolinalool | 78-69-3 | 11.81% |
| | Vanillin | 121-33-5 | 1.20% |
| | Mineral Oil White (USP) | 8042-47-5 | 14.97% |
| | Isopropyl myristate | 110-27-0 | 14.54% |
| | Piperonal (aldehyde)[Heliotropine] | 120-57-0 | 4.85% |
| | Geraniol Fine FCC | 106-24-1 | 6.51% |
| | | | |
| **Blend 24** | BSO | 977017-84-7 | 52.28% |
| | Linalool Coeur | 78-70-6 | 9.63% |
| | Tetrahydrolinalool | 78-69-3 | 11.57% |
| | Vanillin | 121-33-5 | 1.12% |
| | Isopropyl myristate | 110-27-0 | 14.26% |
| | Piperonal (aldehyde)[Heliotropine] | 120-57-0 | 4.75% |
| | Geraniol Fine FCC | 106-24-1 | 6.38% |
| | | | |
| **Blend 25** | Thyme Oil White | 8007-46-3 | 38.21% |
| | Wintergreen Oil | 68917-75-9 | 24.79% |
| | Vanillin | 121-33-5 | 1.11% |
| | Isopropyl myristate | 110-27-0 | 35.89% |
| | | | |
| **Blend 26** | Thyme Oil White | 8007-46-3 | 39.24% |
| | Wintergreen Oil | 68917-75-9 | 24.82% |
| | Isopropyl myristate | 110-27-0 | 35.94% |
| | | | |
| **Blend 27** | Thyme Oil White | 8007-46-3 | 39.24% |
| | Isopropyl myristate | 110-27-0 | 35.94% |
| | Wintergreen Oil | | 24.82% |
| | | | |
| **Blend 28** | Thyme Oil White | 8007-46-3 | 39.24% |
| | Isopropyl myristate | 110-27-0 | 35.94% |
| | Wintergreen Oil | | 24.82% |
| | | | |
| **Blend 29** | D-Limonene | 5989-27-5 | 14.8% |
| | Linalool Coeur | 78-70-6 | 2.9% |
| | Tetrahydrolinalool | 78-69-3 | 3.5% |
| | Vanillin | 121-33-5 | 0.2% |
| | Isopropyl myristate | 110-27-0 | 3.4% |
| | Piperonal (aldehyde)[Heliotropine] | 120-57-0 | 3.6% |
| | Piperonyl Alcohol | 495-76-1 | 1.4% |
| | Blend 106 | | 70.2% |
| | | | |
| **Blend 30** | D-Limonene | 5989-27-5 | 69.8% |
| | Linalool Coeur | 78-70-6 | 2.9% |
| | Tetrahydrolinalool | 78-69-3 | 3.5% |
| | Vanillin | 121-33-5 | 0.2% |
| | Isopropyl myristate | 110-27-0 | 3.4% |
| | Piperonal (aldehyde)[Heliotropine] | 120-57-0 | 3.6% |
| | Piperonyl Alcohol | 495-76-1 | 1.4% |
| | Blend 106 | | 15.2% |
| | | | |
| **Blend 31** | Linalool Coeur | 78-70-6 | 5.7% |
| | Tetrahydrolinalool | 78-69-3 | 6.9% |
| | Vanillin | 121-33-5 | 0.4% |
| | Isopropyl myristate | 110-27-0 | 6.8% |
| | Piperonal (aldehyde)[Heliotropine] | 120-57-0 | 7.1% |
| | Piperonyl Alcohol | 495-76-1 | 2.9% |
| | Blend 106 | | 70.2% |
| | | | |
| **Blend 32** | LFO | | 41.4% |
| | D-Limonene | 5989-27-5 | 27.9% |
| | Thyme Oil White | 8007-46-3 | 30.7% |
| | | | |
| **Blend 33** | D-Limonene | 5989-27-5 | 28.461% |
| | Thyme Oil White | 8007-46-3 | 31.294% |
| | Blend 103 | | 40.245% |
| | | | |
| **Blend 34** | D-Limonene | 5989-27-5 | 27.4% |
| | Thyme Oil White | 8007-46-3 | 30.1% |
| | Linalool Coeur | 78-70-6 | 5.7% |
| | Tetrahydrolinalool | 78-69-3 | 7.9% |
| | Vanillin | 121-33-5 | 0.5% |
| | Isopropyl myristate | 110-27-0 | 8.1% |
| | Piperonal (aldehyde)[Heliotropine] | 120-57-0 | 8.1% |
| | Geraniol Fine FCC | 106-24-1 | 4.2% |
| | Triethyl Citrate | 77-93-0 | 8.1% |
| | | | |
| **Blend 35** | LFO | | 42.57% |
| | D-Limonene | 5989-27-5 | 27.35% |
| | Thyme Oil White | 8007-46-3 | 30.08% |
| | | | |
| **Blend 36** | Phenyl Ethyl Propionate | | 36.30% |
| | Methyl Salicylate | | 36.15% |
| | Blend 78 | | 27.55% |
| | | | |
| **Blend 37** | D-Limonene | 5989-27-5 | 4.05% |
| | Thyme Oil White | 8007-46-3 | 4.45% |
| | Benzyl Alcohol | 100-51-6 | 16.71% |
| | Isopar M | 64742-47-8 | 21.09% |
| | Water | 7732-18-5 | 44.78% |
| | Blend 103 | | 5.73% |
| | Stock 10% SLS Solution | | 3.20% |
| | | | |
| **Blend 38** | D-Limonene | 5989-27-5 | 4.03% |
| | Thyme Oil White | 8007-46-3 | 4.43% |
| | Linalool Coeur | 78-70-6 | 0.84% |
| | Tetrahydrolinalool | 78-69-3 | 1.16% |
| | Vanillin | 121-33-5 | 0.07% |
| | Isopropyl myristate | 110-27-0 | 1.19% |
| | Piperonal (aldehyde)[Heliotropine] | 120-57-0 | 1.19% |
| | Geraniol Fine FCC | 106-24-1 | 0.62% |
| | Triethyl Citrate | 77-93-0 | 1.19% |
| | Benzyl Alcohol | 100-51-6 | 16.61% |
| | Isopar M | 64742-47-8 | 20.95% |
| | Water | 7732-18-5 | 44.53% |
| | Stock 10% SLS Solution | | 3.18% |
| | | | |
| **Blend 39** | D-Limonene | 5989-27-5 | 13.090% |
| | Thyme Oil White | 8007-46-3 | 14.393% |
| | Benzyl Alcohol | 100-51-6 | 54.006% |
| | Blend 103 | | 18.511% |
| | | | |
| **Blend 40** | D-Limonene | 5989-27-5 | 27.35% |
| | Thyme Oil White | 8007-46-3 | 30.08% |
| | Linalool Coeur | 78-70-6 | 5.73% |
| | Tetrahydrolinalool | 78-69-3 | 7.88% |
| | Vanillin | 121-33-5 | 0.50% |
| | Isopropyl myristate | 110-27-0 | 8.08% |
| | Piperonal (aldehyde)[Heliotropine] | 120-57-0 | 8.09% |
| | Geraniol Fine FCC | 106-24-1 | 4.18% |
| | Triethyl Citrate | 77-93-0 | 8.11% |
| | | | |
| **Blend 41** | LFO | | 4.4% |
| | D-Limonene | 5989-27-5 | 82.3% |
| | Thyme Oil White | 8007-46-3 | 3.3% |
| | Blend 106 | | 10.0% |
| | | | |
| **Blend 42** | LFO | | 12.94% |
| | D-Limonene | 5989-27-5 | 8.72% |
| | Thyme Oil White | 8007-46-3 | 9.58% |
| | Blend 106 | | 68.76% |
| | | | |
| **Blend 43** | D-Limonene | 5989-27-5 | 9.8% |
| | Thyme Oil White | 8007-46-3 | 8.6% |
| | Linalool Coeur | 78-70-6 | 1.7% |
| | Tetrahydrolinalool | 78-69-3 | 2.3% |
| | Vanillin | 121-33-5 | 0.1% |
| | Isopropyl myristate | 110-27-0 | 2.4% |
| | Piperonal (aldehyde)[Heliotropine] | 120-57-0 | 2.4% |
| | Blend 106 | | 69.3% |
| | Geraniol Fine FCC | 106-24-1 | 1.2% |
| | Triethyl Citrate | 77-93-0 | 2.4% |
| | | | |
| **Blend 44** | Thyme Oil White | 8007-46-3 | 20.59% |
| | Wintergreen Oil | 68917-75-9 | 45.11% |
| | Isopropyl myristate | 110-27-0 | 34.29% |
| | | | |
| **Blend 45** | BSO | 977017-84-7 | 21.5% |
| | Linalool Coeur | 78-70-6 | 15.8% |
| | Tetrahydrolinalool | 78-69-3 | 19.0% |
| | Vanillin | 121-33-5 | 1.9% |
| | Isopropyl myristate | 110-27-0 | 23.4% |
| | Piperonal (aldehyde)[Heliotropine] | 120-57-0 | 7.8% |
| | Geraniol Fine FCC | 106-24-1 | 10.5% |
| | | | |
| **Blend 46** | Linalool Coeur | 78-70-6 | 6.63% |
| | Soy Bean Oil | 8016-70-4 | 24.03% |
| | Thymol (crystal) | 89-83-8 | 37.17% |
| | A-Pinene, 98% | 80-56-8 | 3.78% |
| | Para-Cymene | 99-87-6 | 28.39% |
| | | | |
| **Blend 47** | Linalool Coeur | 78-70-6 | 8.73% |
| | Thymol (crystal) | 89-83-8 | 48.93% |
| | A-Pinene, 98% | 80-56-8 | 4.97% |
| | Para-Cymene | 99-87-6 | 37.37% |
| | | | |
| **Blend 48** | D-Limonene | 5989-27-5 | 8.72% |
| | Thyme Oil White | 8007-46-3 | 9.58% |
| | Blend 105 | | 68.76% |
| | Linalool Coeur | 78-70-6 | 2.61% |
| | Tetrahydrolinalool | 78-69-3 | 3.13% |
| | Vanillin | 121-33-5 | 0.32% |
| | Isopropyl myristate | 110-27-0 | 3.86% |
| | Piperonal (aldehyde)[Heliotropine] | 120-57-0 | 1.29% |
| | Geraniol Fine FCC | 106-24-1 | 1.73% |
| | | | |
| **Blend 49** | D-Limonene | 5989-27-5 | 28.24% |
| | Thyme Oil White | 8007-46-3 | 4.44% |
| | Methyl Salicylate | | 67.32% |
| | | | |
| **Blend 50** | Thyme Oil White | 8007-46-3 | 20.6% |
| | Isopropyl myristate | 110-27-0 | 34.3% |
| | Wintergreen Oil | | 45.1% |
| | | | |
| **Blend 51** | Thyme Oil White | 8007-46-3 | 0.51% |
| | Wintergreen Oil | 68917-75-9 | 1.13% |
| | Isopropyl myristate | 110-27-0 | 0.86% |
| | Span 80 | 1338-43-8 | 0.50% |
| | Isopar M | 64742-47-8 | 15% |
| | Water | 7732-18-5 | 81.95% |
| | Bifenthrin | 83657-04-3 | 0.05% |
| | | | |
| **Blend 52** | Thyme Oil White | 8007-46-3 | 2.06% |
| | Wintergreen Oil | 68917-75-9 | 4.51% |
| | Isopropyl myristate | 110-27-0 | 3.43% |
| | Span 80 | 1338-43-8 | 0.50% |
| | Isopar M | 64742-47-8 | 15% |
| | Water | 7732-18-5 | 74.45% |
| | Bifenthrin | 83657-04-3 | 0.05% |
| **Blend 53** | Castor Oil hydrogenated - PEO40 | | 54.63% |
| | Lemon Grass Oil - India | | 22.93% |
| | Blend 10 | | 22.44% |
| | | | |
| **Blend 54** | LFO | | 16.18% |
| | D-Limonene | 5989-27-5 | 67.81% |
| | Thyme Oil White | 8007-46-3 | 11.18% |
| | BSO | 977017-84-7 | 4.83% |
| | | | |
| **Blend 55** | LFO | | 16.01% |
| | D-Limonene | 5989-27-5 | 67.09% |
| | Thyme Oil White | 8007-46-3 | 11.59% |
| | BSO | 977017-84-7 | 5.31% |
| | | | |
| **Blend 56** | D-Limonene | 5989-27-5 | 8.83% |
| | Thyme Oil White | 8007-46-3 | 9.71% |
| | Blend 105 | | 55.17% |
| | Linalool Coeur | 78-70-6 | 1.68% |
| | Tetrahydrolinalool | 78-69-3 | 2.31% |
| | Vanillin | 121-33-5 | 0.15% |
| | Isopropyl myristate | 110-27-0 | 2.37% |
| | Piperonal (aldehyde)[Heliotropine] | 120-57-0 | 2.37% |
| | Geraniol Fine FCC | 106-24-1 | 1.23% |
| | Triethyl Citrate | 77-93-0 | 2.38% |
| | Isopar M | 64742-47-8 | 13.80% |
| | | | |
| **Blend 57** | D-Limonene | 5989-27-5 | 8.72% |
| | Thyme Oil White | 8007-46-3 | 9.59% |
| | Blend 105 | | 69.35% |
| | Linalool Coeur | 78-70-6 | 1.66% |
| | Tetrahydrolinalool | 78-69-3 | 2.28% |
| | Vanillin | 121-33-5 | 0.15% |
| | Isopropyl myristate | 110-27-0 | 2.34% |
| | Piperonal (aldehyde)[Heliotropine] | 120-57-0 | 2.34% |
| | Geraniol Fine FCC | 106-24-1 | 1.21% |
| | Triethyl Citrate | 77-93-0 | 2.35% |
| | | | |
| **Blend 58** | LFO | | 16.31% |
| | D-Limonene | 5989-27-5 | 68.34% |
| | Thyme Oil White | 8007-46-3 | 5.37% |
| | Blend 105 | | 9.98% |
| **Blend 59** | Isopropyl myristate | 110-27-0 | 34.29% |
| | Wintergreen Oil | | 45.11% |
| | Blend 108 | | 20.59% |
| | | | |
| **Blend 60** | Isopropyl myristate | 110-27-0 | 34.29% |
| | Wintergreen Oil | | 45.11% |
| | Blend 108 | | 20.59% |
| | | | |
| **Blend 61** | Wintergreen Oil | 68917-75-9 | 45.10% |
| | Isopropyl myristate | 110-27-0 | 34.3% |
| | Thyme Oil Red | 8007-46-3 | 20.6% |
| | | | |
| **Blend 62** | Isopropyl myristate | 110-27-0 | 34.3% |
| | Thyme Oil Red | 8007-46-3 | 20.6% |
| | Wintergreen Oil | | 45.1% |
| | | | |
| **Blend 63** | Isopropyl myristate | 110-27-0 | 34.3% |
| | Thyme Oil Red | 8007-46-3 | 20.6% |
| | Wintergreen Oil | | 45.1% |
| | | | |
| **Blend 64** | Isopropyl myristate | 110-27-0 | 34.3% |
| | Wintergreen Oil | | 45.10% |
| | Blend 108 | | 20.6% |
| | | | |
| **Blend 65** | Thyme Oil White | 8007-46-3 | 20.59% |
| | Wintergreen Oil | 68917-75-9 | 45.10% |
| | Vanillin | 121-33-5 | 0.11% |
| | Isopropyl myristate | 110-27-0 | 34.20% |
| | | | |
| **Blend 66** | Wintergreen Oil | 68917-75-9 | 45.17% |
| | Vanillin | 121-33-5 | 0.11% |
| | Isopropyl myristate | 110-27-0 | 34.26% |
| | Thyme Oil Red | 8007-46-3 | 20.46% |
| | | | |
| **Blend 67** | Thyme Oil White | 8007-46-3 | 41.86% |
| | Isopropyl myristate | 110-27-0 | 38.34% |
| | Geraniol Fine FCC | 106-24-1 | 19.80% |
| | | | |
| **Blend 68** | Thyme Oil White | 8007-46-3 | 21.30% |
| | Isopropyl myristate | 110-27-0 | 58.54% |
| | Geraniol Fine FCC | 106-24-1 | 20.16% |
| **Blend 69** | Thyme Oil White | 8007-46-3 | 31.57% |
| | Isopropyl myristate | 110-27-0 | 38.56% |
| | Geraniol Fine FCC | 106-24-1 | 29.87% |
| | | | |
| **Blend 70** | Thyme Oil White | 8007-46-3 | 36.85% |
| | Isopropyl myristate | 110-27-0 | 48.21% |
| | Geraniol Fine FCC | 106-24-1 | 14.94% |
| | | | |
| **Blend 71** | Isopropyl myristate | 110-27-0 | 48.35% |
| | Geraniol Fine FCC | 106-24-1 | 14.98% |
| | Blend 108 | | 36.67% |
| | | | |
| **Blend 72** | Isopropyl myristate | 110-27-0 | 38.650% |
| | Geraniol Fine FCC | 106-24-1 | 29.940% |
| | Blend 108 | | 31.410% |
| | | | |
| **Blend 73** | Orange Terpenes | 68647-72-3 | 8.68% |
| | Blend 108 | | 9.47% |
| | Blend 109 | | 68.96% |
| | Blend 111 | | 12.89% |
| | | | |
| **Blend 74** | Isopropyl myristate | 110-27-0 | 38.46% |
| | Geraniol Fine FCC | 106-24-1 | 19.87% |
| | Blend 108 | | 41.67% |
| | | | |
| **Blend 75** | Isopropyl myristate | 110-27-0 | 38.46% |
| | Geraniol Fine FCC | 106-24-1 | 19.87% |
| | Blend 108 | | 41.67% |
| | | | |
| **Blend 76** | Linalool Coeur | 78-70-6 | 23.378% |
| | Amyl Butyrate | 540-18-1 | 23.459% |
| | Anise Star Oil | | 53.163% |
| | | | |
| **Blend 77** | Thyme Oil White | 8007-46-3 | 24.747% |
| | Amyl Butyrate | 540-18-1 | 23.040% |
| | Anise Star Oil | | 52.213% |
| | | | |
| **Blend 78** | Tetrahydrolinalool | 78-69-3 | 22.98% |
| | Vanillin | 121-33-5 | 1.17% |
| | Hercolyn D | 8050-15-5 | 4.44% |
| | Isopropyl myristate | 110-27-0 | 15.10% |
| | Piperonal (aldehyde)[Heliotropine] | 120-57-0 | 7.55% |
| | Ethyl Linalool | 10339-55-6 | 22.91% |
| | Hedione | 24851-98-7 | 6.67% |
| | Triethyl Citrate | 77-93-0 | 10.10% |
| | Dipropylene glycol (DPG) | 246-770-3 | 9.09% |
| | | | |
| **Blend 81** | Phenyl Ethyl Propionate | | 17.576% |
| | Benzyl Alcohol | 100-51-6 | 51.575% |
| | Methyl Salicylate | | 17.507% |
| | Blend 78 | | 13.342% |
| | | | |
| **Blend 84** | LFO | | 23.71% |
| | BSO | 977017-84-7 | 23.59% |
| | Benzyl Alcohol | 100-51-6 | 52.70% |
| | | | |
| **Blend 94** | Linalool Coeur | 78-70-6 | 4.67% |
| | Thymol (crystal) | 89-83-8 | 40.80% |
| | A-Pinene, 98% | 80-56-8 | 1.86% |
| | Para-Cymene | 99-87-6 | 34.49% |
| | Trans- Anethole | 4180-23-8 | 18.18% |
| | | | |
| **Blend 95** | Linalool Coeur | 78-70-6 | 6.63% |
| | Soy Bean Oil | 8016-70-4 | 24.03% |
| | Thymol (crystal) | 89-83-8 | 37.17% |
| | A-Pinene, 98% | 80-56-8 | 3.78% |
| | Para-Cymene | 99-87-6 | 28.39% |
| | | | |
| **Blend 96** | Linalool Coeur | 78-70-6 | 37.442% |
| | Thymol (crystal) | 89-83-8 | 36.719% |
| | A-Pinene, 98% | 80-56-8 | 4.664% |
| | Para-Cymene | 99-87-6 | 1.870% |
| | Trans- Anethole | 4180-23-8 | 19.305% |
| | | | |
| **Blend 97** | Linalool Coeur | 78-70-6 | 9.49% |
| | Thymol (crystal) | 89-83-8 | 47.87% |
| | A-Pinene, 98% | 80-56-8 | 9.46% |
| | Para-Cymene | 99-87-6 | 33.18% |
| | | | |
| **Blend 98** | Soy Bean Oil | 8016-70-4 | 24.46% |
| | A-Pinene, 98% | 80-56-8 | 3.84% |
| | Para-Cymene | 99-87-6 | 28.90% |
| | Linalyl Acetate | 115-95-7 | 7.12% |
| | Thymol acetate | 528-79-0 | 35.68% |
| | | | |
| **Blend 99** | A-Pinene, 98% | 80-56-8 | 8.80% |
| | Para-Cymene | 99-87-6 | 16.62% |
| | Linalyl Acetate | 115-95-7 | 22.61% |
| | Thymol acetate | 528-79-0 | 51.97% |
| | | | |
| **Blend 100** | A-Pinene, 98% | 80-56-8 | 10.13% |
| | Para-Cymene | 99-87-6 | 18.13% |
| | Linalyl Acetate | 115-95-7 | 23.92% |
| | Thymol acetate | 528-79-0 | 51.68% |
| | | | |
| **Blend 101** | Linalool Coeur | 78-70-6 | 20.15% |
| | Tetrahydrolinalool | 78-69-3 | 24.23% |
| | Vanillin | 121-33-5 | 2.47% |
| | Isopropyl myristate | 110-27-0 | 29.84% |
| | Piperonal (aldehyde)[Heliotropine] | 120-57-0 | 9.95% |
| | Geraniol Fine FCC | 106-24-1 | 13.36% |
| | | | |
| **Blend 102** | Tetrahydrolinalool | 78-69-3 | 22.98% |
| | Vanillin | 121-33-5 | 1.17% |
| | Hercolyn D | 8050-15-5 | 4.44% |
| | Isopropyl myristate | 110-27-0 | 15.10% |
| | Piperonal (aldehyde)[Heliotropine] | 120-57-0 | 7.55% |
| | Ethyl Linalool | 10339-55-6 | 22.91% |
| | Hedione | 24851-98-7 | 6.67% |
| | Triethyl Citrate | 77-93-0 | 10.10% |
| | Dipropylene glycol (DPG) | 246-770-3 | 9.09% |
| | | | |
| **Blend 103** | Linalool Coeur | 78-70-6 | 13.47% |
| | Tetrahydrolinalool | 78-69-3 | 18.50% |
| | Vanillin | 121-33-5 | 1.18% |
| | Isopropyl myristate | 110-27-0 | 18.99% |
| | Piperonal (aldehyde)[Heliotropine] | 120-57-0 | 18.99% |
| | Geraniol Fine FCC | 106-24-1 | 9.82% |
| | Triethyl Citrate | 77-93-0 | 19.05% |
| | | | |
| **Blend 104** | Linalool Coeur | 78-70-6 | 19.25% |
| | Tetrahydrolinalool | 78-69-3 | 23.19% |
| | Vanillin | 121-33-5 | 1.24% |
| | Isopropyl myristate | 110-27-0 | 22.90% |
| | Piperonal (aldehyde)[Heliotropine] | 120-57-0 | 23.80% |
| | Piperonyl Alcohol | 495-76-1 | 9.61% |
| | | | |
| **Blend 105** | D-Limonene | 5989-27-5 | 48.58% |
| | Linalool Coeur | 78-70-6 | 1.22% |
| | Citral | 5392-40-5 | 10.21% |
| | gamma-terpinene | 99-85-4 | 10.51% |
| | A-Pinene, 98% | 80-56-8 | 1.94% |
| | α-Terpineol | 98-55-5 | 6.80% |
| | Terpinolene | 586-62-9 | 6.30% |
| | Para-Cymene | 99-87-6 | 1.61% |
| | Linalyl Acetate | 115-95-7 | 2.60% |
| | B Pinene | 127-91-3 | 2.80% |
| | Camphor Dextro | 464-49-3 | 0.13% |
| | Terpinene 4 OL | 562-74-3 | 0.11% |
| | A Terpinene | 99-86-5 | 2.80% |
| | Borneol L | 507-70-0 | 1.30% |
| | Camphene | 79-92-5 | 0.54% |
| | Decanal | 112-31-2 | 0.17% |
| | Dodecanal | 112-54-9 | 0.14% |
| | Fenchol A | 512-13-0 | 0.01% |
| | Geranyl Acetate | 105-87-3 | 0.18% |
| | Isoborneol | 124-76-5 | 0.41% |
| | 2-Methyl 1,3-cyclohexadiene | 30640-46-1, 1888-90-0 | 0.38% |
| | Myrcene | 123-35-3 | 1.14% |
| | Nonanal | 124-19-6 | 0.03% |
| | Octanal | 124-13-0 | 0.06% |
| | Tocopherol Gamma (TENOX®) | 54-28-4 | 0.03% |
| | | | |
| **Blend 106** | D-Limonene | 5989-27-5 | 58.54% |
| | Linalool Coeur | 78-70-6 | 1.47% |
| | gamma-terpinene | 99-85-4 | 12.66% |
| | A-Pinene, 98% | 80-56-8 | 2.34% |
| | Terpinolene | 586-62-9 | 7.59% |
| | Para-Cymene | 99-87-6 | 1.94% |
| | Linalyl Acetate | 115-95-7 | 3.13% |
| | B Pinene | 127-91-3 | 3.37% |
| | Camphor Dextro | 464-49-3 | 3.37% |
| | Terpinene 4 OL | 562-74-3 | 0.13% |
| | A Terpinene | 99-86-5 | 0.16% |
| | Borneol L | 507-70-0 | 1.57% |
| | Camphene | 79-92-5 | 0.65% |
| | Decanal | 112-31-2 | 0.20% |
| | Dodecanal | 112-54-9 | 0.17% |
| | Fenchol A | 512-13-0 | 0.01% |
| | Geranyl Acetate | 105-87-3 | 0.22% |
| | Isoborneol | 124-76-5 | 0.49% |
| | 2-Methyl 1,3-cyclohexadiene | 30640-46-1, 1888-90-0 | 0.46% |
| | Myrcene | 123-35-3 | 1.37% |
| | Nonanal | 124-19-6 | 0.04% |
| | Octanal | 124-13-0 | 0.07% |
| | Tocopherol Gamma (TENOX®) | 54-28-4 | 0.04% |
| | | | |
| **Blend 107** | D-Limonene | 5989-27-5 | 34.50% |
| | Linalool Coeur | 78-70-6 | 10.05% |
| | A-Pinene, 98% | 80-56-8 | 5.01% |
| | Terpinolene | 586-62-9 | 10.10% |
| | Para-Cymene | 99-87-6 | 10.04% |
| | Linalyl Acetate | 115-95-7 | 5.30% |
| | B Pinene | 127-91-3 | 5.02% |
| | A Terpinene | 99-86-5 | 4.88% |
| | Camphene | 79-92-5 | 5.84% |
| | Myrcene | 123-35-3 | 9.26% |
| | | | |
| **Blend 108** | D-Limonene | 5989-27-5 | 0.25% |
| | Thyme Oil Red | 8007-46-3 | 1.00% |
| | Thymol (crystal) | 89-83-8 | 51.00% |
| | α-Terpineol | 98-55-5 | 1.94% |
| | Para-Cymene | 99-87-6 | 19.92% |
| | Linalyl Acetate | 115-95-7 | 1.46% |
| | Caryophyllene-B | 87-44-5 | 3.94% |
| | Borneol L | 507-70-0 | 1.94% |
| | Myrcene | 123-35-3 | 0.97% |
| | Tea Tree Oil | | 1.94% |
| | Cypress Oil | | 2.86% |
| | Peppermint Terpenes | 8006-90-4 | 9.72% |
| | Linalool 90 | | 3.06% |
| | | | |
| **Blend 109** | D-Limonene | 5989-27-5 | 55.95% |
| | Citral | 5392-40-5 | 9.15% |
| | gamma-terpinene | 99-85-4 | 10.50% |
| | A-Pinene, 98% | 80-56-8 | 1.45% |
| | α-Terpineol | 98-55-5 | 5.70% |
| | Terpinolene | 586-62-9 | 7.10% |
| | Lime Distilled Oil | | 0.10% |
| | Lime Expressed Oil | | 0.10% |
| | Linalyl Acetate | 115-95-7 | 2.15% |
| | Caryophyllene-B | 87-44-5 | 0.10% |
| | B Pinene | 127-91-3 | 2.50% |
| | Terpinene 4 OL | 562-74-3 | 0.05% |
| | A Terpinene | 99-86-5 | 2.00% |
| | Borneol L | 507-70-0 | 1.40% |
| | Camphene | 79-92-5 | 0.50% |
| | Geranyl Acetate | 105-87-3 | 0.15% |
| | Isoborneol | 124-76-5 | 0.10% |
| | Linalool 90 | | 0.80% |
| | Camphor Gum | | 0.05% |
| | Aldehyde C-10 | | 0.05% |
| | Aldehyde C-12 | | 0.10% |
| | | | |
| **Blend 110** | Eugenol | 97-53-0 | 0.03% |
| | Eucalyptol (1,8 Cineole) | | 0.07% |
| | Methyl Salicylate | | 99.75% |
| | Linalool 90 | | 0.07% |
| | Ethyl Salicylate | | 0.08% |
| | | | |
| **Blend 111** | Tetrahydrolinalool | 78-69-3 | 11.50% |
| | Hercolyn D | 8050-15-5 | 7.50% |
| | Isopropyl myristate | 110-27-0 | 5.80% |
| | Piperonal (aldehyde)[Heliotropine] | 120-57-0 | 10.00% |
| | Ethyl Linalool | 10339-55-6 | 10.50% |
| | Triethyl Citrate | 77-93-0 | 9.50% |
| | Dipropylene glycol (DPG) | 246-770-3 | 10.10% |
| | Cinnamic Alcohol | 104-54-1 | 1.70% |
| | Eugenol | 97-53-0 | 1.60% |
| | Phenyl Ethyl Alcohol | 60-12-8 | 21.50% |
| | Iso Eugenol | | 0.30% |
| | Methyl Dihydrojasmonate | | 10.00% |
| | | | |
| **Blend 112** | Linalool Coeur | 78-70-6 | 14.12% |
| | Tetrahydrolinalool | 78-69-3 | 24.24% |
| | Vanillin | 121-33-5 | 2.47% |
| | Isopropyl myristate | 110-27-0 | 28.87% |
| | Piperonal (aldehyde)[Heliotropine] | 120-57-0 | 9.95% |
| | Piperonyl Alcohol | 495-76-1 | 10.07% |
| | Geraniol Fine FCC | 106-24-1 | 10.28% |
| | | | |
| **Blend 113** | Blend 44 | | 90% |
| | Stock 10% SLS Solution | | 10% |
| | | | |
| | | | |
| **Blend 114** | Polyglycerol-4-oleate | 9007-48-1 | 0.90% |
| | Lecithin | 8002-43-5 | 0.20% |
| | Water | 7732-18-5 | 9.8% |
| | Blend 44 | | 89.1% |
| | | | |
| **Blend 115** | Potassium Sorbate | 590-00-1 or 24634-61-5 | 1.00% |
| | Xanthan Gum | 11138-66-2 | 0.28% |
| | Water | 7732-18-5 | 81.82% |
| | Blend 114 | | 16.90% |
| | | | |
| **Blend 116** | Potassium Sorbate | 590-00-1 or 24634-61-5 | 0.11% |
| | Polyglycerol-4-oleate | 9007-48-1 | 0.15% |
| | Xanthan Gum | 11138-66-2 | 0.28% |
| | Lecithin | 8002-43-5 | 0.034% |
| | Water | 7732-18-5 | 84.4% |
| | Blend 44 | | 15% |
| | | | |
| **Blend 117** | Thyme Oil White | 8007-46-3 | 3.09% |
| | Wintergreen Oil | 68917-75-9 | 6.77% |
| | Isopropyl myristate | 110-27-0 | 5.15% |
| | Potassium Sorbate | 590-00-1 or 24634-61-5 | 0.11% |
| | Polyglycerol-4-oleate | 9007-48-1 | 0.15% |
| | Xanthan Gum | 11138-66-2 | 0.28% |
| | Lecithin | 8002-43-5 | 0.03% |
| | Water | 7732-18-5 | 84.41% |
| | | | |
| **Blend 118** | Polyglycerol-4-oleate | 9007-48-1 | 0.90% |
| | Lecithin | 8002-43-5 | 0.20% |
| | Water | 7732-18-5 | 9.8% |
| | Blend 26 | | 89.10% |
| | | | |
| **Blend 119** | Water | 7732-18-5 | 3.1% |
| | Blend 114 | | 84.2% |
| | Stock 2.5% Xanthan-1% K sorbate | | 12.7% |
| | | | |
| **Blend 120** | Thyme Oil White | 8007-46-3 | 15.5% |
| | Wintergreen Oil | 68917-75-9 | 33.8% |
| | Isopropyl myristate | 110-27-0 | 25.7% |
| | Potassium Sorbate | 590-00-1 or 24634-61-5 | 0.13% |
| | Polyglycerol-4-oleate | 9007-48-1 | 0.76% |
| | Xanthan Gum | 11138-66-2 | 0.32% |
| | Lecithin | 8002-43-5 | 0.17% |
| | Water | 7732-18-5 | 23.6% |
| | | | |
| **Blend 121** | Water | 7732-18-5 | 9.2% |
| | Blend 114 | | 78.87% |
| | Stock 2.5% Xanthan-1% K sorbate | | 11.90% |
| | | | |
| **Blend 122** | Potassium Sorbate | 590-00-1 or 24634-61-5 | 0.13% |
| | Polyglycerol-4-oleate | 9007-48-1 | 0.76% |
| | Xanthan Gum | 11138-66-2 | 0.32% |
| | Lecithin | 8002-43-5 | 0.17% |
| | Water | 7732-18-5 | 28.6% |
| | Blend 44 | | 70% |
| | | | |
| **Blend 123** | Water | 7732-18-5 | 3.1% |
| | Blend 118 | | 84.2% |
| | Stock 2.5% Xanthan-1% K sorbate | | 12.7% |
| | | | |
| **Blend 124** | Potassium Sorbate | 590-00-1 or 24634-61-5 | 1% |
| | Xanthan Gum | 11138-66-2 | 0.28% |
| | Water | 7732-18-5 | 81.8% |
| | Blend 118 | | 16.90% |
| | | | |
| **Blend 125** | Blend 10 | | 2.50% |
| | Water | | 97.50% |
| | | | |
| | | | |
| **Blend 126** | Polyglycerol-4-oleate | 9007-48-1 | 0.90% |
| | Lecithin | 8002-43-5 | 0.20% |
| | Water | 7732-18-5 | 9.8% |
| | Blend 50 | | 89.10% |
| | | | |
| **Blend 127** | Potassium Sorbate | 590-00-1 or 24634-61-5 | 1.00% |
| | Xanthan Gum | 11138-66-2 | 0.28% |
| | Water | 7732-18-5 | 81.82% |
| | Blend 126 | | 16.90% |
| | | | |
| **Blend 128** | Citronella Oil | 106-22-9 | 0.20% |
| | Carbopol 940 | [9003-01-4] | 0.20% |
| | BHT (butylated hydroxytoluene) | 128-37-0 | 0.10% |
| | Water | 7732-18-5 | 59.83% |
| | Emulsifying Wax | 67762-27-0, 9005-67-8 | 14.00% |
| | Light Liquid Paraffin | 8012-95-1 | 4.00% |
| | White Soft Paraffin | [8009-03-8] | 9.00% |
| | Sodium Metabisulphate | [7681-57-4] | 0.25% |
| | Propylene Glycol | [57-55-6] | 2.00% |
| | Methyl Paraben | [99-76-3] | 0.15% |
| | Propyl Paraben | [94-13-3] | 0.05% |
| | Cresmer RH40 hydrogenated castor oil | [61791-12-6] | 5.00% |
| | Triethanolamine | [102-71-6] | 0.15% |
| | Vitamin E Acetate | [58-95-7] | 0.02% |
| | Disodium EDTA | [139-33-3] | 0.05% |
| | Blend 10 | | 5.00% |
| | | | |
| **Blend 129** | Span 80 | 1338-43-8 | 0.05% |
| | Sodium Benzoate | 532-32-1 | 0.20% |
| | Isopar M | 64742-47-8 | 29% |
| | A46 Propellant | | 14.50% |
| | Water | 7732-18-5 | 42.25% |
| | Isopropyl alcohol | 67-63-0 | 1.50% |
| | Blend 8 | | 12.50% |
| | | | |
| **Blend 130** | Isopar M | 64742-47-8 | 51.0% |
| | A46 Propellant | | 40.0% |
| | Isopropyl alcohol | 67-63-0 | 3.0% |
| | Blend 39 | | 6.0% |
| | | | |
| **Blend 131** | Isopar M | 64742-47-8 | 51.0% |
| | A46 Propellant | | 40.0% |
| | Bifenthrin | 83657-04-3 | 0.05% |
| | Isopropyl alcohol | 67-63-0 | 3.0% |
| | Blend 39 | | 6.0% |
| | | | |
| **Blend 132** | Isopar M | 64742-47-8 | 54.0% |
| | A46 Propellant | | 40.0% |
| | Blend 33 | | 6.0% |
| | | | |
| **Blend 133** | Thyme Oil White | 8007-46-3 | 2.06% |
| | Wintergreen Oil | 68917-75-9 | 4.51% |
| | Isopropyl myristate | 110-27-0 | 3.43% |
| | Potassium Sorbate | 590-00-1 or 24634-61-5 | 0.11% |
| | Polyglycerol-4-oleate | 9007-48-1 | 0.15% |
| | Xanthan Gum | 11138-66-2 | 0.28% |
| | Lecithin | 8002-43-5 | 0.03% |
| | Water | 7732-18-5 | 89.42% |
| | | | |
| **Blend 134** | Thyme Oil White | 8007-46-3 | 1.03% |
| | Wintergreen Oil | 68917-75-9 | 2.26% |
| | Isopropyl myristate | 110-27-0 | 1.72% |
| | Potassium Sorbate | 590-00-1 or 24634-61-5 | 0.11% |
| | Polyglycerol-4-oleate | 9007-48-1 | 0.15% |
| | Xanthan Gum | 11138-66-2 | 0.28% |
| | Lecithin | 8002-43-5 | 0.03% |
| | Water | 7732-18-5 | 94.43% |
| | | | |
| **Blend 135** | Potassium Sorbate | 590-00-1 or 24634-61-5 | 0.11% |
| | Polyglycerol-4-oleate | 9007-48-1 | 0.15% |
| | Xanthan Gum | 11138-66-2 | 0.28% |
| | Lecithin | 8002-43-5 | 0.034% |
| | Water | 7732-18-5 | 84.4% |
| | Blend 44 | | 15.01% |
| | | | |
| **Blend 136** | Thyme Oil White | 8007-46-3 | 3.09% |
| | Wintergreen Oil | 68917-75-9 | 6.77% |
| | Isopropyl myristate | 110-27-0 | 5.15% |
| | Potassium Sorbate | 590-00-1 or 24634-61-5 | 0.11% |
| | Polyglycerol-4-oleate | 9007-48-1 | 0.15% |
| | Xanthan Gum | 11138-66-2 | 0.28% |
| | Lecithin | 8002-43-5 | 0.03% |
| | Water | 7732-18-5 | 84.41% |
| **Blend 137** | Potassium Sorbate | 590-00-1 or 24634-61-5 | 0.110% |
| | Polyglycerol-4-oleate | 9007-48-1 | 0.152% |
| | Xanthan Gum | 11138-66-2 | 0.225% |
| | Lecithin | 8002-43-5 | 0.030% |
| | Water | 7732-18-5 | 81.985% |
| | Isopropyl alcohol | 67-63-0 | 2.500% |
| | Blend 59 | | 15.000% |
| | | | |
| **Blend 138** | Potassium Sorbate | 590-00-1 or 24634-61-5 | 0.11% |
| | Polyglycerol-4-oleate | 9007-48-1 | 0.15% |
| | Xanthan Gum | 11138-66-2 | 0.225% |
| | Lecithin | 8002-43-5 | 0.030% |
| | Water | 7732-18-5 | 81.985% |
| | Isopropyl alcohol | 67-63-0 | 2.50% |
| | Blend 59 | | 15.00% |
| | | | |
| **Blend 139** | Potassium Sorbate | 590-00-1 or 24634-61-5 | 0.116% |
| | Polyglycerol-4-oleate | 9007-48-1 | 0.161% |
| | Xanthan Gum | 11138-66-2 | 0.238% |
| | Lecithin | 8002-43-5 | 0.032% |
| | Water | 7732-18-5 | 86.81% |
| | Blend 59 | | 12.643% |
| | | | |
| **Blend 140** | Potassium Sorbate | 590-00-1 or 24634-61-5 | 0.11% |
| | Polyglycerol-4-oleate | 9007-48-1 | 0.15% |
| | Xanthan Gum | 11138-66-2 | 0.28% |
| | Lecithin | 8002-43-5 | 0.034% |
| | Water | 7732-18-5 | 84.4% |
| | Blend 59 | | 15.01% |
| | | | |
| **Blend 141** | Isopropyl myristate | 110-27-0 | 3.40% |
| | Geraniol Fine FCC | 106-24-1 | 2.63% |
| | Potassium Sorbate | 590-00-1 or 24634-61-5 | 0.11% |
| | Polyglycerol-4-oleate | 9007-48-1 | 0.60% |
| | Xanthan Gum | 11138-66-2 | 0.30% |
| | Lecithin | 8002-43-5 | 0.060% |
| | Water | 7732-18-5 | 87.63% |
| | Blend 108 | | 2.76% |
| | Isopropyl alcohol | 67-63-0 | 2.50% |
| | | | |
| **Blend 142** | Wintergreen Oil | 68917-75-9 | 4.51% |
| | Isopropyl myristate | 110-27-0 | 3.43% |
| | Thyme Oil Red | 8007-46-3 | 2.06% |
| | Stock 0.3% SLS-0.1% Xanthan Solution | | 90% |
| | | | |
| **Blend 143** | Stock 0.3% SLS & 0.1% Xanthan Solution | | 95% |
| | Blend 67 | | 5% |
| | | | |
| | | | |
| **Blend 144** | Stock 0.3% SLS & 0.1% Xanthan Solution | | 95% |
| | Blend 69 | | 5% |
| | | | |
| | | | |
| **Blend 145** | Stock 0.3% SLS & 0.1% Xanthan Solution | | 95% |
| | Blend 70 | | 5% |
| | | | |
| | | | |
| **Blend 146** | Lecithin, Soya | 8030-76-0 | 0.20% |
| | Polyglycerol-4-oleate | 9007-48-1 | 0.90% |
| | Water | 7732-18-5 | 9.80% |
| | Blend 26 | | 89.10% |
| | | | |
| **Blend 147** | Thyme Oil White | 8007-46-3 | 35.0% |
| | Isopropyl myristate | 110-27-0 | 32.0% |
| | Lecithin, Soya | 8030-76-0 | 0.20% |
| | Polyglycerol-4-oleate | 9007-48-1 | 0.90% |
| | Water | 7732-18-5 | 9.80% |
| | Wintergreen Oil | | 22.1% |
| | | | |
| **Blend 148** | Lecithin, Soya | 8030-76-0 | 0.10% |
| | Polyglycerol-4-oleate | 9007-48-1 | 0.90% |
| | Water | 7732-18-5 | 9.90% |
| | Blend 7 | | 89.1% |
| | | | |
| **Blend 149** | Thyme Oil White | 8007-46-3 | 18.27% |
| | Wintergreen Oil | 68917-75-9 | 40.10% |
| | Vanillin | 121-33-5 | 0.98% |
| | Isopropyl myristate | 110-27-0 | 29.76% |
| | Lecithin, Soya | 8030-76-0 | 0.10% |
| | Polyglycerol-4-oleate | 9007-48-1 | 0.90% |
| | Water | 7732-18-5 | 9.90% |
| | | | |
| **Blend 150** | Polyglycerol-4-oleate | 9007-48-1 | 1.90% |
| | Water | 7732-18-5 | 9.00% |
| | Blend 26 | | 89.10% |
| | | | |
| **Blend 151** | Thyme Oil White | 8007-46-3 | 35.0% |
| | Isopropyl myristate | 110-27-0 | 32.0% |
| | Polyglycerol-4-oleate | 9007-48-1 | 1.90% |
| | Water | 7732-18-5 | 9.00% |
| | Wintergreen Oil | | 22.1% |
| | | | |
| **Blend 152** | Potassium Sorbate | 590-00-1 or 24634-61-5 | 0.11% |
| | Polyglycerol-4-oleate | 9007-48-1 | 1.90% |
| | Xanthan Gum | 11138-66-2 | 0.275% |
| | Water | 7732-18-5 | 86.410% |
| | Blend 148 | | 11.30% |
| | | | |
| **Blend 153** | D-Limonene | 5989-27-5 | 5.67% |
| | Thyme Oil White | 8007-46-3 | 1.25% |
| | Lecithin, Soya | 8030-76-0 | 0.011% |
| | Potassium Sorbate | 590-00-1 or 24634-61-5 | 0.11% |
| | Polyglycerol-4-oleate | 9007-48-1 | 2.002% |
| | Xanthan Gum | 11138-66-2 | 0.275% |
| | Water | 7732-18-5 | 87.529% |
| | Wintergreen Oil | | 3.15% |
| | | | |
| **Blend 154** | Potassium Sorbate | 590-00-1 or 24634-61-5 | 0.11% |
| | Xanthan Gum | 11138-66-2 | 0.275% |
| | Water | 7732-18-5 | 88.315% |
| | Blend 146 | | 11.30% |
| | | | |
| **Blend 155** | Thyme Oil White | 8007-46-3 | 3.95% |
| | Isopropyl myristate | 110-27-0 | 3.62% |
| | Lecithin, Soya | 8030-76-0 | 0.023% |
| | Potassium Sorbate | 590-00-1 or 24634-61-5 | 0.11% |
| | Polyglycerol-4-oleate | 9007-48-1 | 0.102% |
| | Xanthan Gum | 11138-66-2 | 0.275% |
| | Water | 7732-18-5 | 89.422% |
| | Wintergreen Oil | | 2.50% |
| | | | |
| **Blend 156** | Potassium Sorbate | 590-00-1 or 24634-61-5 | 0.11% |
| | Xanthan Gum | 11138-66-2 | 0.275% |
| | Water | 7732-18-5 | 88.315% |
| | Blend 150 | | 11.30% |
| | | | |
| **Blend 157** | Thyme Oil White | 8007-46-3 | 3.95% |
| | Wintergreen Oil | 68917-75-9 | 2.50% |
| | Isopropyl myristate | 110-27-0 | 3.62% |
| | Potassium Sorbate | 590-00-1 or 24634-61-5 | 0.11% |
| | Polyglycerol-4-oleate | 9007-48-1 | 0.21 % |
| | Xanthan Gum | 11138-66-2 | 0.275% |
| | Water | 7732-18-5 | 89.332% |
| | | | |
| **Blend 158** | Potassium Sorbate | 590-00-1 or 24634-61-5 | 1.00% |
| | Xanthan Gum | 11138-66-2 | 2.500% |
| | Water | 7732-18-5 | 96.500% |
| | | | |
| **Blend 159** | Sodium Benzoate | 532-32-1 | 2% |
| | Water | 7732-18-5 | 98% |
| | | | |
| **Blend 160** | Span 80 | 1338-43-8 | 1.20% |
| | Tween 80 | | 1.65% |
| | Isopar M | 64742-47-8 | 14.20% |
| | Water | 7732-18-5 | 68.75% |
| | Blend 8 | | 2.84% |
| | 2% Sodium Benzoate | | 11.36% |
| | | | |
| **Blend 161** | D-Limonene | 5989-27-5 | 1.60% |
| | Thyme Oil White | 8007-46-3 | 0.35% |
| | Wintergreen Oil | 68917-75-9 | 0.89% |
| | Span 80 | 1338-43-8 | 1.20% |
| | Tween 80 | | 1.65% |
| | Sodium Benzoate | 532-32-1 | 0.23% |
| | Isopar M | 64742-47-8 | 14.20% |
| | Water | 7732-18-5 | 79.88% |
| | | | |
| **Blend 162** | Propellent A70 | | 22% |
| | Blend 160 | | 78% |
| | | | |
| | | | |
| **Blend 163** | D-Limonene | 5989-27-5 | 1.25% |
| | Thyme Oil White | 8007-46-3 | 0.27% |
| | Wintergreen Oil | 68917-75-9 | 0.69% |
| | Span 80 | 1338-43-8 | 0.94% |
| | Tween 80 | | 1.29% |
| | Sodium Benzoate | 532-32-1 | 0.18% |
| | Isopar M | 64742-47-8 | 11.08% |
| | Water | 7732-18-5 | 62.30% |
| | Propellent A70 | | 22.0% |
| | | | |
| **Blend 164** | Potassium Sorbate | 590-00-1 or 24634-61-5 | 1% |
| | Xanthan Gum | 11138-66-2 | 2.50% |
| | Water | 7732-18-5 | 96.50% |
| | | | |
| **Blend 165** | Sodium Lauryl Sulfate | 151-21-3 | 10% |
| | Water | 7732-18-5 | 90.00% |
| | | | |
| | | | |
| **Blend 166** | Sodium Lauryl Sulfate | 151-21-3 | 0.30% |
| | Xanthan Gum | 11138-66-2 | 0.10% |
| | Water | 7732-18-5 | 99.60% |
| | | | |
| **Blend 167** | Potassium Sorbate | 590-00-1 or 24634-61-5 | 1.0% |
| | Polyglycerol-4-oleate | 9007-48-1 | 0.15% |
| | Xanthan Gum | 11138-66-2 | 0.28% |
| | Lecithin | 8002-43-5 | 0.034% |
| | Water | 7732-18-5 | 83.5% |
| | Blend 44 | | 15.1% |
| | | | |
| **Blend 168** | Citronella Oil | 106-22-9 | 0.20% |
| | Carbopol 940 | [9003-01-4] | 0.20% |
| | BHT (butylated hydroxytoluene) | 128-37-0 | 0.10% |
| | Water | 7732-18-5 | 59.83% |
| | Emulsifying Wax | 67762-27-0, 9005-67-8 | 14% |
| | Light Liquid Paraffin | 8012-95-1 | 4.00% |
| | White Soft Paraffin | [8009-03-8] | 9% |
| | Sodium Metabisulphate | [7681-57-4] | 0.25% |
| | Propylene Glycol | [57-55-6] | 2% |
| | Cresmer RH40 hydrogenated castor oil | [61791-12-6] | 5% |
| | Triethanolamine | [102-71-6] | 0.15% |
| | Vitamin E Acetate | [58-95-7] | 0.02% |
| | Disodium EDTA | [139-33-3] | 0.05% |
| | Blend 10 | | 5% |
| | | | |
| **Blend 169** | Water | 7732-18-5 | 33.40% |
| | Blend 115 | | 66.60% |
| | | | |
| | | | |
| **Blend 170** | D-Limonene | 5989-27-5 | 4.03% |
| | Thyme Oil White | 8007-46-3 | 4.43% |
| | Benzyl Alcohol | 100-51-6 | 16.61% |
| | Isopar M | 64742-47-8 | 20.95% |
| | Water | 7732-18-5 | 44.53% |
| | Blend 103 | | 6.27% |
| | Stock 10% SLS Solution | | 3.18% |
| | | | |
| **Blend 171** | D-Limonene | 5989-27-5 | 4.048% |
| | Thyme Oil White | 8007-46-3 | 4.451% |
| | Benzyl Alcohol | 100-51-6 | 16.70% |
| | Isopar M | 64742-47-8 | 21.07% |
| | Water | 7732-18-5 | 44.76% |
| | Bifenthrin | 83657-04-3 | 0.05% |
| | Blend 103 | | 5.723% |
| | Stock 10% SLS Solution | | 3.197% |
| | | | |
| **Blend 172** | Thyme Oil White | 8007-46-3 | 2.06% |
| | Wintergreen Oil | 68917-75-9 | 4.51% |
| | Isopropyl myristate | 110-27-0 | 3.43% |
| | Span 80 | 1338-43-8 | 0.50% |
| | Isopar M | 64742-47-8 | 15% |
| | Water | 7732-18-5 | 74.45% |
| | Bifenthrin | 83657-04-3 | 0.05% |
| | | | |
| **Blend 173** | Thyme Oil White | 8007-46-3 | 0.41% |
| | Wintergreen Oil | 68917-75-9 | 0.90% |
| | Isopropyl myristate | 110-27-0 | 0.69% |
| | Sodium Lauryl Sulfate | 151-21-3 | 0.02% |
| | Water | 7732-18-5 | 97.98% |
| | | | |
| **Blend 174** | Thyme Oil White | 8007-46-3 | 1.03% |
| | Wintergreen Oil | 68917-75-9 | 2.26% |
| | Isopropyl myristate | 110-27-0 | 1.71% |
| | AgSorb clay carrier | | 95.00% |
| | | | |
| **Blend 175** | Thyme Oil White | 8007-46-3 | 1.03% |
| | Wintergreen Oil | 68917-75-9 | 2.26% |
| | Isopropyl myristate | 110-27-0 | 1.71% |
| | DG Lite | | 95.0% |
| | | | |
| **Blend 176** | Thyme Oil White | 8007-46-3 | 0.41% |
| | Wintergreen Oil | 68917-75-9 | 0.90% |
| | Isopropyl myristate | 110-27-0 | 0.69% |
| | Sodium Lauryl Sulfate | 151-21-3 | 0.02% |
| | Water | 7732-18-5 | 97.98% |
| | | | |
| **Blend 177** | D-Limonene | 5989-27-5 | 24.76% |
| | Thyme Oil White | 8007-46-3 | 0.98% |
| | Linalool Coeur | 78-70-6 | 0.17% |
| | Tetrahydrolinalool | 78-69-3 | 0.23% |
| | Vanillin | 121-33-5 | 0.02% |
| | Isopropyl myristate | 110-27-0 | 0.24% |
| | Piperonal (aldehyde) [Heliotropine] | 120-57-0 | 0.24% |
| | Blend 106 | | 3.00% |
| | Geraniol 60 | 106-24-1 | 0.12% |
| | Triethyl Citrate | 77-93-0 | 0.24% |
| | Water | 7732-18-5 | 67% |
| | Stock 10% SLS Solution | | 3% |
| | | | |
| **Blend 178** | Potassium Sorbate | 590-00-1 or 24634-61-5 | 1% |
| | Xanthan Gum | 11138-66-2 | 0.28% |
| | Water | 7732-18-5 | 81.82% |
| | Blend 114 | | 16.90% |
| | | | |
| **Blend 179** | Miracle Gro (Sterile) | | 95% |
| | Blend 44 | | 5% |
| | | | |
| | | | |
| **Blend 180** | Thyme Oil White | 8007-46-3 | 0.51% |
| | Wintergreen Oil | 68917-75-9 | 1.13% |
| | Isopropyl myristate | 110-27-0 | 0.86% |
| | Span 80 | 1338-43-8 | 0.50% |
| | Isopar M | 64742-47-8 | 15% |
| | Water | 7732-18-5 | 81.95% |
| | Bifenthrin | 83657-04-3 | 0.05% |
| | | | |
| **Blend 182** | Thyme Oil White | 8007-46-3 | 25.0% |
| | Amyl Butyrate | 540-18-1 | 25.0% |
| | Anise Star Oil | | 49.99% |
| | Genistein | | 0.01% |
| | | | |
| **Blend 184** | D-Limonene | 5989-27-5 | 9.90% |
| | Linalool Coeur | 78-70-6 | 14.14% |
| | Tetrahydrolinalool | 78-69-3 | 24.29% |
| | Vanillin | 121-33-5 | 2.48% |
| | Isopropyl myristate | 110-27-0 | 28.92% |
| | Piperonal (aldehyde) | 120-57-0 | 9.97% |
| | Geraniol 60 | | 10.30% |
| | | | |
| **Blend 185** | D-Limonene | 5989-27-5 | 82.52% |
| | Thyme Oil White | 8007-46-3 | 3.28% |
| | Linalool Coeur | 78-70-6 | 0.57% |
| | Tetrahydrolinalool | 78-69-3 | 0.78% |
| | Vanillin | 121-33-5 | 0.05% |
| | Isopropyl myristate | 110-27-0 | 0.80% |
| | Piperonal (aldehyde) | 120-57-0 | 0.80% |
| | Blend 106 | | 9.99% |
| | Geraniol 60 | | 0.41% |
| | Triethyl Citrate | 77-93-0 | 0.80% |

Furthermore, in addition to the specific amounts of ingredients listed for each blend in Table E above, ranges of amounts are also contemplated that may be derived by multiplying each specific amount by the following four factors: Factor 1 (±200%); Factor 2 (±100%); Factor 3 (±40%); and Factor 4 (±10%). The resulting ranges will not, of course, containg any values less than 0% or greater than 100%.

In some embodiments of the present disclosure, compositions are specifically contemplated that comprise a synergistic combination of at least two compounds listed in any of Tables B, B1, C, D, or E above.

Surprisingly, by blending certain compounds in certain relative amounts, the resulting composition demonstrates an anti-parasitic effect that exceeds the anti-parasitic effect of any component of the composition. As used herein, "component of a composition" refers to a compound, or a subset of compounds included in a composition, e.g., the complete composition minus at least one compound. As used herein, an "anti-parasitic effect" refers to any measurable parameter related to the efficacy of a composition for treating a parasitic infection. The effect can be a parameter related to viability, killing, prophylaxis, or another useful and quantifiable parameter for a set time point, or it can be time to achieve a defined result, e.g., time to achieve 100% killing with a set dose. In this regard, when a first effect and a second effect are compared, the first effect can indicate a greater efficacy for treating a parasitic infection if it exceeds the second effect. For example, when the effect being measured is a time to achieve 100% killing, a shorter time is an anti-parasitic effect that exceeds a longer time. For another example, when the effect being measured is a % killing of target parasites, a greater % killing is an anti-parasitic effect that exceeds a lesser % killing. Effects that can be measured include, but are not limited to: time to kill a given percentage of a target parasite *in vivo* or *in vitro*; percent viability or percent killing of a target parasite *in vivo* or in vitro; percent viability of eggs of a target parasite; percent of a host population that is cured of an infestation by a target parasite; percent of a host population that is protected against infection by a target parasite (prophylactic effect); perturbation of a cell message or cell signal in a target parasite, such as, e.g., calcium, cyclic-AMP, and the like; and diminution of activity or downstream effects of a molecular target in a target parasite.

An exemplary *in vivo* method for assessing the anti-parasitic effect of a particular composition, or component of the composition, can be conducted using host animals. The host animals are infected with a target parasite. The composition or component of interest is administered to the host animal. Administration of the composition or component of interest can be initiated at various times before and/or after infection of the host animal, depending on the target parasite being tested. The eggs generated by the parasite in the host animal are quantified. For example, the eggs in a stool sample collected from the animal can be quantified. The quantification of eggs generated by the parasite in the host animal receiving the composition or component of interest can be compared the quantification of eggs generated by the parasite in another host animal, such as a host animal receiving another composition or component of interest, or a host animal serving as a control, e.g., uninfected control, or untreated control.

An exemplary *in vitro* method for assessing the anti-parasitic effect of a particular composition or component can be conducted using target parasites provided in test plates. The composition or component of interest is contacted with the target parasites, and the effect is observed, e.g., the effect of the composition or component of interest on the vitality of the target parasites. The effect of the treatment on the target parasites can be compared to the effect of another treatment on target parasites, such as target parasites treated with another composition or component of interest, or target parasites serving as a control, e.g., uninfected control, or untreated control.

Other methods can be used to assess the anti-parasitic effect of a particular composition or component, which methods will be evident to one of ordinary skill in the art, or can be determined for use in a particular case by one of ordinary skill in the art using only routine experimentation. Additional information related to assessing anti-parasitic effect can be found in the Examples set forth in this document.

In some embodiments of the present disclosure, a synergistic anti-parasitic effect is achieved when certain compounds are blended, and the synergistic effect can be enhanced when certain compounds are blended in certain relative amounts or ratios. In other words, the compositions including certain combinations of the compounds can have an enhanced ability to treat parasitic infections, as compared to each of the compounds taken alone.

As used herein, "synergy" and "synergistic effect" can refer to any substantial enhancement, in a composition of at least two compounds, of a measurable effect, e.g., an anti-parasitic effect, when compared with the effect of a component of the composition, e.g., one active compound alone, or the complete blend of compounds minus at least one compound. Synergy is a specific feature of a blend of compounds, and is above any background level of enhancement that would be due solely to, e.g., additive effects of any random combination of ingredients.

In some embodiments of the present disclosure, a substantial enhancement of a measurable effect can be expressed as a coefficient of synergy. A coefficient of synergy is an expression of a comparison between measured effects of a composition and measured effects of a comparison composition. The comparison composition can be a component of the composition. In some embodiments of the present disclosure, the synergy coefficient can be adjusted for differences in concentration of the complete blend and the comparison composition.

Synergy coefficients can be calculated as follows. An activity ratio (R) can be calculated by dividing the % effect of the composition (A_{B}) by the % effect of the comparison composition (Xₙ), as follows: $R = {A}_{B} / {X}_{n}$

A concentration adjustment factor (F) can be calculated based on the concentration (Cₙ), i.e., % (wt/wt) or % (vol/vol), of the comparison composition in the composition, as follows: $F = 100 / {C}_{n}$

The synergy coefficient (S) can then be calculated by multiplying the activity ratio (R) and the concentration adjustment factor (F), as follows: $S = \left(R\right) \left(F\right)$

As such, the synergy coefficient (S) can also by calculated, as follows: $S = \left[\left({A}_{B}/{X}_{n}\right)\left(100\right)\right] / {C}_{n}$

In Formula 4, A_{B} is expressed as % effect of the blend, Xₙ is expressed as % effect of the comparison composition (Xₙ), and Cₙ is expressed as % (wt/wt) or % (vol/vol) concentration of the comparison composition in the blend.

In some embodiments of the present disclosure, a coefficient of synergy of about 1.1, 1.2, 1.3, 1.4, or 1.5 can be substantial and commercially desirable. In other embodiments of the present disclosure, the coefficient of synergy can be from about 1.6 to about 5, including but not limited to about 1.8, 2.0, 2.5, 3.0, 3.5, 4.0, and 4.5. In other embodiments of the present disclosure, the coefficient of synergy can be from about 5 to 50, including but not limited to about 10, 15, 20, 25, 30, 35, 40, and 45. In other embodiments of the present disclosure, the coefficient of synergy can be from about 50 to about 500, or more, including but not limited to about 50, 75, 100, 125, 150, 200, 250, 300, 350, 400, and 450. Any coefficient of synergy above 500 is also contemplated within embodiments of the compositions.

Given that a broad range of synergies can be found in various embodiments describe herein, it is expressly noted that a coefficient of synergy can be described as being "greater than" a given number and therefore not necessarily limited to being within the bounds of a range having a lower and an upper numerical limit. Likewise, in some embodiments described herein, certain low synergy coefficients, or lower ends of ranges, are expressly excluded. Accordingly, in some embodiments of the present disclosure, synergy can be expressed as being "greater than" a given number that constitutes a lower limit of synergy for such an embodiment. For example, in some embodiments of the present disclosure, the synergy coefficient is equal to or greater than 25; in such an embodiment, all synergy coefficients below 25, even though substantial, are expressly excluded.

In some embodiments of the present disclosure, synergy or synergistic effect associated with a composition can be determined using calculations similar to those described in Colby, S. R., "Calculating synergistic and antagonistic responses of herbicide combinations," Weeds (1967) 15:1, pp. 20-22. In this regard, the following formula can be used to express an expected % effect (E) of a composition including two compounds, Compound X and Compound Y: $E = X + Y - \left(X*Y/100\right)$

In Formula 5, X is the measured actual % effect of Compound X in the composition, and Y is the measured actual % effect of Compound Y of the composition. The expected % effect (E) of the composition is then compared to a measured actual % effect (A) of the composition. If the actual % effect (A) that is measured differs from the expected % effect (E) as calculated by the formula, then the difference is due to an interaction of the compounds. Thus, the composition has synergy (a positive interaction of the compounds) when A > E. Further, there is a negative interaction (antagonism) when A < E.

Formula 5 can be extended to account for any number of compounds in a composition; however it becomes more complex as it is expanded, as is illustrated by the following formula for a composition including three compounds, Compound X, Compound Y, and Compound Z: $E = X + Y + Z - \left(\left(XY+XZ+YZ\right)/100\right) + \left(X*Y*Z/10000\right)$

An easy-to-use formula that accommodates compositions with any number of compounds can be provided by modifying Formulas 5 and 6. Such a modification of the formula will now be described. When using Formulas 5 and 6, an untreated control value (untreated with composition or compound) is set at 100%, e.g., if the effect being measured is the amount of target parasites killed, the control value would be set at 100% survival of target parasite. In this regard, if treatment with Compound A results in 80% killing of a target parasite, then the treatment with Compound A can be said to result in a 20% survival, or 20%> of the control value. The relationship between values expressed as a percent effect and values expressed as a percent-of-control are set forth in the following formulas, where E' is the expected % of control of the composition, Xₙ is the measured actual % effect of an individual compound (Compound Xₙ) of the composition, Xₙ' is the % of control of an individual compound of the composition, and A' is the actual measured % of control of the of the composition. $E = 100 - {E}^{′}$ ${X}_{n} = 100 = {{X}_{n}}^{′}$ $A = 100 - {A}^{′}$

By substituting the percent-of-control values for the percent effect values of Formulas 5 and 6, and making modifications to accommodate any number (n) of compounds, the following formula is provided for calculating the expected % of control (E') of the composition: ${E}^{′} = \left({\prod }_{i = 1}^{n} {{x}_{i}}^{′}\right) \div {100}^{n - 1}$

According to Formula 10, the expected % of control (E') for the composition is calculated by dividing the product of the measured actual % of control values (Xₙ') for each compound of the composition by 100ⁿ⁻¹. The expected % of control (E') of the composition is then compared to the measured actual % of control (A') of the composition. If the actual % of control (A') that is measured differs from the expected % of control (E') as calculated by the Formula 10, then the difference is due to an interaction of the compounds. Thus, the composition has synergy (a positive interaction of the compounds) when A' < E'. Further, there is a negative interaction (antagonism) when A'> E'.

Compositions containing two or more compounds in certain ratios or relative amounts can be tested for a synergistic effect by comparing the anti-parasitic effect of a particular composition of compounds to the anti-parasitic effect of a component the composition. Additional information related to making a synergy determination can be found in the Examples set forth in this document.

It is contemplated that the compositions of the presently-disclosed subject matter can be formulated for and delivered by carriers, including food products. For example, additives are added to baked goods, such as cookies, breads, cakes, etc., to enhance or modify flavor or color, increase shelf life, enhance their nutritional value, and generally produce a desired effect. Similarly, compositions of the presently-disclosed subject matter can be formulated with food products as carriers and delivered by ingestion to produce their desired effect. Of course, numerous types of foods can be used to deliver the compositions, including but not limited to: beverages, breakfast cereals, and powdered drink mixes.

Further, the compositions disclosed herein can take such forms as suspensions, solutions or emulsions in oily or aqueous carriers, and can contain formulatory agents such as suspending, stabilizing and/or dispersing agents. For oral administration, the compositions can take the form of, for example, tablets or capsules prepared by a conventional technique with pharmaceutically acceptable excipients such as binding agents (e.g., pregelatinized maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycollate); or wetting agents (e.g., sodium lauryl sulphate). The tablets can be coated by methods known in the art. For example, a composition disclosed herein can be formulated having an enteric or delayed release coating which protects the composition until it reaches the colon.

Liquid preparations for oral administration can take the form of, for example, solutions, syrups or suspensions. Such liquid preparations can be prepared by conventional techniques with pharmaceutically acceptable additives such as suspending agents (e.g., sorbitol syrup, cellulose derivatives or hydrogenated edible fats); emulsifying agents (e.g. lecithin or acacia); non-aqueous vehicles (e.g., almond oil, oily esters, ethyl alcohol or fractionated vegetable oils); and preservatives (e.g., methyl or propyl-*p*-hydroxybenzoates or sorbic acid). Liquid preparations for oral administration can also be formulated for delayed release, such as for example in "gel caps".

In certain embodiments of the present disclosure, the compositions can be provided in an encapsulated or microencapsulated form. Microencapsulation is a process where small particles of the composition are coated or encapsulated with an outer shell material for controlling the release of the composition or for protecting the composition. Exemplary outer shell material includes proteins, polysaccharides, starches, waxes, fats, natural and synthetic polymers, and resins. Microencapsulation can be done either chemically or physically. For example, physical methods of encapsulating the compositions can include: spray drying, spray chilling, pan coating, or coextrusion. Chemical methods of encapsulation can include coacervation, phase separation, solvent extraction, or solvent evaporation.

As one example, for coextrusion of a liquid core, liquid core and shell materials are pumped through concentric orifices, with the core material flowing in the central orifice, and the shell material flowing through the outer annulus. An enclosed compound drop is formed when a droplet of core fluid is encased by a layer of shell fluid. The shell is then hardened by appropriate means; for example, by chemical cross-linking in the case of polymers, cooling in the case of fats or waxes, or solvent evaporation. Additional information about methods and systems for providing compositions formulated for and delivered via food products can be found in U.S. Patent Nos. 5,418,010, 5,407,609, 4,211,668, 3,971,852, and 3,943,063.

The compositions of the presently-disclosed subject matter can be used for treating parasitic infections. The presently-disclosed subject matter includes methods for treating a parasitic infection in a subject, including administering an effective amount of a composition described herein.

As used herein, the terms "host" and "subject" are used interchangeably and refer to a plant or an animal capable of being infected by a parasite. The animal can be a vertebrate. The vertebrate can be warm-blooded. The warm-blooded vertebrate can be a mammal. The mammal can be a human. The human can be an adult or a child. As used herein, the terms "host" and "subject" include human and animal hosts and subjects. Thus, veterinary therapeutic uses are provided in accordance with the presently-disclosed subject matter. As such, the presently-disclosed subject matter provides for the treatment of mammals such as humans, as well as those mammals of importance due to being endangered, such as Siberian tigers or snow leopards; of economic importance, such as animals raised on farms for consumption by humans; and/or animals of social importance to humans, such as animals kept as pets or in zoos. Examples of such animals include but are not limited to: carnivores such as cats and dogs; swine, including pigs, hogs, and wild boars; ruminants and/or ungulates such as cattle, oxen, sheep, giraffes, deer, goats, bison, and camels; and horses. Also provided is the treatment of birds, including the treatment of those kinds of birds that are endangered and/or kept in zoos, as well as fowl, and more particularly domesticated fowl, i.e., poultry, such as turkeys, chickens, ducks, geese, guinea fowl, and the like, as they are also of economic importance to humans. Thus, also provided is the treatment of livestock, including, but not limited to, domesticated swine, ruminants, ungulates, horses (including race horses), poultry, and the like.

As used herein, the terms "treat," "treating," and "treatment" refer to: conferring protection against infection; preventing infection; alleviating infection; reducing the severity of symptoms and/or sequelae of infection; eliminating infection; and/or preventing relapse of infection. As used herein, the terms "treat," "treating," and "treatment" also refer to conferring protection against, preventing, alleviating, reducing the severity of, eliminating, and/or preventing relapse associated with a disease or symptoms caused by a parasitic infection.

As used herein, the term "effective amount" refers to a dosage sufficient to provide treatment for a parasitic infection. The exact amount that is required can vary, for example, depending on the target parasite, the treatment being affected, age and general condition of the subject, the particular formulation being used, the mode of administration, and the like. As such, the effective amount will vary based on the particular circumstances, and an appropriate effective amount can be determined in a particular case by one of ordinary skill in the art using only routine experimentation.

The presently-disclosed subject matter includes methods of screening for compositions useful for treating a parasitic infection. In some embodiments of the present disclosure, the screening method is useful for narrowing the scope of possible compounds that are identified as components for a composition for treating a parasitic infection.

In some embodiments of the present disclosure, a method of selecting a composition for use in treating a parasitic infection includes the following. A cell expressing a tyramine receptor is provided and is contacted with test compounds. The receptor binding affinity of the compounds is measured. At least one parameter selected from the following parameters is measured: intracellular cAMP level, and intracellular Ca²⁺ level. A first compound for the composition is identified, which is capable of altering at least one of the parameters, and which has a high receptor binding affinity for the tyramine receptor; and a second compound for the composition is identified, which is capable of altering at least one of the parameters, and which has a low receptor binding affinity for the tyramine receptor. A composition is selected that includes the first and second compounds. In some embodiments of the present disclosure, a composition is selected that includes the first and second compounds and demonstrates an anti-parasitic effect that exceeds the anti-parasitic effect of any of the compounds when used alone.

The cell used for the method can be any cell capable of being transfected with and express a Tyramine Receptor (TyrR). Examples of cells include, but are not limited to: insect cells, such as *Drosophila* Schneider cells, *Drosophila* Schneider 2 cells (S2 cells), and *Spodoptera frugiperda* cells (e.g., Sf9 or Sf21); or mammalian cells, such as Human Embryonic Kidney cells (HEK-293 cells), African green monkey kidney fibroblast cells (COS-7 cells), HeLa Cells, and Human Keratinocyte cells (HaCaT cells). Additional information about preparing cells expressing receptors can be found in U.S. Patent Publication Serial Nos.US 2005-0008714;US 2005-0214267; and US 2006-0263403.

The tyramine receptor (TyrR) can be a full-length TyrR, a functional fragment of a TyrR, or a functional variant of a TyrR. A functional fragment of a TyrR is a TyrR in which amino acid residues are deleted as compared to the reference polypeptide, i.e., full-length TyrR, but where the remaining amino acid sequence retains the binding affinity of the reference polypeptide for tyramine. A functional variant of a TyrR is a TyrR with amino acid insertions, amino acid deletions, or conservative amino acid substitutions, which retains the binding affinity of the reference polypeptide for tyramine. A "conservative amino acid substitution" is a substitution of an amino acid residue with a functionally similar residue. Examples of conservative substitutions include the substitution of one non-polar (hydrophobic) residue such as isoleucine, valine, leucine or methionine for another; the substitution of one polar (hydrophilic) residue for another such as between arginine and lysine, between glutamine and asparagine, between glycine and serine; the substitution of one basic residue such as lysine, arginine or histidine for another; or the substitution of one acidic residue, such as aspartic acid or glutamic acid for another. A conservative amino acid substitution also includes replacing a residue with a chemically derivatized residue, provided that the resulting retains the binding affinity of the reference polypeptide for tyramine. Examples of TyrRs include, but are not limited to: TyrRs, such as, *Drosophila melanogaster* TyrR (GENBANK® accession number (GAN) CAA38565), *Locusta migratoria* TyrR (GAN: Q25321), TyrRs of other invertebrates, and TyrRs of nematodes, including *Ascaris.*

In some embodiments of the present disclosure, other receptors, such as G-protein coupled receptors (GPCRs), whether having native affinity for tyramine or other ligands, can be employed in methods of screening for compositions useful for treating a parasitic infection. Examples of receptors that can be used include, but are not limited to: *Anopheles gambiae* (GAN: EAA07468), *Heliothis virescens* (GAN: Q25188), *Mamestra brassicae* (GAN: AAK14402), *Tribolium castaneum* (GAN: XP_970290), *Aedes aegypti* (GAN: EAT41524), *Boophilus microplus* (GAN: CAA09335); *Schistosoma mansoni* (GAN: AAF73286); and *Schistosoma mansoni* (GAN: AAW21822).

In some embodiments of the present disclosure, receptors of the nuclear hormone receptor superfamily can be employed in methods of screening for compositions useful for treating a parasitic infection. Examples of receptors that can be used include, but are not limited to receptors from parasites or invertebrates that are analogous to the DAF family of nuclear receptors such as DAF-2 and DAF-12. In other embodiments of the present disclosure, nuclear receptor proteins from Drosophila or other invertebrate can be employed, such as: nuclear receptors of subfamily 1 such as E78, E75, DHR3, EcR, and DHR96; nuclear receptors of subfamily 2 such as USP, DHR78, HNF4, SVP, TLL, DSF, DHR51, or DHR83; nuclear receptors of subfamily 3 such as ERR, nuclear receptors of subfamily 4 such as DHR38; nuclear receptors of subfamily 5 such as FTZ-F1 or DHR39; or nuclear receptors of subfamily 6 such as DHR4. In other embodiments of the present disclosure, invertebrate or parasite nuclear receptor proteins analogous to certain human nuclear receptors can be employed, such as: nuclear receptors of subfamily 1 such as PPAR, RAR, TR, REV-ERB, ROR, FXR, LXR, VDR, SXR, or CAR; nuclear receptors of subfamily 2 such as RXR, TR2/TR4, HNF4, COUP-TF,TLX, or PNR; nuclear receptors of subfamily 3 such as ERR, ER, or MR/PR/AR/GR; nuclear receptors of subfamily 4 such as NURRI/NGFIB; nuclear receptors of subfamily 5 such as LRH/SF1; or nuclear receptors of subfamily 6 such as GCNF. In other embodiments of the present disclosure, invertebrate or parasite nuclear receptor proteins having as their native ligand naturally occurring hormones such as la, 25(OH)2-vitamin D3, 17p-oestradiol, testosterone, progesterone, cortisol, aldosterone, *all-trans* retinoic acid, 3,5,3'-L-triiodothyronine, cc-ecdysone, or brassinolide, among others, can be employed.

In other embodiments of the present disclosure, invertebrate or parasite nuclear receptor proteins analogous to certain human nuclear receptors can be employed, such as the receptors listed in Table F below. In the Table, *a, b* and *g* correspond to the Greek letters α, β and gamma, respectively.

| **TABLE F** | | | |
|---|---|---|---|
| **Subfamilies and Group** | **Genes** | **Trivial Names** | **Accession numbers** |
| 1A | NR1A1 | thyroid hormone receptor, TR*a*, c-erbA-1, THRA | M24748 |
| | NR1A2 | thyroid hormone receptor, TR*b*, c-erbA-2, THRB | X04707 |
| IB | NR1B1 | retinoic acid receptor, RAR*a* | X06538 |
| | NR1B2 | retinoic acid receptor, RAR*b*, HAP | Y00291 |
| | NR1B3 | retinoic acid receptor, RAR*g*, RARD | M57707 |
| | NR1B4 | retinoic acid receptor, RAR | AF378827 |
| 1C | NR1C1 | peroxisomeproliferator -activated receptor, PPAR*a* | L02932 |
| | NR1C2 | peroxisomeproliferator -activated receptor, PPAR*b*, NUC1, PPAR*d*, FAAR | L07592 |
| | NR1C3 | peroxisomeproliferator-activated receptor, PPAR*g* | L40904 |
| ID | NR1D1 | reverse erbA, REVERB*a*, EAR1, EAR1A | M24898 |
| | NR1D2 | reverse erbA, REVERB*b*, EAR 1*b*, BD73, RVR, HZF2 | L31785 |
| | NR1D3 | reverse erbA, E75 | X51548 |
| IE | NR1E1 | E78, DR-78 | U01087 |
| IF | NR1F1 | RAR-related orphan receptor, ROR*a*, | U04897 |
| | | RZR*a* | |
| | NR1F2 ; | RAR-related orphan receptor, ROR*b*, RZR*b* | Y08639 |
| | NR1F3 | RAR-related orphan receptor, ROR*g*, TOR | U16997 |
| | NR1F4 | HR3, DHR3, MHR3, GHR3 | M90806 |
| | | CNR3, CHR3 | U13075 |
| 1G | NR1G1 | CNR 14 | U13074 |
| 1H | NR1H1 | ECR | M74078 |
| | NR1H2 | Liver X receptor, UR, OR-1, NER1, RIP15, LXR*b* | U07132 |
| | NR1H3 | Liver X receptor, RLD1, LXR, LXR*a* | U22662 |
| | NR1H4 | Farnesoid X receptor, FXR, RIP14, HRR1 | U09416 |
| | NR1H5 | Farnesoid X receptor, FXRB | A Y094586 |
| 1I | NR1I1 | Vitamin D receptor, VDR | J03258 |
| | NR1I2 | Pregnane X receptor, ONR1, PXR, SXR, BXR | X75163 |
| | NR1I3 | Constitutive androstane receptor, MB67,CAR1,CAR*a* | Z30425 |
| | NR1I4 | CAR2, CAR*b* | AF009327 |
| U | NR1J1 | DHR96 | U36792 |
| IK | NR1K1 | NHR1 | U19360 |
| 2A | NR2A1 | Human nuclear factor 4, HNF4 | X76930 |
| | NR2A2 | Human nuclear factor 4, HNF4G | Z49826 |
| | NR2A3 | HNF4B | Z49827 |
| | NR2A4 | DHNF4, HNF4D | U70874 |
| 2B | NR2B1 | Retinoid X receptor, RXRA | X52773 |
| | NR2B2 | Retinoid X receptor, RXRB, H-2RIIBP, RCoR-1 | M84820 |
| | NR2B3 | Retinoid X receptor, RXRG | X66225 |
| | NR2B4 | USP, Ultraspiracle, 2C1, CF1, RXR1, RXR2 | X52591 |
| 2C | NR2C1 | Testis receptor, TR2, TR2-11 | M29960 |
| | NR2C2 | Testis receptor, TR4, TAK1 | L27586 |
| | NR2C3 | TR2-4 | AF378828 |
| 2D | NR2D1 | DHR78 | U36791 |
| 2E | NR2E1 | TLL, TLX, XTLL | S72373 |
| | NR2E2 | TLL, Tailless | M34639 |
| | NR2E3 | Photoreceptor-specific nuclear receptor, PNR | AF121129 |
| | NR2E4 | dissatisfaction | 096680 |
| | NR2E5 | fax-1 | Q9U4I0 |
| 2F | NR2F1 | Chicken ovalbumin upstream promoter- transcription factor, COUP-TFI, COUPTFA, EAR3, SVP44 | XI2795 |
| | NR2F2 | Chicken ovalbumin upstream promoter- transcription factor, COUP-TFII, COUPTFB, ARP1, SVP40 | M64497 |
| | NR2F3 | SVP, COUP-TF | M28863 |
| | NR2F4 | COUP-TFIII, COUPTFG | X63092 |
| | NR2F5 | SVP46 | X70300 |
| | NR2F6 | ErbA2-related gene 2, EAR2 | XI2794 |
| | NR2F7 | AmNR7 | AF323687 |
| 2G | NR2G1 | HNF,RXR | AJ517420 |
| 2H | NR2H1 | AmNR4, AmNR8 | AF323683 |
| 3A | NR3A1 | ERa | X03635 |
| | NR3A2 | ERb | U57439 |
| 3B | NR3B1 | ERR1,ERRa | X51416 |
| | NR3B2 | ERR2, ERRb | X51417 |
| | NR3B3 | ERR3, ERRg | AF094318 |
| | NR3B4 | Drosophila ERR | AE003556 |
| 3C | NR3C1 | GR | X03225 |
| | NR3C2 | MR | M16801 |
| | NR3C3 | PR | M15716 |
| | NR3C4 | AR | M20132 |
| 4A | NR4A1 | NGFIB, TR3, N10, NUR77, NAK1 | LI3740 |
| | NR4A2 | NURR1, NOT, RNR1, HZF-3, TINOR | X75918 |
| | NR4A3 | NOR 1, MINOR | D38530 |
| | NR4A4 | DHR38, NGFIB | U36762 |
| | | CNR8, C48D5 | U13076 |
| 5A | NR5A1 | SF1,ELP,FTZ-F1,AD4BP | D88155 |
| | NR5A2 | LRH1, xFF1rA, xFF1rB, FFLR, PHR, | U93553 |
| | | FTF | |
| | NR5A3 | FTZ -F1 | M63711 |
| | NR5A4 | 4FF1b | Q9IAI9 |
| 5B | NR5B1 | DHR39,FTZF1B | L06423 |
| 6A | NR6A1 | GCNF1,RTR | U14666 |
| | NR6A2 | HR4, THR4, GRF | AL035245 |
| 0A | NR0A1 | KNI,Knirps | X13331 |
| | NR0A2 | KNRL, Knirps related | X14153 |
| | NR0A3 | EGON, Embryonic gonad, EAGLE | X16631 |
| | NR0A4 | ODR7 | U16708 |
| | NR0A5 | Trithorax | M31617 |
| 0B | NR0B1 | DAX1,AHCH | S74720 |
| | NR0B2 | SHP | L76571 |

When such nuclear receptors are employed in a screening platform, known downstream effects of the receptors can be used as indicative of an effect of an agent or blend of agents on the receptor. For example, levels of RNA transcribed from known targets of activated receptors can be assessed, or downstream effects of known regulatory cascades can be assessed.

Other molecular targets of interest include those listed in Table G:

| **TABLE G** | | |
|---|---|---|
| **Organism** | **Molecular Target** | **Reason for targeting** |
| *Entamoeba histolytica* | Sialidase | Motility of intact *E. hystolytica* cells was enhanced by 0.05-0.1 mM Neu5Acα2,31ac, 4-MU-Neu5Ac and fetuin. However, the motility of the parasite was highly diminished when incubated with Neu5Acα2en and sialic acid-containing compounds. Lysed *E*. *histolytica* trophozoites were found to lack neuraminic acid. Nok, A.J., Parasitol Res. 2003 Mar;89(4):302-7. |
| | serine-rich E. histolytica protein (SREHP) | An antibody to SREHP blocked lectin independent uptake of apoptotic cells, with >90% inhibition at a dose of 20 microg/ml. The same antibody also inhibited adherence to apoptotic lymphocytes, and, to a lesser extent, adherence to and killing of viable lymphocytes. Teixeira, J.E. Infect Immun. 2007 Dec 17 [Epub ahead of print]. |
| | amebic galactose-specific lectin galactose/N-acetyl-D-galactosamine-inhibitable lectin (Gal-lectin) | Prior to phagocytosis of host cells, *E*. *histolytica* induces apoptotic host cell death using a mechanism that requires contact via an amebic galactose-specific lectin. Teixeira, J.E. Infect Immun. 2007 Dec 17 [Epub ahead of print]. |
| | | The Gal-lectin is a protein involved in parasite virulence and adherence and is known to activate immune cells. Ivory, C.P., Infect Immun. 2007 Oct;75(10):4917-22. |
| | | Initiation of inflammation and cell death during liver abscess formation by *Entamoeba histolytica* depends on activity of the galactose/N-acetyl-D-galactosamine lectin. Blazquez, S. Int J Parasitol. 2007 Mar;37(3-4):425-33. |
| | KERP1 | Experimentally induced liver abscesses reveal a parallel between the intricate upregulation of KERP1 gene expression during abscess development and the increased abundance of KERP1 in virulent trophozoites. Trophozoites affected in kerp1 expression by an antisense strategy were unable to form liver abscesses. Santi-Rocca, J., Cell Microbiol. 2008 Jan;10(1):202-17. Epub 2007 Aug 17. |
| | pyruvate phosphate dikinase | Pyruvate phosphate dikinase (PPDK) is the key enzyme essential for the glycolytic pathway in most common and perilous parasite Entamoeba histolytica. Inhibiting the function of this enzyme will control the wide spread of intestinal infections caused by *Entamoeba histolytica* in humans. Stephen, P. J Comput Aided Mol Des. 2007 Aug 21 |
| | glyceraldehyde-3 - phosphate dehydrogenase | Glyceraldehyde-3 -phosphate dehydrogenase (GAPDH) of *Entamoeba histolytica* (Eh) is a major glycolytic enzyme and an attractive drug target since this parasite lacks a functional citric acid cycle and is dependent solely on glycolysis for its energy requirements. Kundu, S., J Biomol Struct Dyn. 2007 Aug;25(1):25-33 [Epub ahead of print]. |
| | 140kDaFN-binding molecule (EhFNR) | EhFNR (Igl) plays an important role in the adhesion process during abscess development. EhFNR is specifically regulated in FN-interacted amoebas, as well as in trophozoites recovered at different stages of abscess development. This regulation involves mobilization of the receptor molecule from internal vesicles to the plasma membrane. Hemandez-Ramirez VI, Parasitology. 2007 Feb;134(Pt 2): 169-77. |
| *Giardia lamblia* | aurora kinase | During interphase, *Giardia* aurora kinase (gAK) localises exclusively to the nuclei, but is not phosphorylated. During mitosis phosphorylated aurora kinase (pAK) localises to the basal bodies/centrosomes and co-localises with tubulin to the spindle. During specific stages of mitosis, giardial pAK also localises dynamically to cytoskeletal structures unique to Giardia: the paraflagellar dense rods of the anterior flagella and the median body, as well as to the parent attachment disc. Two AK inhibitors significantly decreased giardial growth and increased the numbers of cells arrested in cytokinesis. These inhibitors appeared to increase microtubule nucleation and cell-ploidy. Davids, B.J., Int J Parasitol. 2007 Sep 21 [Epub ahead of print] |
| | α 14-Giardin (annexin E1) | Alpha 14-Giardin (annexin E1) is specifically localized to the flagella and to the median body of the trophozoites. Alpha 14-Giardin resides at local slubs near the proximal part and the ends of the flagella. Vahrmann, A., Parasitol Res. 2008 Jan;102(2):321-6. Epub 2007 Oct 17. |
| | dynamin-related protein (G1DRP) | G1DRP is necessary for secretion of the cyst wall material and ESV homeostasis. G1DRP colocalizes with clathrin at the cell periphery and is necessary for endocytosis of surface proteins to endosomal-lysosomal organelles in trophozoites. Gaechter, V., Traffic. 2008 Jan;9(1):57-71. Epub 2007 Oct 31. |
| | Nitroreductase (G1NR1) | Antigiardial activity of thiazolides, represented by the nitrothiazole analogue nitazoxanide [NTZ; 2-acetolyloxy-N-(5-nitro-2-thiazolyl)benzamide] is at least partially mediated through inhibition of G1NR1. Miller, J., Antimicrob Agents Chemother. 2007 Jun;51(6): 1979-86. |
| | UDP-N-acetylglucosamine 4'-epimerase | The Giardia epimerase catalyzes the reversible epimerization of UDP-N-acetylglucosamine to UDP-N-acetylgalactosamine, which forms the ultimate regulatory step in cyst wall biosynthesis. Lopez, A.B., J Eukaryot Microbiol. 2007 Mar-Apr;54(2): 154-60. |
| *Cryptosporidium muris* | CM250 | CM250 is found in electron-dense vesicles and cytoplasm of developing macrogametocytes, and ultimately localizes to the oocyst wall of mature oocysts of both *C. muris* and *C. parvum.* Ju, J.R., Parasitol Res. 2002 Can;88(5):412-20. |
| *Cryptosporidium parvum* | thrombospondin-related protein CpMICl (CpTSP8) | *Cryptosporidium parvum* encodes 11 thrombospondin-related proteins (CpTSP2 through CpTSP12). The thrombospondin-related protein CpMIC1 (CpTSP8) belongs to the repertoire of micronemal proteins of *Cryptosporidium parvum.* CpTSP8 localizes to the apical complex of both sporozoites and type I merozoites, and upon sporozoite exposure to host cells in vitro, the protein is translocated onto the parasite surface as typical of micronemal proteins (MICs). Putignani, L., Mol Biochem Parasitol. 2008 Jan;157(1):98-101. Epub 2007 Sep 29. |
| | p30 | p30 is a 30-kDa Gal/GalNAc-specific lectin isolated from *C. parvum* and *Cryptosporidium hominis* sporozoites. The p30 gene is expressed at 24-72 h after infection of intestinal epithelial cells. p30 localizes to the apical region of sporozoites and is predominantly intracellular in both sporozoites and intracellular stages of the parasite. p30 associates with gp900 and gp40, Gal/GalNAc- containing mucin-like glycoproteins that are also implicated in mediating infection. Bhat, N., J. Biol Chem. 2007 Nov 30;282(48):34877-87. |
| | Cpal35 | Cpal35 is expressed and secreted through the apical complex at the invasive stage of sporozoite. This protein is characterised by an LCCL domain, a common trait of various secreted proteins within Apicomplexa. Cpal35 orthologous genes in four apicomplexan species (*Plasmodium falciparum, Theileria parva, Toxoplasma gondii* and *Eimeria tenella*) have been identified. The architecture of the deduced proteins shows that the Cpal35-related proteins are a distinct family among the apicomplexan LCCL proteins. Tosini, F., et al., Parassitologia. 2006 Jun;48(1-2):105-7. |
| *Trypanosomatidae cruzi* | TcRBP19 | TcRBP19 is a 17 kDa RNA-binding protein from *Trypanosoma cruzi* containing an RNA recognition motive (RRM). TcRBP19 shows target selectivity since among the different homoribopolymers it preferentially binds polyC. TcRBP19 is a low expression protein only barely detected at the amastigote stage, and localizes in a diffuse pattern in the cytoplasm. Perez-Diaz, L., Exp Parasitol. 2007 Sep;117(1):99-105. Epub 2007 Mar 27. |
| | gp82 defined by monoclonal antibody 3F6 | A member of the *Trypanosoma cruzi* gp82 family, expressed on metacyclic trypomastigote surface and identified by monoclonal antibody (MAb) 3F6, plays a key role in host cell invasion. Host cell invasion of metacyclic forms was inhibited by MAb 3F6, recombinant protein including the epitope recognized by MAb 3F6, and a polyclonal antibody against the recombinant protein. Atayde, V.D., Infect Immun. 2007 Jul;75(7):3264-70. |
| | TcPIN1 | Parvulins are a conserved group ofpeptidylprolyl cis/trans isomerases (PPIases) that catalyze the cis/trans isomerization of proline-preceding peptide bonds. In *Trypanosoma cruzi,* parvulin TcPIN1 is a homolog of the human hPinl PPIase. The 117 amino acids of the TcPIN1 display 40% identity with the catalytic core of hPinl and exhibit prolyl cis/trans isomerase activity. The enzyme is present both in dividing and non-dividing forms of *T. cruzi.* Erben, E.D., Mol Biochem Parasitol. 2007 Jun; 153(2): 186-93. |
| | metacaspases TcMCA3 and TcMCA5 | Metacaspases TcMCA3 and TcMCA5 participate in programmed cell death induced by fresh human serum. |
| *Leishmania brucei* | OP-Tb | OP-Tb is a soluble serine oligopeptidase (OP-Tb) that is released into the host bloodstream during infection, where it has been postulated to participate in the pathogenesis of African trypanosomiasis. It has activity toward substrates of trypsin-like enzymes. Morty, R.E., J Biol Chem. 1999 Sep 10;274(37):26149-56. |
| *Leishmania spp.* | Major surface protease (MSP) | The *Leishmania spp.* protozoa have an abundant surface metalloprotease MSP (major surface protease), which in *Leishmania* chagasi is encoded by three distinct gene classes (MSPS, MSPL, MSPC). Although MSP has been characterized primarily in extracellular promastigotes, it also facilitates survival of intracellular amastigotes. Promastigotes express MSPS, MSPL, and two forms of MSPC RNAs, whereas amastigotes express only MSPL RNA and one MSPC transcript. More than 10 MSP isoforms are present in both amastigotes and promastigotes. Promastigote MSPs were N-glycosylated, whereas most amastigote MSPs were not. Two-thirds of the promastigote MSP is distributed along the cell surface. In contrast, most amastigote MSP localized at the flagellar pocket, the major site of *Leishmania* endocytosis/exocytosis. Most amastigote MSP is soluble in the cytosol, vesicles or organelles, whereas most promastigote MSP is membrane-associated and GPI anchored. Hsiao, C.H., Mol Biochem Parasitol. 2007 Oct 30 [Epub ahead of print]. |
| | UDP-galactopyranose mutase (GLF) | Considering the high incidence of galactofuranose (Gal(f)) in pathogens and its absence from higher eukaryotes, the enzymes involved in the biosynthesis of this unusual monosaccharide appear as attractive drug targets. UDP-galactopyranose mutases (GLF) holds a central role in Gal(f) metabolism by providing UDP-Gal(f) to all galactofuranosyltransferases. In *L. major,* Gal(f) is present in the membrane anchor of the lipophosphoglycan (LPG) and in glycoinositolphospholipids. Accordingly, the generated glf(-) mutant is deficient in LPG backbone and lead to an attenuation of virulence. Kleczka, B., et al, J. Biol. Chem. 2007 Apr;282(14): 10498-505. Epub 2007 Feb 6. |
| | Surface-metalloprotease (leishmanolysin) | Leishmanolysin is a virulence factor which contributes to a variety of functions including evasion of complement-mediated parasite-killing, host intramacrophage survival, and antimicobial peptide-mediated apoptotic killing. Kulkarni, M.M., et al., Mol Microbiol. 2006 Dec;62(5): 1484-97. Epub 2006 Oct 27. |
| *Toxoplasma gondii* | rhoptry proteins (ROPs) | ROPs include serine-threonine kinases and protein phosphatases. Secretory ROP kinases dramatically influence host gene expression and are the major parasite virulence factors. Bradley, P.J., and Sibley, L.D., Curr Opin Microbiol. 2007 Dec; 10(6):582-7. Epub 2007 Nov9. |
| | MIC2 | Reduced MIC2 expression resulted in mistrafficking of M2AP, markedly defective host-cell attachment and invasion, the loss of helical gliding motility, and the inability to support lethal infection in a murine model of acute toxoplasmosis. The MIC2 protein complex is a maj or virulence determinant for Toxoplasma infection Huynh, M.H., and Carruthers, V.B., PLoS Pathog. 2006 Aug;2(8):e84. |
| | Acyl Carrier Protein (ACP) | The acyl carrier protein is a central component of the apicoplast-localized fatty acid synthesis (FAS II) pathway of apicomplexan parasites. Loss of FAS II severely compromises parasite growth in culture. Maxumdar, J., et al, Proc Natl Acad Sci U S A. 2006 Aug 29;103(35):13192-7. Epub2006Aug 18. |
| *Plasmodium spp.* | Thromobospondin-related sporozoite protein (TRSP) | Analysis of TRSP knockout sporozoites *in vitro* and *in vivo* indicates that this protein has a significant role in hepatocyte entry and therefore liver infection. Thus, TRSP is an additional TSR-containing malaria parasite protein that is mainly involved in initial infection of the mammalian host. Labaied, M.. et al. Mol Biochem Parasitol. 2007 Jun; 153(2): 158-66. Epub 2007 Mar 6. |
| | Circumsporozoite protein (CSP) | To infect hepatocytes, sporozoites traverse Kupffer cells, but surprisingly, the parasites are not killed by these resident macrophages of the liver. Plasmodium sporozoites and circumsporozoite protein (CSP) suppress the respiratory burst in Kupffer cells. This allows the sporozoites to safely pass through these professional phagocytes and to develop inside neighbouring hepatocytes. Usynin, I., et al, Cell Microbiol. 2007 Nov;9(11):2610-28. Epub 2007 Jun 15. |
| | Duffy-binding-like erythrocyte-binding proteins (DBL-EBP) | Conserved cysteine-rich domains play important roles at critical times during this invasion process and at other stages in the life cycle of malaria parasites. Duffy-binding-like (DBL) domains, expressed as a part of the erythrocyte-binding proteins (DBL-EBP), are such essential cysteine-rich ligands that recognize specific host cell surface receptors. DBL-EBP, which are products of the erythrocyte-binding-like (ebl) gene family, act as critical determinants of erythrocyte specificity and are the best- defined ligands from invasive stages of malaria parasites. Michon, P., et al, Mol Biol Evol. 2002 Jul; 19(7): 1128-42. |
| *Babesia* | Thrombospondin related adhesive proteins (TRAPs) | TRAPs are well conserved among several apicomplexans. *B. gibsoni* TRAP (BgTRAP) showed a bivalent cation-independent binding to canine erythrocytes. BgTRAP is functionally important in merozoite invasion. Zhou, J., et al, Mol Biochem Parasitol. 2006 Aug;148(2):190-8. Epub 2006 Apr 21. |
| *Trichomonas vaginalis* | Cysteine proteases (CPs) | Several cysteine proteinases (CPs) participate in the virulence of *Trichomonas vaginalis.* CP30 is known to play a role in cytoadherence of the parasite to host cells. Mendoza-Lopez, M.R., et al., Infect Immun. 2000 Sep;68(9):4907-12. The CP39 proteinase bound to HeLa epithelial cells, vaginal epithelial cells (VECs), and human prostatic cancer cells (DU-145). CP39 degraded collagens I, III, IV, and V, human fibronectin, human hemoglobin, and human immunoglobulins A and G. Hernandez-Gutierrez, R., et al, Exp Parasitol. 2004 Jul-Aug; 107(3-4): 125-35. CP65 is a surface cysteine proteinase involved in cytotoxicity. It is immunogenic during human infection and degrades some extracellular matrix proteins. Alvarez-Sanchez, M.E., et al., Microb Pathog. 2000 Apr;28(4): 193-202. |
| | AP65 | Four trichomonad surface proteins bind VECs as adhesins, and AP65 is a major adhesin with sequence identity to an enzyme of the hydrogenosome organelle that is involved in energy generation. Reduction in parasite surface expression of AP65 was related to lower levels of adherence to vaginal epithelial cells (VECs). Mundodi, V., et al, Mol Microbiol. 2004 Aug;53(4):1099-108. |
| *Schistosoma spp.* | Ste20 group Serine/threonine kinases | Play important roles in various cellular functions such as growth, apoptosis and morphogenesis. Most of the Ste20-related proteins are active kinases known to regulate mitogen-activated protein kinase (MAPK) cascades. This family includes p21-Activated Kinases (PAKs) and Germinal Center Kinases (GCKs) families which contain their kinase domain in the C-terminal and N-terminal position, respectively. The GCK protein family could participate in the regulation of MAPK cascade activation during host-parasite interactions. Yan, Y., et al., Int J Parasitol. 2007 Dec;37(14): 1539-50. Epub 2007Jun21. |
| *Taenia spp.* | Taenia adhesion family (TAF) | Ts45W and Ts45S genes belong to the *Taenia ovis* 45 W gene family. These domains are expected to be responsible for the demonstrated cell adhesion and the protective nature of this family of molecules. These TAF proteins and HP6, can have evolved the dual functions of facilitating tissue invasion and stimulating protective immunity to first ensure primary infection and subsequently to establish a concomitant protective immunity to protect the host from death or debilitation through superinfection by subsequent infections and thus help ensure parasite survival. Gonzalez, L.M., et al., Parasitol Res. 2007 Feb;100(3):519-28. Epub 2006 Oct 18. |
| *Eimeria spp.* | Flottillin-1 | Flotillin-1, a resident protein of lipid rafts, was identified on *E*. *tenella* sporozoites and was prominently expressed at the apex of the cells, a region mediating host cell invasion. del Cacho, E., et al., J Parasitol. 2007 Apr;93(2):328-32. |
| *Fasciola spp.* | Excretory-secretory products (ESP) | ESP released by helminths have shown wide immunomodulatory properties, such as the induction of cellular apoptosis. Activation of protein tyrosine kinases and caspases are necessary to mediate apoptosis induced by the ESP, and carbohydrate components present in these antigens are involved in this effect. Serradell, M.C., et al., Vet Immunol Immunopathol. 2007 Jun 15; 117(3-4): 197-208. Epub 2007 Mar 25. |
| *Cladosporium spp.* | extracellular avirulence proteins (avrs) | Apart from triggering disease resistance, Avrs are believed to play a role in pathogenicity. The avirulence protein Avr4, which is a chitin-binding lectin containing an invertebrate chitin-binding domain (CBM14), protects chitin against hydrolysis by plant chitinases. van den Burg H.A., et al, Mol Plant Microbe Interact. 2006 Dec;19(12):1420-30. |
| *Colletotrichum spp.* | pH-responsive PacC/RimlOl transcription regulators | Gene disruption at the Pac(KLAP2) locus created fungal mutants that were hypersensitive to alkaline pH, altered in conidium and appressorium production and germination, and concomitant with reduced virulence. You, B.J., et al., Mol Plant Microbe Interact. 2007 Sep;20(9): 1149-60. |
| | cell wall assembly | ClaSSDl is a gene orthologous to *Saccharomyces cerevisiae* SSD1. Transmission electron microscopy suggested that appressorial penetration by classdl mutants was restricted by plant cell wall-associated defense responses, which were observed less frequently with the wild-type strain. Tanaka, S., et al., Mol Microbiol. 2007 Jun;64(5): 1332-49. |
| | STE12-like genes | Activity of a STE12-like gene (CLSTE12) can be modulated by a regulated alternative splicing mechanism and that this factor is involved in the production of cell surface proteins and host cell wall degrading enzymes. Hoi, J.W., et al., Mol Microbiol. 2007Apr;64(1):68-82. |
| *Neospora spp.* | cross-reactive membrane antigens | Pre-incubation of free tachyzoites with anti-rNcAMA1 IgG antibodies, apical membrane antigen 1 (NcAMAl), inhibited the invasion into host cells by *N. caninum* and *T. gondii.* Zhang, H., et al., Mol Biochem Parasitol. 2007 Feb;151(2):205-12. Epub 2006 Nov 30. |
| *Sarcocystis spp.* | nucleoside triphosphate hydrolase (NTPase) | Analyses of the SnNTPl protein demonstrated that it is soluble and secreted into the culture medium by extracellular merozoites. SnNTPl can play a role in events that occur during or proximal to merozoite egress from and/or invasion into cells. Zhang, D., et al., Int J Parasitol. 2006 Sep;36(10-11): 1197-204. Epub 2006 Jun 6. |
| *Ustilago maydis* | ferroxidation/permeation iron uptake system | Two components of a high-affinity iron uptake system: fer2, encoding a high-affinity iron permease; and ferl, encoding an iron multicopper oxidase. fer2 as well as ferl deletion mutants were strongly affected in virulence and highlights the importance of the high-affinity iron uptake system via an iron permease and a multicopper oxidase for biotrophic development in the U. *maydis*/*maize* (Zea cans) pathosystem. Eichhorn, H., et al., Plant Cell. 2006 Nov;18(11):3332-45. Epub 2006 Nov 30. |
| | Bizl | Mutant cells show a severe reduction in appressoria formation and plant penetration, and those hyphae that invade the plant arrest their pathogenic development directly after plant penetration, bizl is induced via the b-mating-type locus, the key control instance for pathogenic development. The gene is expressed at high levels throughout pathogenic development, which induces a G2 cell cycle arrest that is a direct consequence of the downregulation of the mitotic cyclin Clbl. Flor-Parra, I., et al., Plant Cell. 2006 Sep;18(9):2369-87. Epub 2006 Aug 11. |
| *Magnaporthe grisea* | snodprotl family | The snodprotl homolog, MSP1, in the rice blast fungus. Deletion mutants were greatly reduced in virulence primarily due to impaired growth in planta. Western blot analysis showed that the protein was secreted and not associated with the fungal cell wall. Jeong, J.S., et al., FEMS Microbial Lett. 2007 Aug;273(2): 157-65. Epub 2007 Jun 21. |
| | ABC transporters (ABC1) | The ABC1 insertional mutant and a gene-replacement mutant arrest growth and die shortly after penetrating either rice or barley epidermal cells, ABC1 mutants are not hypersensitive to antifungal compounds. Data strongly suggests that *M. grisea* requires the up- regulation of specific ABC transporters for pathogenesis; most likely to protect itself against plant defense mechanisms. Urban, M., et al., EMBO J. 1999 Feb 1; 18(3):512-21. |
| *Fusarium spp.* | secreted lipase (FGL1) | Extracellular lipolytic activity was strongly induced in culture by wheat germ oil. Transformation-mediated disruption of FGL1 led to reduced extracellular lipolytic activity in culture and to reduced virulence to both wheat and maize. Voigt, C.A., et al., Plant J. 2005 Can;42(3):364-75. |
| | Deoxynivalenol biosynthesis | Deoxynivalenol is a trichothecene mycotoxin linked to a variety of animal diseases and feed refusals. TRI14 deletion mutants synthesize deoxynivalenol on cracked maize kernel medium and exhibit wild-type colony morphology and growth rate on complex and minimal agar media. However, assays on greenhouse-grown wheat indicate that TRIM mutants cause 50-80% less disease than wild type and do not produce a detectable quantity of deoxynivalenol on plants. Dyer, R.B., et al., J Agric Food Chem. 2005 Nov 16;53(23):9281-7. |
| *Aspergillus spp.* | extracellular hydrolases | Secretion of the endopolygalacturonase P2c is strongly correlated with isolate virulence (against plants) and maceration of cotton boll tissues. Mellon, J.E., et al., Appl Microbiol Biotechnol. 2007 Dec;77(3):497-504. Epub 2007 Oct 16. |
| | toxin biosynthesis | The gliP gene encodes a nonribosomal peptide synthase that catalyzes the first step in gliotoxin biosynthesis. Sugui, J.A., et al., Eukaryot Cell. 2007 Sep;6(9): 1562-9. Epub 2007 Jun 29. The cytolytic protein Asphemolysin can induce effective permeabilization of both chondrocytes and osteoblasts and is considered a possible virulence factor of *Aspergillus fumigatus* during the infection of bone and cartilage. Malicev, E., et al, Med Mycol. 2007 Mar;45(2): 123-30. |

In response to ligand binding, GPCRs can trigger intracellular responses such as changes in levels of Ca²⁺ or cAMP. G protein uncoupling in response to phosphorylation by both second messenger-dependent protein kinases and G protein-coupled receptor kinases (GRKs) leads to GPCR desensitization. GRK-mediated receptor phosphorylation promotes the binding of β-arrestins, which in addition to uncoupling receptors from heterotrimeric G proteins also target many GPCRs for intemalization in clathrin-coated vesicles. B-arrestin proteins play a dual role in regulating GPCR responsiveness by contributing to both receptor desensitization and intemalization.

Following desensitization, GPCRs can be resensitized. GPCR sequestration to endosomes is thought to be the mechanism by which GRK-phosphorylated receptors are dephosphorylated and resensitized. The identification of β-arrestins as GPCR trafficking molecules suggested that β-arrestins can be determinants for GPCR resensitization. However, other cellular components also play pivotal roles in the de- and resensitization (D/R) process, including, for example, GRK, N-ethylmaleimide-sensitive factor (NSF), clathrin adaptor protein (AP-2 protein), protein phosphatases, clathrin, dynamin, and the like. In addition to these molecules, other moieties such as, for example, endosomes, lysosomes, and the like, also influence the D/R process. These various components of the D/R cycle provide opportunities to disrupt or alter GPCR "availability" to extracellular stimuli, and thus attenuate or intensify the effect of those extracellular stimuli upon target organisms. Attenuation, achieved, for example, by inhibition of the resensitization process, or the like, can limit the effects of extracellular stimuli (such as, for example, UV exposure, toxins, or the like) on the GPCR signaling process. Intensifying a signal cascade, achieved, for example, by inhibition of the desensitization process, or the like, can increase the effects of extracellular stimuli (such as, for example, pharmaceuticals, insecticides, or the like) on the GPCR signaling process.
Embodiments in accordance with the present disclosure can include a method to disrupt or alter parasite GPCR D/R by altering or disrupting the various signal cascades triggered through GPCR action. Certain embodiments of the present disclosure can disrupt or alter parasite GPCR D/R in various ways, including, for example, the application of small molecules, including, for example, essential oils, and the like. These small molecules can include, for example, any of the following, or the like:

| **TABLE H** | | | |
|---|---|---|---|
| dihydrotagentone | eugenol | furanodiene | geraniol acetate |
| | | furanoeudesma-1,3-diene | |
| β-elemene | eugenol acetate | furanoeudesma-1,4-diene | lilac flower oil (LFO) |
| gamma-elemene | α-farnesene | furano germacra 1,10(15)-diene-6-one | lime oil |
| Elmol | (Z,E)-α-farnesene | furanosesquiterpene | d-limonene |
| Estragole | E-β-farnesene | Furanosesquiterpene | linalool |
| 2-ethyl-2-hexen-1-ol | fenchone | geraniol | linalyl acetate |
| linalyl anthranilate | methyl citrate | myrtenal | orange sweet oil |
| lindestrene | methyl di-hydrojasmonate | neraldimethyl acetate | 1 -octanol |
| lindenol | menthyl salicylate | nerolidol | E ocimenone |
| linseed oil | mineral oil | nonanone | piperonal |
| methyl-allyl-trisulfide | musk ambrette | gamma-nonalactone | piperonyl |
| menthol | myrcene | oil of pennyroyal | piperonyl acetate |
| methyl cinnamate | | olive oil | piperonyl alcohol |
| piperonyl amine | sabinyl acetate | sesame oil | tagetone |
| prenal | safflower oil | β-sesquphelandrene | tangerine oil |
| pulegone | α-santalene | silicone fluid | α-terpinene |
| quinine | santalol | sodium lauryl sulfate | terpinene 900 |
| rosemary oil | sativen | soybean oil | α-terpineol |
| sabinene | δ-selinene | spathulenol | α-terpinolene |
| gamma-terpineol | cinnamon oil | citronellyl acetate | germacrene B |
| a-terpinyl acetate | citral A citral B | citronellyl formate | grapefruit oil |
| 2-tert-butyl-*p*-quinone | isopropyl citrate | clove oil | α-gurjunene |
| α-thujone | citronellal | α-copaene | α-humulene |
| cinnamaldehyde | citronella oil | cornmint oil | α-ionone |
| cinnamyl alcohol | citronellol | germacrene D | β-ionone |
| isoborneol | lemon oil | peanut oil | phenyl acetaldehyde |
| isofuranogermacrene | lemon grass oil | perillyl alcohol | α-pinene |
| iso-menthone | Z ocimenone | peppermint oil | β-pinene |
| iso-pulegone | 3 -octanone | α-phellandrene | pine oil |
| jasmone | ocimene | β-phellandrene | trans-pinocarveol |
| lecithin | octyl acetate | phenethyl proprionate | |
| thyme oil | vanillin | yomogi alcohol | |
| thymol | trans-verbenol | zingiberene | |
| thymyl methyl ether | cis-verbenol | | |
| gamma-undecalactone | verbenone | | |
| valeric anhydride | white mineral oil | | |

Alternatively, the small molecules can include members of any of the non-essential oil small molecule classes described above.

Embodiments of the present disclosure can include a method for screening a composition for indirect parasite GPCR desensitization inhibitory activity. In certain embodiments of the present disclosure, an indication that the test composition has indirect parasite GPCR desensitization inhibitory activity can be apparent when a test composition has parasite GPCR desensitization inhibitory activity with respect to different GPCRs. In certain embodiments of the present disclosure, an indication that the test composition has indirect parasite GPCR desensitization inhibitory activity can be apparent when parasite GPCR cycling is inhibited without the composition binding the receptor itself. In certain embodiments of the present disclosure, indications of desensitization can include a reduced response to extracellular stimuli, such as, for example, a reduction in GPCR recycling from the plasma membrane to the cell's interior and back to the plasma membrane, or the like. Such a reduced response can result in lowered receptor dephosphorylation and recycling, thus leading to the presence of fewer sensitized receptor molecules on the cell surface. Another indication can be an altered period for the GPRC regulated activation of the Ca²⁺ cascade or the cAMP levels in the organism.

Embodiments of the present disclosure can include a method for screening a composition for indirect parasite GPCR resensitization inhibitory activity. In certain embodiments of the present disclosure, an indication that the test composition has indirect parasite GPCR resensitization inhibitory activity can be apparent when a test composition has parasite GPCR resensitization inhibitory activity with respect to different GPCRs. In certain embodiments of the present disclosure, an indication that the test composition has indirect parasite GPCR resensitization inhibitory activity can be apparent when parasite GPCR cycling is inhibited without the composition binding the receptor itself. In certain embodiments of the present disclosure, indications of resensitization can include a reduced response to extracellular stimuli, such as, for example, a reduction in GPCR recycling from the plasma membrane to the cell's interior and back to the plasma membrane, or the like. Where the receptor does not require segregation to endosomal comparments to undergo dephosphorylation, such a reduction in GPCR cycling can result in the presence of more sensitized receptor molecules on the cell surface. Another indication can be a recovery to normal or static level of Ca²⁺ or cAMP

Embodiments of the present disclosure can include a method for screening a composition for non-specific parasite GPCR desensitization inhibitory activity. The method can include screening a test composition for parasite GPCR desensitization inhibitory activity against two or more different parasite GPCRs. In certain embodiments of the present disclosure, an indication that the test composition has non-receptor-specific parasite GPCR desensitization inhibitory activity can be apparent when a test composition has parasite GPCR desensitization inhibitory activity with respect to each of the two or more different GPCRs. In certain embodiments of the present disclosure, indications of desensitization inhibitory activity can include a reduced response to extracellular stimuli, such as, for example, a reduction in GPCR recycling from the plasma membrane to the cell's interior and back to the plasma membrane, or the like. Another indication can be an altered period for the GPRC regulated activation of the Ca²⁺ cascade or the cAMP levels in the organism.

Embodiments of the present disclosure can include a method for screening a composition for non-specific parasite GPCR resensitization inhibitory activity. The method can include screening a test composition for parasite GPCR resensitization inhibitory activity against two or more different parasite GPCRs. In certain embodiments of the present disclosure, an indication that the test composition has non-receptor-specific parasite GPCR resensitization inhibitory activity can be apparent when a test composition has parasite GPCR resensitization inhibitory activity with respect to each of the two or more different parasite GPCRs. In certain embodiments of the present disclosure, indications of resensitization inhibition can include a reduced response to extracellular stimuli, such as, for example, a reduction in GPCR recycling from the plasma membrane to the cell's interior and back to the plasma membrane, or the like. Another indication can be an altered period for the GPRC regulated activation of the Ca²⁺ cascade or the cAMP levels in the organism.

In an embodiment of the present disclosure, one cell can be used to screen a test composition for indirect parasite GPCR desensitization inhibitory activity. In such an embodiment of the present disclosure, the cell can express two or more parasite GPCRs that are different from each other such that a detection method can be used for determining whether there is an indication that a test composition has parasite GPCR desensitization inhibitory activity with respect to each of the different parasite GPCRs.

In some embodiments of the present disclosure, a multi-well format can be used to screen a test composition for indirect parasite GPCR desensitization inhibitory activity. In some embodiments of the present disclosure, each well of the plate can contain at least one cell that includes a parasite GPCR, and the assay can include adding a compound in an amount known to activate that parasite GPCR, and thus affect intracellular Ca²⁺ levels, to each well. In some embodiments of the present disclosure, at least one test compound can also be added to each well. In some embodiments of the present disclosure, Ca²⁺ level can be tested at various time points after adding the at least one test compound. In certain embodiments of the present disclosure, time points used for testing intracellular Ca²⁺ level can extend beyond the time points where an increase in Ca²⁺ level can be seen without the presence of the at least one test compound. In some embodiments of the present disclosure, methods in accordance with the present disclosure can identify compounds that prolong agonist effect on GPCRs. In some embodiments of the present disclosure, cAMP levels can be evaluated to gauge the effect of the at least one test compound on GPCR response.

In some embodiments of the present disclosure, a multi-well format can be used to screen a test composition for indirect GPCR desensitization inhibitory activity. In some embodiments of the present disclosure, each well of the plate can contain at least one cell that includes a GPCR, and the assay can include adding a compound in an amount less than that required to activate that GPCR, and thus affect intracellular Ca²⁺ levels, to each well. In some embodiments of the present disclosure, at least one test compound can also be added to each well. In some embodiments of the present disclosure, Ca²⁺ level can be tested at various time points after adding the at least one test compound. In certain embodiments of the present disclosure, time points used for testing intracellular Ca²⁺ level can extend beyond the time points where an increase in Ca²⁺ level can not be seen without the presence of the at least one test compound. In certain embodiments of the present disclosure, time points used for testing intracellular Ca²⁺ level can extend beyond the time points where an increase in Ca²⁺ level can be seen with the presence of an GPCR-activating dose of the agonist compound. In some embodiments of the present disclosure, methods in accordance with the present disclosure can identify compounds that enhance agonist effect on GPCRs. In some embodiments of the present disclosure, cAMP levels can be evaluated to gauge the effect of the at least one test compound on GPCR response.

Some of the receptors disclosed herein are cross-referenced to GENBANK® accession numbers. The sequences cross-referenced in the GENBANK® database are expressly incorporated by reference as are equivalent and related sequences present in GENBANK® or other public databases. Also expressly incorporated herein by reference are all annotations present in the GENBANK® database associated with the sequences disclosed herein.

As used herein, the term "receptor binding affinity" refers to an interaction between a composition or component, e.g., compound, and a receptor binding site. The interaction between a composition or component, and the receptor binding site, can be identified as specific or non-specific. In some embodiments of the present disclosure, the specificity of an interaction between a composition or component, and a TyrR binding site, can be determined in the following manner. A wild type fly *(*D*rosophila melanogaster)* and a mutant fly are provided, where the mutant fly lacks a TyrR. The wild type and mutant flies are exposed to a composition or component of interest. If the exposure negatively affects the wild type fly, (e.g., knock down, death), but does not negatively affect the mutant fly, then the treatment with the composition or component of interest can be said to be specific for the TyrR. If the exposure negatively affects the wild type fly and the mutant fly, then the treatment with the composition or component of interest can be said to be non-specific for the TyrR.

A "high receptor binding affinity" can be a specific interaction between a composition or component, and the receptor binding site. In some embodiments of the present disclosure, a high receptor binding affinity is found when the equilibrium dissociation constant (K_{d}) is less than about 100nM, 75nM, 50nM, 25nM, 20nM, 10nM, 5nM, or 2nM. In some embodiments of the present disclosure, a high receptor binding affinity is found when the equilibrium inhibitor dissociation constant (Kᵢ) is less than about is less than about 100µM, 75µM, 50µM, 25µM, 20µM, 10µM, 5µM, or 2µM, when competing with tyramine. In some embodiments of the present disclosure, a high receptor binding affinity is found when the effective concentration at which tyramine binding is inhibited by 50% (EC50) is less than about 500µM, 400µM, 300µM, 100µM, 50µM, 25µM, or 10µM.

A "low receptor binding affinity" can be a non-specific interaction between a composition or component, and the receptor binding site. In some embodiments of the present disclosure, a low receptor binding affinity is found when the equilibrium dissociation constant (K_{d}) is greater than about 100nM, 125nM, 150nM, 175nM, 200nM, 225nM, or 250nM. In some embodiments of the present disclosure, a low receptor binding affinity is found when the equilibrium inhibitor dissociation constant (Kᵢ) is greater than about 100µM, 125µM, 150µM, 175µM, 200µM, 225µM, or 250µM, when competing with tyramine. In some embodiments of the present disclosure, a low receptor binding affinity is found when the effective concentration at which tyramine binding is inhibited by 50% (EC50) is greater than about 500µM, 625µM, 750µM, 875µM, 1000µM, 1125µM, or 1250µM.

Unless defined otherwise, all technical and scientific terms used herein have the same meaning as commonly understood by one of ordinary skill in the art to which the presently-disclosed subject matter belongs. Although any methods and materials similar or equivalent to those described herein can be used in the practice or testing of the presently-disclosed subject matter, representative methods and materials are now described.

Following long-standing patent law convention, the terms "a", "an", and "the" refer to "one or more" when used in this application, including the claims. Thus, for example, reference to "a cell" includes a plurality of such cells, and so forth.

Unless otherwise indicated, all numbers expressing quantities of ingredients, properties such as reaction conditions, and so forth used in the specification and claims are to be understood as being modified in all instances by the term "about." Accordingly, unless indicated to the contrary, the numerical parameters set forth in this specification and claims are approximations that can vary depending upon the desired properties sought to be obtained by the presently-disclosed subject matter. As used herein, the term "about," when referring to a value or to an amount of mass, weight, time, volume, concentration, or percentage is meant to encompass variations of in some embodiments ± 20%, in some embodiments ± 10%, in some embodiments ± 5%, in some embodiments ± 1%, in some embodiments ± 0.5%, and in some embodiments ± 0.1% from the specified amount, as such variations are appropriate to perform the disclosed method.

The presently-disclosed subject matter is further illustrated by the following examples. The following examples can include compilations of data that are representative of data gathered at various times during the course of development and experimentation related to the presently-disclosed subject matter. The following examples include prophetic examples. Examples not falling under the scope of the appended claims do not form part of the invention.

### EXAMPLES

### EXAMPLES 1-3

An example of a parasite that commonly infects humans is *Hymenolepsis nana,* which is an intestinal parasite. *H. nana* is a difficult worm to eliminate from the human intestine. See John Rim, Treatment of Hymenolepis nana infection. Post-Graduate Doctor Journal. Middle East Edition, 5:330-334, 1985. *H. nana* is found worldwide and infection can occur in humans of any age; however, due to the increased likelihood of exposure to human feces, small children have the highest risk of contracting hymenolepiasis, the disease associated with *H. nana* infection.

*H. nana* has a characteristic life cycle of about 7 days. When a host has been infected, the *H. nana* eggs pass into the ileum of the small intestine and hatch into oncospheres, motile larvae of *H. nana,* which penetrate the lamina propria of the villus of the small intestine. Within about 3 to 4 days, the larvae mature into pre-adult cysticercoids, which then enter the gut lumen, attaching to the mucosa of the villus of the small intestine. Many infections are asymptomatic, keeping some infected individuals from seeking medical treatment and being cured. Symptomatic forms of the infection are characterized by irritability, diarrhea, abdominal pain, restless sleep, anal pruritus, nasal pruritus, behavior disturbance, and seizures.

In the present Examples, *H. nana* is selected as an exemplary parasite used to study the efficacy *in vitro* and *in vivo* of compositions disclosed herein for treating parasitic infections. Laboratory-raised Swiss albino mice are used as host animals. Uninfected males and females are used. Pregnant females are isolated from other mice. The newly born litters are maintained to avoid infection thereof. The mother mice are checked twice weekly by direct saline fecal smear and the negative sample is re-examined by zinc sulphate centrifugation floatation and saline sedimentation techniques to exclude those parasitologically infected. *See* Melvin and Brooke, Laboratory procedures for the diagnosis of intestinal parasites. DHEW Publications No. (CDC) 76-828, Public Health Services, 1975.

After weaning the litters, the mice are checked twice weekly and uninfected litters are used for the Examples. Mice are kept under scrupulous hygienic conditions and fed one day milk and the other day wheat. Diet and water are available *ad libitum.*

Eggs of *H. nana,* free of debris, teased from gravid segments are used for infection. *See* Ito, *In vitro* oncospheral agglutination given by immune sera from mice infected and rabbits injected with eggs of *Hymenolepis nana.* Parasito, 71: 465, 1975. Prior to inoculation, the egg shells are removed and every mouse is inoculated with a known number of eggs to maintain the infection cycle. *See* Bernetzen and Voge, In vitro hatching of oncosphere of Hymenolepidid cestodes. J. Parasitol., 5:235, 1965.

Maximum tolerated dose (MTD) of each test agent is determined before starting the *in vivo* study. Worm-free 5 weeks old mice (25-30 grams) are used in the experiment. Each mouse is inoculated with 150 eggs. Then they are subdivided into groups, each group containing 15 mice. Each of these groups is specified for testing the efficacy of one test agent as a potential therapeutic drug against adult worm of *H. nana.* A control group composed of 15 mice is also infected with the same number of eggs but not subjected to the test agents. Infection is monitored and a base egg count from feces is determined for each mouse (experimental and control groups).

### EXAMPLE 1

The following compositions were each tested for anti-parasitic effects against *H. nana* in vivo: Rx1 - Black seed cumin oil; Rx2 - Lilac flower oil; Rx3 - thyme oil (white); Rx4 - carvacrol; Rx5 - geraniol; Rx6 - cineol; and Rx7 - wintergreen oil; Rx8 - Lilac Flower oil-V3; Rx9 - trans-anethole; Rx1O-*p*-cymene; Rx11 - thymol.

Each mouse in the experimental groups was inoculated orally with 400 mg/kg body weight of the specified test compound (Rx) daily for 5 successive days beginning 24 hours following detection of eggs in feces. At the same time, each mouse of the control group was inoculated orally with 400 mg/kg body weight of the suspension material only, i.e. soybean oil, daily for 5 successive days. The egg count of every mouse (experimental and control) was determined daily during the periods of treatment and for further 2 days after the last dose treatment. On the 3rd day after the last dose treatment the cure rate was determined. The criteria for cure was assessed according to: (1) determination of egg-reduction rate; and (2) the absence of the adult worms. The mouse being assessed was killed by decapitation and the small intestine dissected for detecting the adult worms.

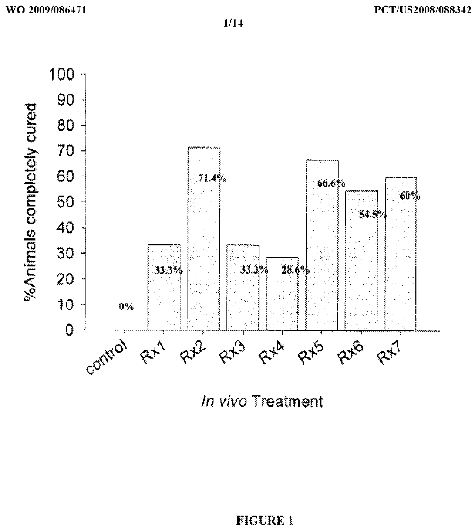

With reference to Table 1 and Figure 1 the cure rate ranged between about 30% to about 70% following treatment with the tested compounds. An infected animal was determined to be cured when it was completely free of worms and eggs at the time of assessment. Various compositions showed a significant cure rate, including: Rx2 (cure rate: 71.4%), Rx5 (cure rate: 66.6%), and Rx7 (cure rate: 60%).

| **Table 1** | | | | | | | | |
|---|---|---|---|---|---|---|---|---|
| **Variable** | **Egg Count (X ± SD)** | | | | | | | |
| | **Control** | **Rx 1** | **Rx2** | **Rx3** | **Rx4** | **Rx5** | **Rx6** | **Rx7** |
| **Pre Treatment (Base Line Data)** | 3± 1 | 2± 1 | 3± 1.2 | 3± 1.2 | 3± 1 | 3± 1 | 3.1 ±1.2 | 3+1 |

| **During Treatment** | | | | | | | | |
|---|---|---|---|---|---|---|---|---|
| **1st** Day | 3± 1 | 2± 1 | 1 ± 1.2 | 12 ± 9.1 | 5±0.6 | 14± 13.9 | 5±9.5 | 1.4±1.1 |
| **2nd** Day | 5±9.5 | 17.7±45 .9 | 0.8±0.9 | 26±25.6 | 2.2±3.4 | 1.4±2.1 | 3.8±14. 3 | 10.6± 17.9 |
| **3rd Day** | 31±14 | 17.5± 19 | 1.8±2.5 | 66±57.9 | 1±1.9 | 4.1±9.6 | 1±1.2 | 22.7± 39.7 |
| 4th Day | 27±17 | 33.4± 55.7 | 3.3±3.2 | 25.4± 15.4 | 0.9±1.2 | 2.6±7.4 | 1.8±1.7 | 9.8±13. 2 |
| **5th** Day | 5.3±4.7 | 33.5± 25.7 | 1.7±1.8 | 5.3±8.9 | 2±2 | 2.3±3.6 | 1.6±1.5 | 1.6±1.7 |

| **Post treatment** | | | | | | | | |
|---|---|---|---|---|---|---|---|---|
| 2 Days after last dose | 125±42.1 | 75.8±21 .3 | 2±3.6 | 17.5±20 .3 | 1.3±1.1 | 0.5±0.9 | 2.5±3.5 | 2.8±5.2 |
| 3 Days after last dose | | | | | | | | |
| **Positiveity rate (%)** | 100 | 66.7 | 28.6 | 66.7 | 71.4 | 33.4 | 45.5 | 40 |
| **Cure rate (%)** | 0 | 33.3 | 71.4 | 33.3 | 28.6 | 66.6 | 54.5 | 60 |

Post treatment dissection of the positive infected mice showed the following: the worms were intact, living, and active; the scolex (head) of the worm was intact keeping its anatomical feature with moving rostellum and contracting suckers; the neck, which is considered the area of segmentation (producing new segments), was intact; and the strobila (the body of the worm) was intact, maintaining its anatomical feature with 3 groups of segments (immature segments or segments with immature reproductive organs, mature segments or segments with mature reproductive organs, and gravid segments or segments with uteri full of mature eggs). Worms were absent or dead in mice treated for 5 consecutive days with Rx2 (71%), Rx5 (67%), and Rx7 (60%).

These experiments can also be conducted to study the treatment efficacy of the presently-disclosed compositions against *Trichuris trichiura in vivo.*

### EXAMPLE 2

The compounds are combined to produce the compositions having anti-parasitic properties disclosed herein. The compositions tested are set forth in Table 2. An "X" in a cell the table indicates that a particular compound is included in a particular test composition. For example, in the column labeled "SI," there is an X in the row setting forth thymol. As such, composition "S1" includes Thymol. Composition S1 further includes carvacrol, trans-anethole, and *p*-cymene.

**TABLE 2**

| | S1 | S2 | S3 | S4 | S5 | S6 | S7 | S8 | S9 | S10 | Sll | S12 | S13 | S14 | S15 | S16 |
|---|---|---|---|---|---|---|---|---|---|---|---|---|---|---|---|---|
| thymol | X | | X | | X | | | | X | | | X | X | | X | X |
| thyme oil (white) | | X | | X | | X | | X | | X | X | | | X | | |
| linalool | | | | | | | | | | | | | | | | X |
| carvacrol | X | X | X | X | X | | | | | | | | | | | |
| trans-anethole | X | X | X | X | | | X | | | | | | | | | X |
| α-pinene | | | | | | | | | | | | | | | | X |
| *p*-cymene | X | X | | | | | | | | | | | | | | X |
| black seed cumin oil | | | | | | | | X | | X | | X | | | | |
| Lilac flower oil | | | | | | | | | X | | X | | X | | | |
| geraniol | | | | | | | | X | X | | | X | X | | | |
| wintergreen oil | | | | | | | | | | | | | | X | X | |
| cineol | | | | | | | | | | | | | | X | X | |
| lime oil | | | | | | | | X | | X | X | | | | | |
| d-limonene | | | | | | | | | X | | | | | | | |

Each mouse in the experimental groups is inoculated orally with 400 mg/kg body weight of the specified test composition daily for 5 successive days. At the same time, each mouse of the control group is inoculated orally with 400 mg/kg body weight daily for 5 successive days of the suspension material only, i.e. soybean oil. The egg count of every mouse (experimental and control) is determined daily during the periods of treatment and for a further 2 days after the last dose treatment. On the 3rd day after the last dose treatment the cure rate is determined. The criteria for cure are assessed according to: (1) determination of egg-reduction rate; and (2) the absence of adult worms. The mouse being assessed is killed by decapitation and the small intestine is dissected for detecting the adult worms.

The cure rate is between about 25% and 80% following treatment with compositions S1 through S16. An infected animal is determined to be cured when it is completely free of worms and eggs at the time of assessment. Worms are absent or dead in mice treated for multiple consecutive days with the compositions having cure rates of about 60% or higher.

These experiments can also be conducted to study the treatment efficacy of the presently-disclosed compositions against *Trichuris trichiura* in vivo.

### EXAMPLE 3

The following compounds and blend compositions were each tested for anti-parasitic effects against *H. nana in vivo:* (1) *p*-cymene; (2) thymol; (3) α-pinene; (4) linalool; (5) soybean oil (control); and (6) blend of 30% *p*-cymene, 35% thymol, 4% α-pinene, 7% linalool, and 24% soybean oil, where percentages are by weight.

Each mouse in the groups was inoculated orally with 100 mg/kg body weight of the specified compound or blend composition daily for 5 successive days. The egg count of each mouse (experimental and control) was determined daily during the periods of treatment and for 2 more days after the last dose treatment. Following the 3rd day of the last dose treatment the cure rate was determined. The criteria for cure was assessed according to: (1) determination of egg-reduction rate; and (2) the absence of the adult worms. The mouse being assessed was killed by decapitation and the small intestine dissected for detecting the adult worms.

With reference to Table 3, the cure rate ranged from 0%, for the soybean oil (control), to 100%, for the blend composition containing 30% *p*-cymene, 35% thymol, 4% α-pinene, 7% linalool, and 24% soybean oil. Cure rate represents the number of infected animals that demonstrate no eggs in their stool and no worms found in their intestine following treatment with the tested compounds.

**TABLE 3**

| **Group** | **Compound** | **Tested dose (mg/kg b.w.)** | **Cure rate (%)** |
|---|---|---|---|
| 1 | *p*-cymene | 100 | 13.3 |
| 2 | thymol | 100 | 33.3 |
| 3 | a-pinene | 100 | 25.0 |
| 4 | linalool | 100 | 23.3 |
| 5 | soybean oil (control) | 100 | 00.0 |
| 6 | blend composition* | 100 | 100 |
| *30% *p*-cymene, 35% thymol, 4% α-pinene, 7% linalool, and 24% soybean oil | | | |

As indicated by the data above, the blend composition has a synergistic effect, as compared to the individual compounds that are components of the blend. A coefficient of synergy can be calculated for the blend, relative to each individual compound, i.e., comparison composition. Such synergy coefficients are set forth in Table 4.

**TABLE 4**

| **Comparison Composition** | **Cure rate (%)** | **Activity Ratio** | **Concentration of Comparison Composition in Blend (%,by wt)** | **Concentration Adjustment Factor** | **Synergy Coefficient** |
|---|---|---|---|---|---|
| *p*-cymene | 13.3 | (1.00)/(0.133)= 7.52 | 30 | (1.00)/(0.300)= 3.33 | 25.1 |
| thymol | 33.3 | (1.00)/(0.333)= 3.00 | 35 | (1.00)/(0.350)= 2.86 | 8.57 |
| a-pinene | 25.0 | (1.00)/(0.250)= 4.00 | 4 | (1.00)/(0.040)=25.0 | 100 |
| linalool | 23.3 | (1.00)/(0.233)= 4.29 | 7 | (1.00)/(0.070)=14.2 9 | 61.3 |
| soybean oil (control) | 00.0 | - | 24 | (1.00)/(0.240)= 4.17 | - |
| blend | 100 | (1.00)/(1.00)= 1.00 | 100 | (1.00)/(1.00)= 1.00 | 1.00 |

For example, the activity ratio for *p*-cymene is 7.52 because the effect of the blend is a cure rate of 100%, while the effect of *p*-cymene alone is 13.3% [(1.00)/(0.133)=7.52]. The concentration adjustment factor for *p*-cymene is 3.33 because the blend contains 30% *p-*cymene, as compared to the 100% *p*-cymene tested alone [(1.00)/(0.300)=3.33]. The synergy coefficient of the blend, relative to *p*-cymene (S_{*p-*cymene}) is therefore 25.1 [((1.00)/(0.133))/(0.300)=25.1]. With further reference to Table 4, the synergy coefficients for the blend are as follows: S_{*p*-cymene} = 25.1; S_{thymol} = 8.57; S _{α-pinene} = 100; and Sₗᵢₙₐₗₒₒₗ = 61.3.

### EXAMPLES 4-6

*D. caninum,* also called the cucumber tapeworm or the double-pore tapeworm, is a cyclophyllid cestode that infects organisms afflicted with fleas, including canids, felids, and pet-owners, especially children. Adult worms are about 18 inches long. Eggs (or "egg clusters" or "egg balls") are passed in the host's feces and ingested by fleas, which are in turn ingested by another mammal after the tapeworm larvae partially develop. Examples of fleas that can spread *D. caninum* include *Ctenocephalides canis* and *Ctenocephalides felis.*

In the present Examples, *D. caninum* is selected as an exemplary parasite used to study the efficacy *in vitro* and *in vivo* of compositions disclosed herein for treating parasitic infections. Laboratory-raised Swiss albino mice are used as host animals. Uninfected males and females are used. Pregnant females are isolated from other mice. The newly born litters are maintained to avoid infection thereof. The mother mice are checked twice weekly by direct saline fecal smear and the negative sample is re-examined by zinc sulphate centrifugation floatation and saline sedimentation techniques to exclude those parasitologically infected.

After weaning the litters, the mice are checked twice weekly and uninfected litters are used for the Examples. Mice are kept under scrupulous hygienic conditions and fed one day milk and the other day wheat. Diet and water are available *ad libitum.*

Eggs of *D. caninum,* free of debris, teased from gravid segments are used for infection. Prior to inoculation, the egg shells are removed and every mouse is inoculated with a known number of eggs to maintain the infection cycle.

Maximum tolerated dose (MTD) of each test agent is determined before starting the *in vivo* study. Worm-free 5 weeks old mice (25-30 grams) are used in the experiment. Each mouse is inoculated with 150 eggs. Then they are subdivided into groups, each group containing 15 mice. Each of these groups is specified for testing the efficacy of one test agent as a potential therapeutic drug against adult worm of *D. caninum.* A control group composed of 15 mice is also infected with the same number of eggs but not subjected to the test agents. Infection is monitored and a base egg count from feces is determined for each mouse (experimental and control groups).

### EXAMPLE 4

The following compositions are each tested for anti-parasitic effects against *D. caninum in vivo:* Rx1 - Black seed cumin oil; Rx2 - Lilac flower oil; Rx3 - thyme oil (white); Rx4 - carvacrol; Rx5 - geraniol; Rx6 - cineol; and Rx7 - wintergreen oil; Rx8 - Lilac Flower oil-V3; Rx9 - trans-anethole; Rx10-_{*p*-}cymene; Rx11 - thymol.

Each mouse in the experimental groups is inoculated orally with 400 mg/kg body weight of the specified test compound (Rx) daily for 5 successive days beginning 24 hours following detection of eggs in feces. At the same time, each mouse of the control group is inoculated orally with 400 mg/kg body weight of the suspension material only, i.e. soybean oil, daily for 5 successive days. The egg count of every mouse (experimental and control) is determined daily during the periods of treatment and for further 2 days after the last dose treatment. On the 3rd day after the last dose treatment the cure rate is determined. The criteria for cure is assessed according to: (1) determination of egg-reduction rate; and (2) the absence of the adult worms. The mouse being assessed is killed by decapitation and the small intestine is dissected for detecting the adult worms.

An infected animal is determined to be cured when it is completely free of worms and eggs at the time of assessment.

Post treatment dissection of the positive infected mice show the following: the worms are intact, living, and active; the scolex (head) of the worm is intact keeping its anatomical feature with moving rostellum and contracting suckers; the neck, which is considered the area of segmentation (producing new segments), is intact; and the strobila (the body of the worm) is intact, maintaining its anatomical feature with 3 groups of segments (immature segments or segments with immature reproductive organs, mature segments or segments with mature reproductive organs, and gravid segments or segments with uteri full of mature eggs).

### EXAMPLE 5

The compounds are combined to produce the compositions having anti-parasitic properties disclosed herein. The compositions tested are set forth in Table 5. An "X" in a cell of the table indicates that a particular compound is included in a particular test composition. For example, in the column labeled "S1," there is an X in the row setting forth thymol. As such, composition "S1" includes Thymol. Composition S1 further includes carvacrol, trans-anethole, and *p*-cymene.

| | S1 | S2 | S3 | S4 | S5 | S6 | S7 | S8 | S9 | S10 | S1 | S12 | S13 | S14 | S15 | S16 |
|---|---|---|---|---|---|---|---|---|---|---|---|---|---|---|---|---|
| thymol | X | | X | | X | | | | X | | | X | X | | X | X |
| thyme oil (white) | | X | | X | | X | | X | | X | X | | | X | | |
| linalool | | | | | | | | | | | | | | | | X |
| carvacrol | X | X | X | X | X | | | | | | | | | | | |
| trans-anethole | X | X | X | X | | | X | | | | | | | | | X |
| α-pinene | | | | | | | | | | | | | | | | X |
| *p*-cymene | X | X | | | | | | | | | | | | | | X |
| black seed cumin oil | | | | | | | | X | | X | | X | | | | |
| Lilac flower oil | | | | | | | | | X | | X | | X | | | |
| geraniol | | | | | | | | X | X | | | X | X | | | |
| wintergreen oil | | | | | | | | | | | | | | X | X | |
| cineol | | | | | | | | | | | | | | X | X | |
| lime oil | | | | | | | | X | | X | X | | | | | |
| d-limonene | | | | | | | | | X | | | | | | | |

Each mouse in the experimental groups is inoculated orally with 400 mg/kg body weight of the specified test composition daily for 5 successive days. At the same time, each mouse of the control group is inoculated orally with 400 mg/kg body weight daily for 5 successive days of the suspension material only, i.e. soybean oil. The egg count of every mouse (experimental and control) is determined daily during the periods of treatment and for a further 2 days after the last dose treatment. On the 3rd day after the last dose treatment the cure rate is determined. The criteria for cure are assessed according to: (1) determination of egg-reduction rate; and (2) the absence of adult worms. The mouse being assessed is killed by decapitation and the small intestine is dissected for detecting the adult worms.

The cure rate is between about 25% and 80% following treatment with compositions S1 through S16. An infected animal is determined to be cured when it is completely free of worms and eggs at the time of assessment. Worms are absent or dead in mice treated for multiple consecutive days with the compositions having cure rates of about 60% or higher.

### EXAMPLE 6

The following compounds and blend compositions are each tested for anti-parasitic effects against *D. caninum in vivo:* (1) *p*-cymene; (2) thymol; (3) α-pinene; (4) linalool; (5) soybean oil (control); and (6) blend of 30% *p*-cymene, 35% thymol, 4% α-pinene, 7% linalool, and 24% soybean oil, where percentages are by weight.

Each mouse in the groups is inoculated orally with 100 mg/kg body weight of the specified compound or blend composition daily for 5 successive days. The egg count of each mouse (experimental and control) is determined daily during the periods of treatment and for 2 more days after the last dose treatment. Following the 3rd day of the last dose treatment the cure rate is determined. The criteria for cure is assessed according to: (1) determination of egg-reduction rate; and (2) the absence of the adult worms. The mouse being assessed is killed by decapitation and the small intestine is dissected for detecting the adult worms.

### EXAMPLE 7

In the present Example, *Schistosoma mansoni* is selected as an exemplary parasite used to study the efficacy *in vivo* of compositions disclosed herein for treating parasitic infections, such as compositions Rx1 - Rx11 and S1-S16 described above. Assessment of the efficacy of the tested compositions against *S. mansoni* infection is with regard to worm load, sex ratio of worms, distribution of worms, fecundity of female worms, and egg deposition in liver and intestine.

Female Swiss Albino mice, 8 weeks in age, from 18-22 gm in weight, which can be obtained from Theodore Bilharz Research Institute, Cairo, are infected percutaneously by *S. mansoni* cercariae (100 cercariae/mouse). Each group consists of 15 mice.

For each test composition, three concentrations are tested. For each concentration nine groups of mice are studied. One group of *S. mansoni-infected* mice receives Praziquantel (PZQ), which is the present standard antischistosomal drug. Three groups of uninfected mice receive the test compound in the same schedule and concentration as the test drug groups. One group of uninfected and untreated mice and one group of *S. mansoni* infected mice that do not receive any treatment are maintained as controls.

Three different concentrations from each of the test compositions are determined after estimation of the LD50. The schedule for drug administration is as follows: (1) four days post-infection (PI); (2) one week PI; and seven weeks PI. Praziquantel (Distocide), 600 mg/Kg body weight, is administered seven weeks PI. All drugs are administered orally using a stomach tube.

For the parasitological studies, fecal egg counts are done for all infected groups twice weekly starting from the 5^{th} week PI.

Mice are sacrificed 9 weeks PI. Perfusion of the portal system is done for the recovery of the schistosome worms. The total number, sex, maturation and distribution of the worms are determined. Four portions, two from the jejunum and two from the ileum, are taken from each mouse, washed with PBS, opened and compressed between two slides and examined microscopically for detection of the stage of maturation. 0.3 gram of the liver and of the intestine are digested in 4% potassium hydroxide overnight, and *S. mansoni* ova counted.

### EXAMPLE 8

In the present Example, *Opisthorchis sinensis* is selected as an exemplary parasite used to study the efficacy *in vivo* of compositions disclosed herein for treating parasitic infections, such as compositions Rx1 - Rx11 and S1-S16 described above. Assessment of the efficacy of the tested compositions against *O*. *sinensis* infection is with regard to worm load, sex ratio of worms, distribution of worms, fecundity of female worms, and egg deposition in liver and intestine.

Female Swiss Albino mice, 8 weeks in age, from 18-22 gm in weight, which can be obtained from Theodore Bilharz Research Institute, Cairo, are infected percutaneously by *S. mansoni* cercariae (100 cercariae/mouse). Each group consists of 15 mice.

For each test composition, three concentrations are tested. For each concentration nine groups of mice are studied. One group of *O*. *sinensis*-infected mice receives the present standard treatment drug. Three groups of uninfected mice receive the test compound in the same schedule and concentration as the test drug groups. One group of uninfected and untreated mice and one group of *O*. *sinensis* infected mice that do not receive any treatment are maintained as controls.

Three different concentrations from each of the test compositions are determined after estimation of the LD50. The schedule for drug administration is as follows: (1) four days post-infection (PI); (2) one week PI; and seven weeks PI. Praziquantel (Distocide), 600 mg/Kg body weight, is administered seven weeks PI. All drugs are administered orally using a stomach tube.

For the parasitological studies, fecal egg counts are done for all infected groups twice weekly starting from the 5^{th} week PI.

Mice are sacrificed 9 weeks PI. Perfusion of the portal system is done for the recovery of the worms. The total number, sex, maturation and distribution of the worms are determined. Four portions, two from the jejunum and two from the ileum, are taken from each mouse, washed with PBS, opened and compressed between two slides and examined microscopically for detection of the stage of maturation. 0.3 gram of the liver and of the intestine are digested in 4% potassium hydroxide overnight, and *O*. *sinensis* ova counted.

### EXAMPLE 9

Three groups of mice are treated with each test compound or composition blend of compounds. For Groups 1 and 2, treatment starts 4 and 7 days after infection, respectively. For Group 3, treatment starts 7 weeks after infection. For the control group, the mice are injected 7 weeks after infection with Praziquantel at 600 mg/kg. Efficacy of test agents is determined based on: worm load; sex ratio; distribution of worms; fecundity of female worms; and egg deposition in liver and intestine.

### EXAMPLE 10

Adult male and female *S. mansoni* were collected from infected mice and transferred into 100 ml saline treated with test compositions Rx1-Rx10 (as disclosed in Example 1) or Praziquantel at varying concentrations and incubated at 37°C in 5% CO₂. In many cases adult male and females were collected as couples. Viability of worms was examined under a binuclear microscope. Controls were treated in parallel. The experiment was terminated either when all worms are dead in the treated samples or when the first death among controls is found.

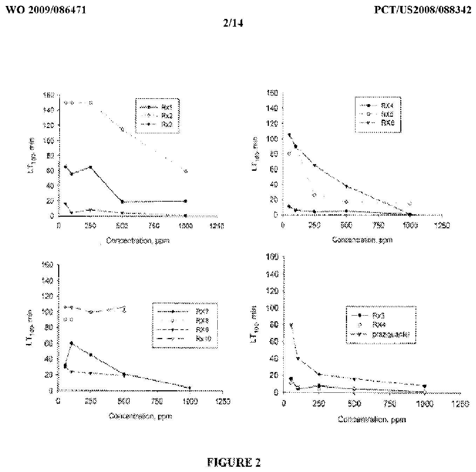

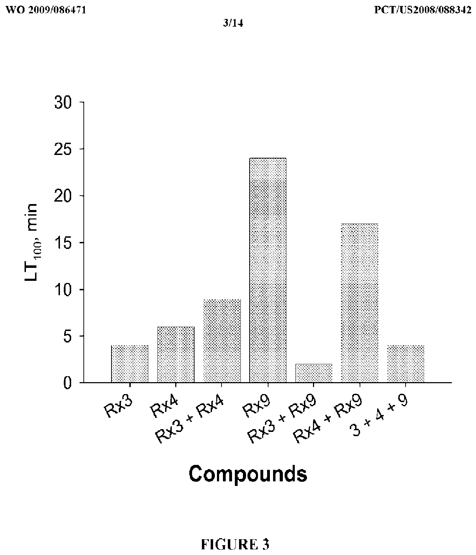

Each of the compounds were tested individually at differing concentrations and the data from these experiments are presented in Figure 2. Next, each compound was tested by itself at 100 ppm final concentration and then compositions were combined at 1:1 ratios when two compounds were combined or 1:1:1 ratios when three compounds were combined and each combined composition tested at 100 ppm final concentration. Data from these experiments are presented in Figure 3. In the Figure, Rx 1 through Rx9 have the meaning set forth in Example 1.

### EXAMPLE 11

The present Example provides an *in vitro* study testing treatment of *Histomonas meleagridis,* a protozoan parasite causing blackhead disease of chickens and turkeys, using the presently-disclosed compounds and blend compositions of the compounds.

*H. meleagridis* is cultured *in vitro* and prepared for use in screw-capped glass vials containing 1 ml of Dwyer's medium and inoculated with 20,000 cells. The test compounds and/or compositions are diluted to appropriate concentrations, so that the desired dose is administered to the tubes in 0.1 ml. Each treatment is replicated in duplicate cultures. The cultures are incubated for 2 days.

The number of *H. meleagridis* cells/ml can be counted using a standard hemocytometer (Neubauer) and the actual number of cells/ml is reported.

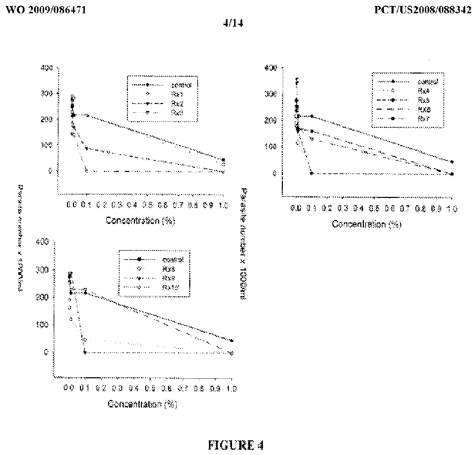

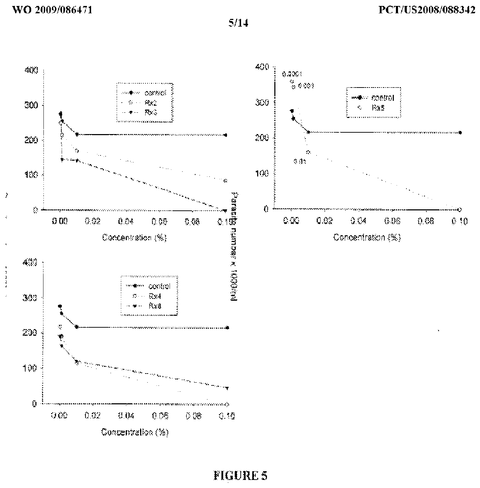

Each compound and/or composition is tested at 1, 0.1, 0.01, 0.001 and 0.0001%. Controls are included as untreated and with solvent (ethanol). Data from the experiments are presented in Figures 4 and 5.

### EXAMPLE 12

The present Example provides an *in vitro* study testing treatment of *Cryptosporidium parvum* using the presently-disclosed compounds and blend compositions of the compounds, such as compositions Rx1 - Rx11 and S1-S16 described above. Cryptosporidiosis is a parasitic infection of human and animal importance. The organism can affect the epithelial cells of the human gastrointestinal, bile duct and respiratory tracts. Over 45 different species of animals including poultry, fish, reptiles, small mammals (rodents, dogs, and cats) and large mammals (including cattle and sheep) can become infected with C. *parvum.* The reservoir for this organism includes people, cattle, deer and many other species of animal.

Transmission is fecal-oral, which includes contaminated food and water, animal-to-person and person-to-person. The parasite infects intestinal epithelial cells and multiplies. Oocysts are shed in the feces and can survive under very adverse environmental conditions. The oocysts are very resistant to disinfectants. People can re-infect themselves one or more times.

*C. parvum* is cultured *in vitro* and prepared for use in screw-capped glass vials containing 1 ml of Dwyer's medium and inoculated with 20,000 cells. The test compounds and/or compositions are diluted to appropriate concentrations, so that the desired dose is administered to the tubes in 0.1 ml. Each treatment is replicated in duplicate cultures. The cultures are incubated for 2 days.

The number of C. *parvum* cells/ml can be counted using a standard hemocytometer (Neubauer) and the actual number of cells/ml is reported. Each compound and/or composition is tested at 1, 0.1, 0.01, 0.001 and 0.0001%. Controls are included as untreated and with solvent.

### EXAMPLE 13

Trichinellosis (previously referred to as 'trichinosis') is a zoonosis caused by parasitic nematodes of the genus Trichinella. The most common species is *Trichinella spiralis,* but other species such as *Trichinella trichuris* are also infective. It is a serious food born parasitic zoonosis with worldwide distribution whenever pork including domestic and wild pig is an important component of the diet (Frierson, 1989). The infection has a worldwide occurrence specifically, it has been estimated that 10 million people worldwide are infected (Jean Dupouy, 2000) and in the past 10 years an increase in the occurrence of infection has been reported among domestic pigs and wildlife, with a consequent increase among humans (Murrell & Pozio, 2000).

Transmission occurs when pork containing infective, encysted larvae is eaten. Also, inadvertent or deliberate mixing of pork with other meat products as grinding beef and pork in the same grinder or mixing pork in the same grinder or mixing pork with beef in sausages can result in infection (Kejenie and Bero, 1992). The larvae burrow beneath the mucosa of the small intestine where they mature into adult worms. Within 7 days, female worms release another generation of larvae which migrate to striated skeletal muscle and become encysted. Larvae often reach to the myocardium but do not become encysted there. The larvae produce intense allergic and inflammatory reactions which are expressed clinically as fever, muscle pains, periorbital oedema and eosinophilia. The initial intestinal infection often induces nausea, diarrhea and abdominal cramps, but these are rarely serious. However, subsequent complications such as myocarditis, pneumonia and meningoencephalitis can be fatal.

Death from trichinellosis is rare. For example, of the > 6500 infections reported in the European Union in the past 25 years, only five deaths have been recorded, all of which were due to thromboembolic disease and recorded in people aged > 65 years as reported by Ancelle et al. 1988. Twenty fatalities out of 10,030 cases were reported in a worldwide survey performed by the International Commission on Trichinellosis (January 1995 - June 1997) as reported by Jean Dupouy, 2000.

Each case of confirmed or even suspected infection must be treated in order to prevent the continued production of larvae. The medical treatment includes anthelmintics (mebendazole or albendazole) and glucocorticosteroids. Mebendazole is usually administered at a daily dose of 5 mg/kg but higher doses (up to 20 - 25 mg/ kg/day) are recommended in some countries. Albendazole is used at 800 mg/ day (15 mg/kg/day) administered in two doses. These drugs are taken for 10 - 15 days. The use of mebendazole or albendazole is contraindicated during pregnancy and not recommended in children aged < 2 years. The most commonly used steroid is prednisolone, which can alleviate the general symptoms of the disease. It is administered at a dose of 30 - 60 mg/day for 10 - 15 days (Jean Dupouy et.,al, 2002).

*Trichuris trichiura* is a common nematode infection worldwide. The highest prevalence occurs in tropic climates with poor sanitation practices, as it has fecal/oral transmission. *T. trichiura* does not migrate through the tissues, and it does not cause eosinophilia. It can survive 6 yrs. in host (average 3 years), living in the large intestine with its head imbedded in intestinal mucosa, but there is virtually no cellular response. Diagnosis of *T. trichiura* is made through finding the eggs in feces. Infection with *T. trichiura* is frequently asymptomatic. However, in heavy infection in undernourished children, *T. trichiura* can cause rectal prolapse following chronic bloody diarrhea.

Compounds and blended compositions of the compounds, such as compositions Rx1 - Rx11 and S1-S16 described above, as disclosed herein, are tested for *in vitro* anti-parasitic activity using the protocols following. Ten groups (8 different concentrations of compositions and 2 controls) can be tested. Tests are performed in sterile six well plates with 1-4 worms per well. Each well contains 3 mL RPMI 1640 containing a 10X antibiotic/antimycotic (penicillin/streptomycin/amphotercin B) solution to prevent overgrowth of contaminating organisms. Worm motility is observed at all initial time points, as well as 24 hour post treatment, i.e. following wash and placement in media without test compounds.

As indicated, eight concentrations and two controls are tested. The controls indicated for these tests will be a surfactant control and a media control. The protocol utilizes 5 - 10X of the final concentrations of test compounds to be added to the media at the time of testing.

Once the test is initiated, motility is checked at 15, 30, 60, 120, 240, and 360 minutes post-treatment. Following the last time point, the worms are removed from the treated media, rinsed and placed into untreated media. A last motility check is performed at 24 hours post-treatment. Worms not observed to be motile are prodded with a sterile (autoclaved) wooden applicator stick to confirm lack of responsiveness. An effective concentration of the compounds and blended compositions of the compounds is determined.

### EXAMPLE 14

The human pinworm *Enterobius vermicularis* is a ubiquitous parasite of man, it being estimated that over 200 million people are infected annually. It is more common in the temperate regions of Western Europe and North America and is particularly in common in children. Samples of Caucasian children in the U.S.A. and Canada have shown incidences of infection of between 30% to 80%, with similar levels in Europe, and although these regions are the parasites strongholds, it can be found throughout the world. For example in parts of South America, the incidence in children can be as high as 60%. Interestingly non-Caucasians appear to be relatively resistant to infection with this nematode. As a species, *E*. *vermicularis* is entirely restricted to man, other animals harboring related but distinct species that are non-infective to humans, although their fur can be contaminated by eggs from the human species.

The adult parasites live predominantly in the caecum. The male and females mate, and the uteri of the females become filled with eggs. Eventually the female die, their bodies disintegrating to release any remaining eggs. These eggs, which are clear and measure -55 by 30 µm, then mature to the infectious stage (containing an LI larvae) over 4 to 6. Infection of the host typically follows ingestion of these eggs, the eggs hatching in the duodenum.

Compounds and blended compositions of the compounds, such as compositions Rx1 - Rx11 and S1-S16 described above, as disclosed herein, can be tested for *in vitro* anti-parasitic activity against *E. vermicularis* using the protocols following. Ten groups (8 different concentrations of compositions and 2 controls) can be tested. Tests are performed in sterile six well plates with 1-4 worms per well. Each well contains 3 mL RPMI 1640 containing a 10X antibiotic/antimycotic (penicillin/streptomycin/amphotercin B) solution to prevent overgrowth of contaminating organisms. Worm motility is observed at all initial time points, as well as 24 hour post treatment, i.e. following wash and placement in media without test compounds.

As indicated, eight concentrations and two controls are tested. The controls indicated for these tests are a surfactant control and a media control. The protocol utilizes 5 - 10X of the final concentrations of test compounds to be added to the media at the time of testing.

Once the test is initiated, motility is checked at 15, 30, 60, 120, 240, and 360 minutes post-treatment. Following the last time point, the worms are removed from the treated media, rinsed and placed into untreated media. A last motility check is performed at 24 hours post-treatment. Worms not observed to be motile are prodded with a sterile (autoclaved) wooden applicator stick to confirm lack of responsiveness. An effective concentration of the compounds and blended compositions of the compounds is determined.

### EXAMPLE 15

Compounds and blended compositions of the compounds, such as compositions Rx1 - Rx11 and S1-S16 described above, as disclosed herein, are tested for *in vitro* anti-parasitic activity using the protocols following. Ten groups (8 different concentrations of compositions and 2 controls) can be tested. Tests are performed in sterile 150 cm³ flasks with 1-2 worms per flask. Each flask contains 200 mL RPMI 1640 containing a 10X antibiotic/antimycotic (penicillin/streptomycin/amphotercin B) solution to prevent overgrowth of contaminating organisms. Worm motility is observed at all initial time points, as well as 24 hour post treatment, i.e. following wash and placement in media without test compounds.

As indicated, eight concentrations and two controls are tested. The controls indicated for these tests will be a surfactant control and a media control. The protocol utilizes 5 - 10X of the final concentrations of test compounds to be added to the media at the time of testing.

Once the test is initiated, motility is checked at 15, 30, 60, 120, 240, and 360 minutes post-treatment. Following the last time point, the worms are removed from the treated media, rinsed and placed into untreated media. A last motility check is performed at 24 hours post-treatment. Worms not observed to be motile are prodded with a sterile (autoclaved) wooden applicator stick to confirm lack of responsiveness. An effective concentration of the compounds and blended compositions of the compounds is determined.

### EXAMPLE 16

Testing was conducted to determine the dose-response of test agents against larvae of *Trichinella spiralis* under *in vitro* conditions.

Two test agents were used in this Example, designated Agents A and B. Agent A comprised 7% linalool coeur, 35% thymol, 4% α-pinene, 30% *p*-cymene, and 24% soybean oil. Agent B comprised Agent A with the addition of 1.2% of a surfactant, the commercially available Sugar Ester OWA-1570. The stock solution (A or B) was diluted by normal sterile saline solution into five concentrates: 100 ppm, 50 ppm, 25ppm, 10 ppm and 1 ppm. Each concentrate was agitated by vortex for 15 minutes before use.

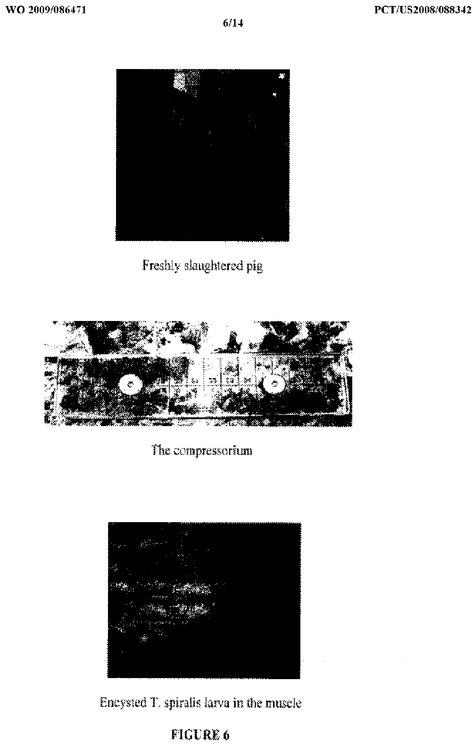

Infective larvae were obtained from muscle samples mainly from the diaphragm taken from freshly slaughtered pigs. These were compressed by the compressorium (consisting of two glass slides of 6 mm thickness, each one measuring 20 x 5 cm with one hole on each side, each hole being provided with a screwed nail, and the upper surface of the lower slide being marked with a diamond pencil into 28 divisions having serial numbers to enable the examiner to examine 28 specimens at one setting) into a thin layer suitable for microscopic examination and examined for Trichinellosis by Trichinoscope in the slaughter house. The infected carcasses were obligatorily condemned. Infected muscle samples were taken, kept in ice box and transferred to the laboratory. The muscle samples were cut into small pieces (oat grains) parallel to the muscle fibers. Randomly selected muscle specimens were taken placed between two slides, pressed until obtaining a thin layer to be examined under the low power objective of the microscope (X10) to detect the encysted larvae of *Trichinella spiralis* in order to reconfirm the infection before doing the digestion technique (see FIG. 6). The infective free larvae were obtained by digestion technique according to Schad et al., 1987. This method consists of 1 gram pepsin and 1 ml concentrated HCL in 100 ml distilled water for 10 gram of muscle. The muscle was digested at 37C for 1 hour under continuous agitation by using a magnetic stirrer. The content was filtered through two layers of gauze on 200 mesh/cm² sieve for centrifugation. The supernatant was poured off and the sediment was washed with normal saline 3 times by repeated sedimentation to obtain clear larvae.

In this Example, the infective larvae were obtained by this method from freshly slaughtered infected pigs to test the efficacy of the tested drug agents upon the larvae, so as to simulate the natural mode of human infection. However, larvae obtained from infected laboratory animals in a lab can also be employed.

Five free active infective larvae were placed in a Petri dish (50x9mm) and the tested agents (A or B) with different concentrations: 100 ppm, 50 ppm, 25 ppm, 10 ppm and 1 ppm were added to the infective larvae in sufficient quantity (to cover the larvae) to be examined carefully for their activities and vitality (viability testing) according to Ismail, 1979. This method reported that when adding the test material to the living larvae and their movement ceased, the larvae were stimulated with a needle to observe any further movement. When no movement occurred the larvae were transferred to another Petri dish containing hot water (38-40C). The occurrence of a sudden movement indicates that the tested drug agent has relaxant effects on the larvae. When no signs of recovery occurred this indicates a sign for killing effect of the tested drug agent. The time duration, from adding the tested agent to the larvae till there was no movement of the all larvae in the Petri dish (5 larvae) was calculated.

The experiment for each concentration was repeated for 5 replicates, each one with 5 larvae (i.e. a total of 25 larvae for each concentration).

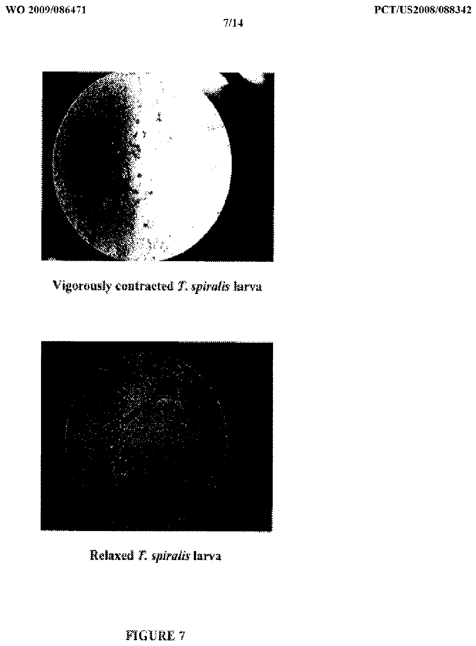

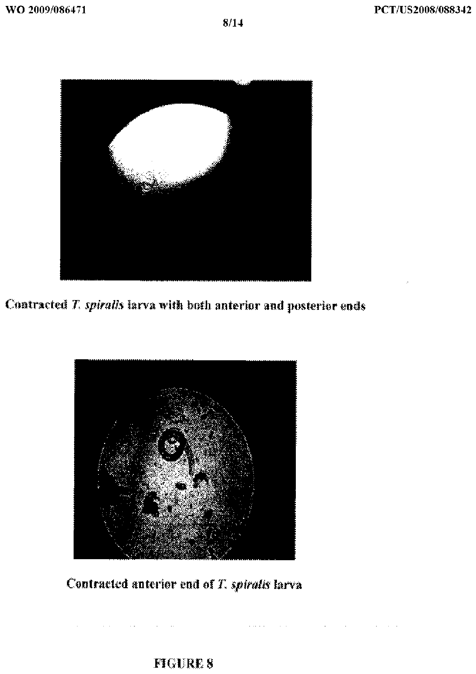

The following was observed for both groups of tested agents (A or B) with their different concentrations (100 ppm, 50 ppm, 25 ppm, 10 ppm & 1 ppm): once the tested agent became in contact with the larvae, the larvae showed vigorous contractions of their whole bodies, mainly the anterior and posterior ends, followed by relaxation as shown in the photos of FIGS. 7 and 8. These strong contractions slowly diminished until no movement was observed. When the tested larvae were stimulated with a needle, they showed no response i.e. no movement. In addition, when the larvae were transferred to another Petri dish containing hot water, they also showed no movement. This observation indicated that there was no sign of recovery for either tested agent (A or B) with their different concentrations employed.

This observation demonstrated the killing effect of both compositions (A&B), regardless of concentration, on Trichinella spiralis larvae under *in vitro* conditions according to Ismail (1979), but they differ from each other according to the mean time to death of the tested larvae.

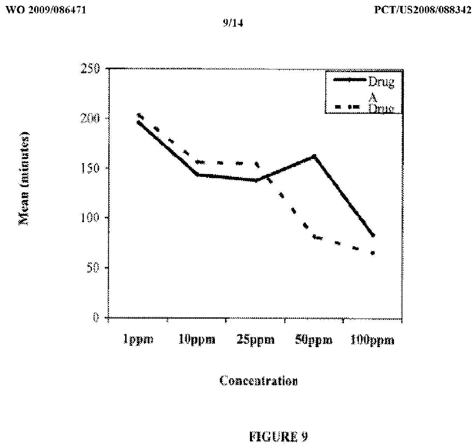

The following table, and the graph shown in FIG. 9, show the mean time to death for *T. spiralis* larvae by the tested agents (A or B) and their different concentrations.

**Table 6: Mean time to death by tested agents and concentrations**

| Test Agent | Concentration | | Time to death (Minutes) | | | | Scheffe multiple comparison for cone. Significantly different from |
|---|---|---|---|---|---|---|---|
| | (ppm) | | Mean | SD | Min | Max | |
| A | | 1 | 195.74 | 33.49 | 140 | 225 | 10,25,50,100 |
| | | 10 | 143.90 | 12.10 | 130 | 161 | 25,50,100 |
| | | 25 | 138.11 | 14.27 | 121 | 158 | 50,100 |
| | | 50 | 162.52 | 10.58 | 150 | 174 | 100 |
| | | 100 | 83.54 | 17.04 | 62 | 103 | |
| Total | | | 144.76 | 41.35 | 62 | 225 | |
| B | | 1 | 203.49 | 6.08 | 198 | 213 | 10,25,50,100 |
| | | 10 | 156.43 | 14.50 | 136 | 174 | 50,100 |
| | | 25 | 154.71 | 12.84 | 143 | 171 | 50,100 |
| | | 50 | 81.79 | 12.78 | 70 | 104 | |
| | | 100 | 65.68 | 14.30 | 55 | 91 | |
| Total | | | 132.42 | 53.55 | 55 | 213 | |
| F(Drug | | | 7.18* | | | | |
| (Cone.) | | | 78.01** | | | | |
| F(Drug*Conc) | | | 15.47** | | | | |

| | | | | | | | |
|---|---|---|---|---|---|---|---|
| *P<0.05 **P<0.01 | | | | | | | |

The table shows that the overall mean time to death with test agent A is significantly longer (144.76 + 41.35 minutes) than with test agent B (132.42 + 53.55 minutes). As regards concentration, the mean time to death significantly decreased with increasing concentration, with no significant difference between test agents in each concentration except for concentration 50 ppm which showed a mean of 162.52 + 10.58 minutes with test agent A compared to 81.79 + 12.78 minutes with test agent B.

Next, the infectivity of the treated larvae were tested with test agent B at a concentration of 50 ppm. This agent and concentration were chosen because the test agent B at a concentration 50 ppm exhibited a significant decrease in the mean time to death for *T. spiralis* larvae of about 50% (81.79 + 12.78 minutes) in comparison with test agent A at the same concentration (162.52 + 10.58 minutes).

Fifteen laboratory raised Swiss albino mice aged 6 weeks were used to execute the study. They were kept under scrupulous hygienic conditions and feed one day milk and other day wheat. Diet and water were available *ad libitum.* All animals were acclimatized to these conditions for 1 week prior to the experiment

Proven infected muscle samples (mainly from the diaphragm) containing encysted larvae of *Trichinella spiralis* were taken from freshly slaughtered pigs at slaughter house in Alexandria and immediately transferred to the laboratory. The infective free larvae were obtained by digestion technique according to Schad et al., 1987. The larvae were treated with the test agent B at a concentration of 50 ppm till no signs of recovery were obtained.

A dose of 150 treated larvae were inoculated orally per mouse (15 mice) at day 0 (Infection day). At day 7 post infection (adult stage), 5 mice were decapitated, their small intestines were washed by normal saline, opened and scraped. The content was filtered through 2 layers of gauze and centrifuged. The supernatant was poured off and the sediment was examined for the adult worms of T. spiralis.

At day 45 post infection (encysted larvae stage in muscle), the remaining 10 mice were decapitated, muscle samples were taken from the diaphragm and other skeletal muscle and examined under the low power objective of the microscope (X10) to detect the encysted larvae of T. spiralis in order to reconfirm the infection.

At day 7 post infection, no adult worms were detected. In addition no encysted larvae of T. spiralis were detected in the muscle on day 45 post infection. This demonstrates the killing effect of the test agent B at a concentration of 50 ppm. The test agent B at a concentration of 50 ppm thus had a lethal effect on *T spiralis* larvae, making them non viable and non infective.

In summary, both agents A & B exhibited a killing effect on *Trichinella spiralis* larvae under *in vitro* conditions regardless of concentration, but they differed from each other according to the mean time to death of the tested larvae. The overall mean time to death with test agent A was significantly longer than with test agent B. As regards concentration, the mean time to death decreased significantly with increasing concentration, with no significant difference in the rate of decrease between test agents at each concentration, except that test agent B at 50 ppm decreased the mean time to death for *T. spiralis* larvae to about 50% of that of test agent A of the same concentration. The test agent B at 50 ppm was thus proved to have a lethal effect on *T. spiralis* larvae, makes them non viable and non infective under *in vivo* testing.

### EXAMPLE 17

It is estimated that more than 1.4 billion people are infected with Ascaris lumbricoides, a nematode of the secementea class. This infected population represents 25 percent of the world population (Seltzer, 1999). Although ascariasis occurs at all ages, it is most common in children 2 to 10 years old, and prevalence decreases over the age of 15 years. Infections tend to cluster in families, and worm burden correlates with the number of people living in a home (Haswell et.al., 1989). The prevalence is also greatest in areas where suboptimal sanitation practices lead to increased contamination of soil and water. The majority of people with ascariasis live in Asia (73 percent), Africa (12 percent) and South America (8 percent), where some populations have infection rates as high as 95 percent (Sarinas and Chitkara, 1997). In the United States the prevalence of infection decreased dramatically after the introduction of modern sanitation and waste treatment in the early 1900s as reported by Jones, 1983.

Children are particularly vulnerable since they are at risk of ingesting *Ascaris* eggs while playing in soil contaminated with human faeces. Dust and contaminated fruits and vegetables pose a hazard to all members of the community. Once ingested, the eggs hatch in the small intestine and motile larvae penetrate the mucosal blood vessels. They are carried first to the liver and then to lungs where they ascend the bronchial tree before being swallowed. Eventually they re-enter the small intestine where they mature, over the period of two months into adult worms. Adult worm can live from 1-2 years.

This larval migration sometimes induces transient hypersensitivity and inflammatory reactions resulting in pneumonitis, bronchial asthma and urticaria. Subsequently, colonization of the gastrointestinal tract by adult worms, which survive for about one year, can cause anorexia, abdominal pain and discomfort and other gastrointestinal symptoms. From time to time all or part of the worms can be vomited or passed in the stools. Obstruction of the small intestine by worms or less frequently their migration, often subsequent to inadequate treatment into biliary tract, the appendix, the pancreatic ducts or even the upper respiratory tract can create a life-threatening emergency requiring surgical interference.

*Ascaris suum* (Goeze, 1782) or pig *Ascaris* is morphologically identical to *A. lumbricoides* with slight differences. The copulatory spicules are thinner and sharper on the tip in *A. suum* than in A. *lumbricoides.* The prepatent period in *A.suum* is shorter than in A. *lumbricoides* (Galvin, 1968).

*Ascaris suum* is commonly called the large roundworm of pigs and its predilection site is the small intestine. It is the largest and most common nematode of pigs on a worldwide basis. Boes et al., 1998 reported that the prevalence and intensity as well as the distribution observed for *A. suum* infection in pigs were comparable to those reported for *A. lumbricoides* in endemic areas, and there was an evidence for predisposition to *A. suum* in pigs, with an estimated correlation coefficient similar to that found in humans. They concluded that A. *suum* infections in pigs are a suitable model to study the population dynamics of *A. lumbricoides* in human populations.

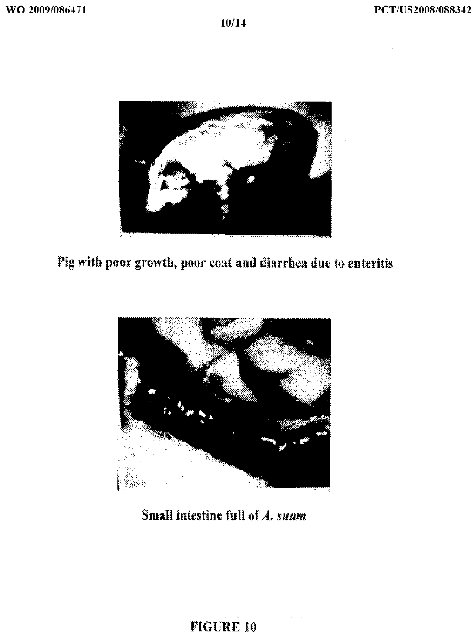

The life cycle of the parasite, *A. suum* is similar to the one in *A*. *lumbricoides.* The adult worms are large worms (males 15-25 cm; females 20-40 cm) that occur in the small intestine. They feed upon the intestinal contents, competing with the host for food. Eggs are environmentally resistant. Female worms are very prolific producing 0.5 to 1 million eggs per day and these will survive outside the pig for many years (up to 20 years). They are resistant to drying and freezing but sunlight kills them in a few weeks. Eggs become infective after 18 to 22 days. When ingested eggs are hatched in the stomach and upper intestine, and larvae migrate to the liver and then to the lungs. After about 10 days, larvae migrate to esophagus and will be swallowed and return to the intestine, where after two molts develop to the adult worms between 15 and 18 cm long. Infection with *A*. *suum* affects pigs, principally the young. Signs include poor growth, poor coat and diarrhea due to enteritis (see photo in FIG. 10). Migration by the larvae results in the development of hepatitis and pneumonia. Other less common sequelae include biliary duct obstruction.

The infection does not restricted to pigs only but also can infect cattle as reported by Borgsteede et al., 1992. The infection causing a sudden decrease in milk yield, increased respiratory rate and occasional coughing were observed in dairy cows on farms where pigs were also kept on these farms, and pastures grazed by the cattle had been fertilized with pig slurry. Laboratory investigations of some of the cattle showed eosinophilia and high ELISA titres of antibodies against Ascaris suum. The clinical symptoms disappeared after the animals had been treated.

Human infection occurs also as the result of exposure to the pig farms, or the use of pig manure in the vegetable gardens. An outbreak of infection with swine *Ascaris lumbricoides suum* with marked eosinophilia was reported from southern part of Kyushu District, Japan (Maruyama et al, 1997).

The clinical symptoms of infection with *A suum* in man are similar to A. *lumbricoides* and high burden will cause sever diseases. As described by Phills et al (1972), four male students in Montreal, Canada who unknowingly swallowed eggs of *A. suum* became hospitalized with severe pneumonitis, high eosinophilia and asthma. The infection can also produce failure to thrive, stunting, pot belly and diarrhea (Merle and Nicole (2000).

Chemotherapy is the cornerstone of the strategy of control of morbidity and reduction of transmission. Individual human infections are eradicated by a single dose of pyrantel or levamisole. Piperazine is also effective but it less well tolerated. The most commonly used drugs are broad-spectrum anthelminthics as benzimidazole, mebendazole, albendazole and flubendazole are each effective.

In this Example, the dose-response of two test agents against adult worms of Ascaris lumbricoides suum was determined under *in vitro* conditions.

Two test agents were used in this Example, designated Agents A and B. Agent A comprised 7% linalool coeur, 35% thymol, 4% α-pinene, 30%*p-*cymene, and 24% soybean oil. Agent B comprised Agent A with the addition of 1.2% of a surfactant, the commercially available Sugar Ester OWA-1570. The stock solution (A or B) was diluted by sterile normal saline solution into five concentrates: 100 ppm, 50 ppm, 25 ppm, 10 ppm and 1 ppm. Each concentrate was agitated by vortex for 15 minutes before use.

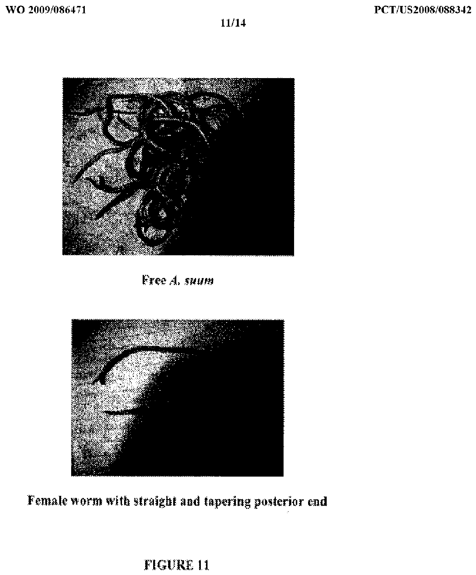

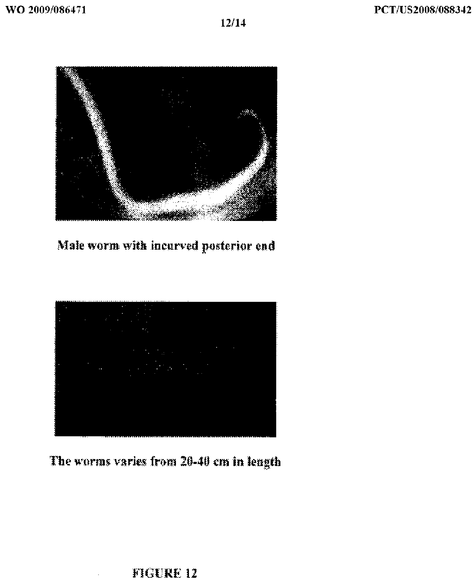

The adult worms of *A. suum* were obtained from intestines of slaughtered pigs condemned in the slaughter houses, as unfit for human consumption or use. The pig's intestines were taken and opened; their content was examined for the presence of adult worms of *A. suum* (see the photo in FIG. 10). The adult worms were washed twice with normal saline and kept in container with sufficient quantity of normal saline and immediately transferred to the laboratory (see the photos in FIGS. 11 and 12).

Five living adult worms of both sexes of *A. suum* were placed in a suitable dish and the tested agents (A or B) with different concentrations: 1 ppm, 10 ppm, 25 ppm, 50 ppm and 100 ppm were added to the living adult worms in sufficient quantity (to cover the adult worms) to be examined carefully for their activities and vitality (viability testing) according to Ismail, 1979. This method reported that when adding the test material to the living worms and their movement ceased, the worms were stimulated with needle to observe any further movement. When no movement occurred the worms were transferred to another dish containing hot water (38-400C). The occurrence of a sudden movement indicates that the tested agent has relaxant effects on the worms. When no signs of recovery occurred this indicates a sign for killing effect of the tested agent. The time duration, from adding the tested agent to the worms till there was no movement of the all worms in the dish (5 worms) was calculated.

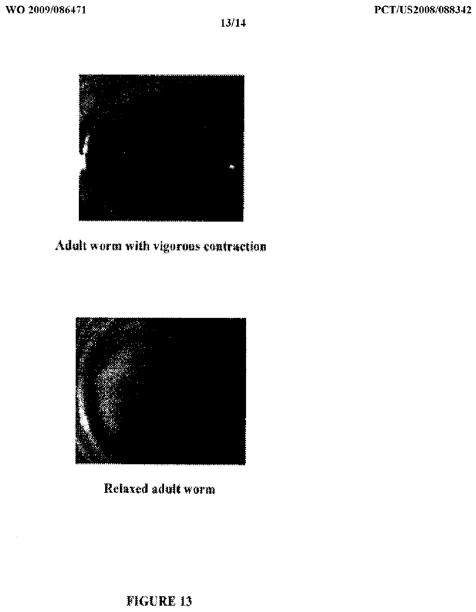

The experiment for each concentration was repeated for 5 replicates, each with 5 adult worms (i.e. a total of 25 adult worms of both sexes for each concentration). The following was observed for both groups of tested agents (A or B) regardless of concentration: once the tested agent came into contact with the adult worms, the worms showed vigorous contractions of their whole bodies (see the upper photo in FIG. 13) followed by relaxations (see the lower photo in FIG. 13). These strong contractions slowly diminished until no movement was observed. When the tested worms were stimulated with a needle, they showed no response i.e. no movement. When the worms were transferred to another dish containing hot water, they showed strong contraction movement. This showed that both tested agents (A or B) have a relaxant effect regardless of concentration under *in vitro* conditions according to Ismail (1979). They only differ from each other according to the mean time to show this effect.

It is worth noting that the damage caused by the adult worms seems largely related to their size. The large and muscular adult worms do not attach to the intestinal wall but maintain their position by constant movement. They occasionally force their way into extra intestinal sites or if present in large numbers form tangled masses that occlude the bowel as reported by Markell et al., 1999. This fact can be used to explain the importance of the relaxant effect of the tested agents A or B to expel the worms out of the intestine, if given the test agent then followed by giving a suitable purgative.

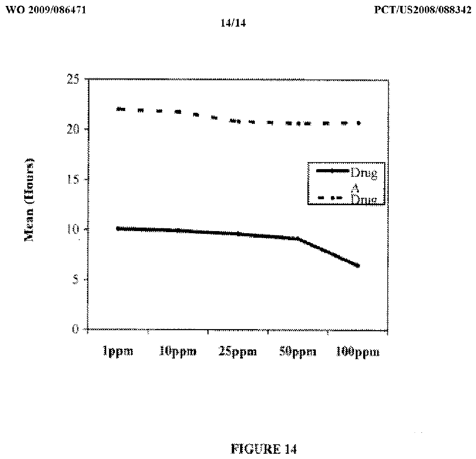

The following table and the graph shown in FIG. 14 show the mean time to cause the relaxing effect of the test agents (A or B) on the adult worms of *Ascaris suum* at different agent concentrations.

**Table 7: Efficacy of the test agents A and B on the adult worms of Ascaris suum**

| Drug | Concentration (ppm) | Time in H ours | | | | Scheffe multiple comparison for cone. Significantly different from |
|---|---|---|---|---|---|---|
| | | Mean | SD | Min | Max | |
| A | 1 | 10.07 | 0.06 | 10.00 | 10.17 | 10,25,50,100 |
| | 10 | 9.89 | 0.15 | 9.70 | 10.02 | 25,50,100 |
| | 25 | 9.57 | 0.11 | 9.42 | 9.67 | 50,100 |
| | 50 | 9.12 | 0.11 | 9.00 | 9.23 | 100 |
| | 100 | 6.47 | 0.12 | 6.30 | 6.62 | |
| Total | | 9.02 | 1.35 | 6.30 | 10.17 | |
| B | 1 | 22.02 | .55 | 21.17 | 22.67 | 25,50,100 |
| | 10 | 21.76 | .41 | 21.23 | 22.17 | 50,100 |
| | 25 | 20.80 | .45 | 20.25 | 21.33 | |
| | 50 | 20.66 | .63 | 20.05 | 21.50 | |
| | 100 | 20.74 | .23 | 20.50 | 21.02 | |
| Total | | 21.20 | .73 | 20.05 | 22.67 | |
| | F(Drug) | 15428.94* | | | | |
| | (Cone.) | 77.16* | | | | |
| | F(Drug*Conc) | 30.38* | | | | |
| | | | | | | |

| | | | | | | |
|---|---|---|---|---|---|---|
| *P<0.01 | | | | | | |

The table shows that the overall mean time to show the relaxing effect on the adult worms of *A. suum* with test agent B is significantly longer (21.20 + 0.73 hours) than with test agent A (9.02 + 1.35 hours).

As regards concentration, the mean time to show this effect was significantly decreasing by increasing concentration with significant difference between the test agents in each concentration. Multiple comparisons among means showed that with test agent A each concentration had shorter time to bring relaxation than the preceding concentration while with test agent B no significant change was gained after 25 ppm. A significant interaction effect of test agents and concentration was revealed which indicated that increase dose of test agent A significantly decreased the time to bring the relaxing effect from 10.07 hours with 1 ppm to 6.47 hours with 100 ppm. On the other hand increase dose of test agent B showed minimal decrease of time to show the relaxing effect from 22.02 hours with 1 ppm to 20.74 hours with 100 ppm.

In sum, both tested agents (A and B), regardless of concentration, exhibited a relaxant effect on the adult worms of *Ascaris lumbricoides suum* under *in vitro* conditions, but they differ from each other according to the mean time to show this effect. The overall mean time to show the relaxing effect on the adult worms of *A. suum* with test agent B is significantly longer than with test agent A. A significant interaction effect of test agents and concentration was revealed which indicated that increase dose of test agent A significantly decreased the time to bring the relaxing effect from 10.07 hours with 1 ppm to 6.47 hours with 100 ppm. On the other hand, an increased dose of test agent B showed minimal decrease in the time required to produce the relaxing effect, from 22.02 hours with 1 ppm to 20.74 hours with 100 ppm. This result indicated that test agent A at 100 ppm is more potent, in that it causes a relaxing effect on the adult worms of *A. suum* in a short time of about 6 hours.

### EXAMPLE 18

The results of Examples 16 and 17 indicate that the test agents A and B had different modes of action on nematode parasites. Both agents had a lethal effect on the larvae of *Trichinella spiralis* under *in vitro* conditions, with test agent B exhibiting a shorter mean time to show its effect than test agent A, and both made the larvae non-viable and non-infective under *in vivo* testing. Both agents had a relaxing effect on the adult worms of *Ascaris lumbricoides suum* under *in vitro* conditions, with test agent A exhibiting a shorter mean time to show its effect than test agent B.

Based on these results, the efficacy of the test agent B at different concentrations on the treatment of *Trichinella spiralis* is assessed in experimentally infected mice. Female Swiss Albino mice, 8 weeks in age, from 18-22 gm in weight, which can be obtained from Theodore Bilharz Research Institute, Cairo, are infected with by T *spiralis* larvae (100 larvae/mouse). Each group consists of 15 mice.

For each test composition, three concentrations are tested. For each concentration nine groups of mice are studied. One group of *T spiralis-*infected mice receives the present standard treatment drug. Three groups of uninfected mice receive the test compound in the same schedule and concentration as the test drug groups. One group of uninfected and untreated mice and one group of *T. spiralis* infected mice that do not receive any treatment are maintained as controls.

Three different concentrations from each of the test compositions are determined after estimation of the LD50. The schedule for drug administration is as follows: (1) four days post-infection (PI); (2) one week PI; and seven weeks PI. All drugs are administered orally using a stomach tube.

For the parasitological studies, fecal egg counts are done for all infected groups twice weekly starting from the 5^{th}. week PI.

Mice are sacrificed 9 weeks PI. Perfusion of the portal system is done for the recovery of the worms. The total number, sex, maturation and distribution of the worms are determined. Four portions, two from the jejunum and two from the ileum, are taken from each mouse, washed with PBS, opened and compressed between two slides and examined microscopically for detection of the stage of maturation. 0.3 gram of the intestine are digested in 4% potassium hydroxide overnight, and *T. spiralis* larvae counted.

Due to the relaxant effect of the tested agents A or B on the adult worms of *Ascaris lumbricoides suum,* they will be useful in treating *Ascaris*-infected subjects so as to expel the worms out of the intestine of the infected hosts after giving a suitable purgative.

### EXAMPLE 19

An exemplary test composition is used, which comprises: 7% (vol/vol) linalool; 35% (vol/vol) thymol; 4% (vol/vol) α-pinene; 30% (vol/vol) *p*-cymene; and 24% (vol/vol) soy bean oil. Test doses are: 1 mg/kg Body Weight (BW), 10 mg/kg BW, 20 mg/kg BW, and 100 mg/kg BW.

Criteria of cure used for the experiments are: (1) exposure time and efficacious dose level to produce 100% kill of *H. nana* in a minimum of 80% of infected mice (e.g., cure = 0 viable worms in intestine and 0 viable eggs in stool). The short life cycle of *H. nana* can facilitate rapid prophylactic testing. *H. nana* has about a 14-day life cycle from egg infection until maturation and egg laying.

Several administration protocols are implemented to test the efficacy of the exemplary composition against infection. In a first protocol, an oral dose is administered to 5 groups of mice via gel capsule at 3 days prior to infection and daily until mice are sacrificed. In a second protocol, an oral dose is administered to 5 groups of mice via gel capsule at 3 weeks prior to infection and daily until mice are sacrificed. In a third protocol, an oral dose is administered to 5 groups of mice via gel capsule daily starting 3 weeks prior to infection, and treatment is discontinued after infection until mice are sacrificed. Control groups of mice in each of the protocols are dosed with soy bean oil only. Data from the three protocols using different mg/kg BW of the exemplary test composition are presented in Tables 8-12.

**TABLE 8**

| **Tested dose** | **Total number of animals** | **Number of animals carry worm** | | **% Cure** |
|---|---|---|---|---|
| | | **Positive** | **Negative** | |
| Control Infected only | 25 | 13 (52%) | 12 | |
| 20 mg/kg 3 wks stopped | 25 | 9 | 16 | 64.0% |
| Control Infected only 20 mg/kg 3 wks continued | 25 | 18(72%) | 7 | 76.0% |
| | 25 | 6 | 19 | |
| Control Infected only | 24 | 18(75%) | 6 | 87.8% |
| 20 mg/kg 3 days continued | 41 | 5 | 36 | |

**TABLE 9**

| | %Reduction in egg production in stool at day 14 | %Reduction in ova count/worm |
|---|---|---|
| Control Infected only | 0.0% | ND |
| 20 mg/kg 3 wks stopped | 76.39% | ND |
| Control Infected only | 0.0% | 0.0% |
| 20 mg/kg 3wks continued | 93.59% | (77.85%) |
| Control Infected only | 0.0% | 0.0% |
| 20 mg/kg 3 days continued | 68.44% | (40.58%) |

**TABLE 10**

| Groups | | %Reduction in egg production in stool |
|---|---|---|
| | Day 10 | Day 14 |
| Control Infected only | 0.0% | 0.0% |
| 10 mg/kg 3 days continued | 0.0% | 0.0% |
| Control* | 0.0% | 0.0% |
| 10 mg/kg 3wks continue | 100% | 79% |
| 1 Omg/kg 3wks stopped | 85% | 43% |

**TABLE 11**

| **Groups** | **%Cure** | **% Reduction in ova count/worm** |
|---|---|---|
| **Control Infected only** | **0.0%** | **ND** |
| **10 mg/kg 3days** | **52.0%** | **ND** |
| **continued Control** | **0.0%** | **0.0%** |
| **10 mg/kg 3wks continue** | **91.3%** | **95%** |
| **10mg/kg 3wks stopped** | **80%** | **91%** |

**TABLE 12**

| | | Infection status | | %reduction in egg production/gm stool/mouse | | | | |
|---|---|---|---|---|---|---|---|---|
| Treatmen t | N | +ve | -ye | day 10 post infection | day 14 post infection | Number of worms/ mouse | %reduction in Ova/worm | %Cure rate |
| Control | 2 3 | 12 | 11 | | | 5.72 ±12 | | |
| 10mg/kg 3wks continue | 2 3 | 2 | 21 | 100%** | 79% | 0.4 ±2.3 | 95% | 91.3% * |
| Control Infected only | 2 4 | 18 | 6 | | | 9.75 + 28.2 | | |
| 20 mg/kg 3 days continued | 4 1 | 5 | 36 | ND | 68.4 | 0.07 ± 0.35 | 40.6% | 87.8% * |

### EXAMPLE 20

An exemplary test composition is used, which comprises: 7% (vol/vol) linalool; 35% (vol/vol) thymol; 4% (vol/vol) α-pinene; 30% (vol/vol) *p*-cymene; and 24% (vol/vol) soy bean oil.

Test groups of mice are provided for infection and treatment, each containing about 20 mice (e.g., 5 test groups x 20 mice per test group = 100 mice). Animals are selected and examined to ensure they are worm-free. The following test groups are designated to be infected and to received the following treatment:
Group 1: soy bean oil carrier only;
Group 2: 1 mg/kg body weight (BW) composition;
Group 3:10 mg/kg BW composition;
Group 4: 20 mg/kg BW composition; and
Group 5: 100 mg/kg BW composition.

An additional control group that is not infected can be provided and administered the exemplary composition. Test groups of mice designated for infection are infected, for example with *H. nana.* About 150 viable eggs per mouse is determined to be useful for infecting mice such that test animal exposure to the parasite's infective stage is predictive of realistic environmental exposure.

An oral dose is administered via gel capsule to the test groups of mice at 2 days after egg shedding is observed. The oral dose is administered daily until mice are sacrificed. Half-life of doses of exemplary composition can be determined in mammalian blood to guide specification of prophylactic and therapeutic regiments.

### EXAMPLE 21

Resistance studies of exemplary compositions are conducted. An exemplary test composition is used, which comprises: 7% (vol/vol) linalool; 35% (vol/vol) thymol; 4% (vol/vol) α-pinene; 30% (vol/vol) *p*-cymene; and 24% (vol/vol)soy bean oil.

Test groups of mice are provided, each containing about 20 mice (e.g., 5 test groups x 20 mice per test group = 100 mice). Animals are selected and examined to ensure they are worm-free. The following test groups are designated to be infected and to received the following treatment:
Group 1: soy bean oil carrier only;
Group 2: 1 mg/kg body weight (BW) composition;
Group 3: 10 mg/kg BW composition;
Group 4: 20 mg/kg BW composition; and
Group 5: 100 mg/kg BW composition.

An additional control group that is not infected can be provided and administered the exemplary composition.

Test groups of mice designated for infection are infected, for example with H. nana. About 150 viable eggs per mouse is determined to be useful for infecting mice such that test animal exposure to the parasite's infective stage is predictive of realistic environmental exposure. Target DNA from the eggs used for the initial infection is sequenced prior to treatment with exemplary compositions, for use as a control sequence.

An oral dose is administered via gel capsule to the test groups of mice at 2 days after egg shedding observed. The oral dose is administered daily until the mice are sacrificed. The viable eggs are counted and collected. The collected viable eggs are used to re-infect the previously uninfected animal test group, which are then treated with the exemplary composition as before. The step is repeated, for a total of three counts and collections of viable eggs. Following the third count and collection of viable eggs, the viable egg target DNA is sequenced.

The parasite is assumed to have gone through three reproductive cycles. The control unexposed DNA sequence can be compared to the target DNA sequence obtained from eggs after the third cycle, having three successive exposures to the exemplary treatment compositions. Resistance is determined by considering: no change in exposed target DNA sequence vs. control target DNA sequence results in one or more amino acid changes.

### EXAMPLE 22

Safety studies are of exemplary compositions are conducted. Safety studies include acute toxicity tests (range finding), *in vitro* genetic toxicology studies, and sub-chronic rodent toxicity study (90-day) conducted under Good Laboratory Practices (GLP).

Animals are exposed to daily doses of the therapeutic compositions being tested. For example, an exemplary test composition can be used, which comprises: 7% (vol/vol) linalool; 35% (vol/vol) thymol; 4% (vol/vol) α-pinene; 30% (vol/vol) *p*-cymene; and 24% (vol/vol) soy bean oil. The following test groups are designated to receive the following treatment:
Group 1: soy bean oil carrier only;
Group 2: 0.07 g/kg body weight (BW) per day;
Group 3: 0.7g/kg BW per day; and
Group 4: 7g/kg BW per day.

All appropriate observational and clinical tests (including histopathology) are performed to assess any treatment-related effects. Safety measures (see Table 10) are made at 100X the efficacious dose using a prophylactic efficacy protocol. For example, if the efficacious dose is 10 mg/kg, the safety test dose is 1 g/kg.

**TABLE 13**

| **Safety Measures** | **Sample size (# of mice)** | **Key Metric** |
|---|---|---|
| changes in body weight | 20-40 | less than 11% body weight change, test vs. control |
| changes in water intake | 20-40 | less than 11% differential, test vs. control |
| changes in food intake | 20-40 | less than 11% differential, test vs. control |
| red blood cell count | 20-40 | no significant difference vs. control or within normal range |
| white blood cell count | 20-40 | no significant difference vs. control or within normal range |
| hemoglobin | 20-40 | no significant difference vs. control or within normal range |
| sGOT (liver function) | 20-40 | no significant difference vs. control or within normal range |
| sGPT (liver function) | 20-40 | no significant difference vs. control or within normal range |
| creatinine | 20-40 | no significant difference vs. control or within normal range |
| fecal matter consistency | 20-40 | no significant difference vs. control or within normal range |

Relative palatability of exemplary compositions is also tested. Synergistic combinations of compounds can be designed to favor compounds with preferred palatability.

### EXAMPLE 23

A receptor gene encoding the Tyramine receptor (TyrR) has been isolated from the American cockroach, fruit fly, mosquito, and other organisms. The present subject matter provides methods of utilizing the TyrR protein expressed in cells to screen for compounds useful for treating parasitic infections.

In the present Example, the genes encoding TyrR were incorporated into model cells in culture that mimic receptors in insects. The screening process uses the cultured cells in combination with [Ca²]i and [cAMP]i measuring assays to quantitatively determine effectiveness of test compound to treat parasitic infections. The screening process allows for identification of compounds that produce highly efficacious anti-parasitic compositions.

The assay steps are as follows. A cell expressing a tyramine receptor is contacted with a test compound and the receptor binding affinity of the test compound is measured. Cells which can be used include, for example, HEK293 cells, COS cells, Drosophila Schneider or S2 cells, SF9, SF21, T.ni cells, or the like. cAMP and/or Ca²⁺ levels within the cell are also monitored and any changes from contacting the test compound with the cell are noted for each compound tested. A test compound is identified as a potential therapeutic compound if it exhibits a high receptor binding affinity for the tyramine receptor as well as an ability to effect change in cAMP and/or Ca²⁺levels within the cell. A test compound is also identified as a potential therapeutic compound if it exhibits a low receptor binding affinity for the tyramine receptor as well as an ability to effect change in cAMP and/or Ca levels within the cell. A composition for use in treating a parasitic formulation can then be selected that includes a plurality of the identified compounds. In particular, the composition can comprise at least one compound identified as having a high receptor binding affinity for the tyramine receptor as well as an ability to effect change in cAMP and/or Ca²⁺ levels within the cell and at least one additional compound identified as having a low receptor binding affinity for the tyramine receptor as well as an ability to effect change in cAMP and/or Ca²⁺ levels within the cell.

Table 14 lists compounds tested with the present screening method and the determined capacity of each compound to bind the tyramine receptor, affect intracellular Ca²⁺ and affect intracellular cAMP. These results can then be utilized to select a composition comprising two or more of the tested compounds with desirable characteristics. For example, *p-*cymene and linalool can be select to include in a composition for treating parasitic infections according to the screening method criteria since *p*-cymene exhibits low tyramine receptor binding affinity, linalool exhibits high tyramine receptor binding affinity, and both compounds effect change in cAMP and/or Ca2+ levels. Similarly, *p*-cymene and thymol can be select to include in a composition for treating parasitic infections according to the screening method criteria since *p*-cymene exhibits low tyramine receptor binding affinity, thymol exhibits high tyramine receptor binding affinity, and both compounds effect change in cAMP and/or Ca2+ levels. Further, compositions for treating parasitic infections can be formulated that include more than two compounds, such as for example a composition that includes α-pinene, *p*-cymene, linalool, thymol, and soybean oil. It can be preferable to formulate a composition that displays an anti-parasitic effect exceeding the anti-parasitic effect of any of the compounds when used alone.

**TABLE 14**

| **Compound** | **Tyramine Receptor Binding Affinity (High or Low)** | **Affects Intracellular Ca²⁺ (Yes or No)** | **Affects Intracellular cAMP (Yes or No)** |
|---|---|---|---|
| α-pinene | Low | No | No |
| anethole | Low | Yes | Yes |
| benzyl alcohol | Low | No | Yes |
| black seed oil | High | Yes | Yes |
| cedar oil | Low | Yes | Yes |
| cineol | Low | Yes | Yes |
| cinnamon oil | Low | No | No |
| cinnamyl alcohol | Low | Yes | No |
| citronella oil | Low | No | Yes |
| clove oil | Low | Yes | Yes |
| *p*-cymene | Low | Yes | Yes |
| d-limonene | High | Yes | Yes |
| Eugenol | Low | Yes | No |
| garlic oil | Low | Yes | Yes |
| lemon oil | Low | No | No |
| lemongrass oil | Low | No | No |
| lilac flower oil | High | Yes | Yes |
| lime oil | Low | Yes | Yes |
| d-limonene | Low | Yes | No |
| linalool | High | Yes | No |
| linseed oil | Low | No | No |
| oil of pennyroyal | Low | Yes | Yes |
| orange sweet oil | Low | Yes | No |
| peppermint oil | Low | No | Yes |
| phenethyl proprionate | Low | No | Yes |
| pine oil | Low | No | No |
| rosemary oil | Low | No | No |
| sodium lauryl sulfate | Low | No | No |
| soybean oil | Low | No | No |
| thyme oil | High | Yes | Yes |
| thymol | High | Yes | No |
| vanillin | Low | Yes | No |
| white mineral oil | Low | Yes | Yes |
| geraniol | High | Yes | Yes |
| tetrahydrolinalool | High | Yes | Yes |

### EXAMPLE 24

HEK293 cells are transfected with the pcDNA3.1 /V5 -HisA vector using Lipofectamine (Invitrogen). The vector contains a full-length construct of the C. *elegans* tyramine receptor. 48 h after transfection cells are selected in a culture medium containing 0.5 mg/ml G418 (Invitrogen). Cells that survive from the first round of G418 selection are further subjected to limiting dilution for single clone selection. Clones are selected and then cell stocks are grown for assay purposes.

Growth media is replaced with serum free media (i.e., Eagle's minimum essential medium (EMEM) buffered with 10 mMHEPES (N-2-Hydroxyethylpiperazine-N'-2-ethanesulfonic acid)) 24 hours after plating of the cells.

Linalool is used as the receptor activator for the assay, and is added to each well on each plate. Sufficient linalool is added to ensure receptor activation and a resulting increase in intracellular Ca²⁺ levels.

Essential oil test compounds of varying concentrations are added to the wells of each of the four plates (four plates are used per replicate). The assay is conducted at room temperature.

At time points of 30 seconds, 60 seconds, 90 seconds, 120 seconds, 180 seconds, 240 seconds, 300 seconds, and 600 seconds post-addition of test compound, the assay is terminated and the cells are analyzed to determine intracellular Ca²⁺ levels.

### EXAMPLE 25

HEK293 cells are transfected with the pcDNA3.1 /V5 -HisA vector using Lipofectamine (Invitrogen). The vector contains a full-length construct of the C. *elegans* tyramine receptor. 48 h after transfection cells are selected in a culture medium containing 0.5 mg/ml G418 (Invitrogen). Cells that survive from the first round of G418 selection are further subjected to limiting dilution for single clone selection. Clones are selected and then cell stocks are grown for assay purposes.

Growth media is replaced with serum free media (i.e., Eagle's minimum essential medium (EMEM) buffered with 10 mMHEPES (N-2-Hydroxyethylpiperazine-N'-2-ethanesulfonic acid)) 24 hours after plating of the cells.

Linalool is used as the receptor activator for the assay, and is added to each well on each plate. The amount of linalool added is less-than that required to ensure receptor activation and a resulting increase in intracellular Ca²⁺ levels.

Essential oil test compounds of varying concentrations are added to the wells of each of the four plates (four plates are used per replicate). The assay is conducted at room temperature.

At time points of 30 seconds, 60 seconds, 90 seconds, 120 seconds, 180 seconds, 240 seconds, 300 seconds, and 600 seconds post-addition of test compound, the assay is terminated and the cells are analyzed to determine intracellular Ca²⁺ levels.

### EXAMPLE 26

HEK293 cells are transfected with the pcDNA3.1 /V5 -HisA vector using Lipofectamine (Invitrogen). The vector contains a full-length construct of the *C*. *elegans* tyramine receptor as well as an arrestin-GFP conjugate. For transient transfection, cells are harvested 48 h after transfection. For stable transfection, 48 h after transfection cells are selected in a culture medium containing 0.5 mg/ml G418 (Invitrogen). Cells that survive from the first round of G418 selection are further subjected to limiting dilution for single clone selection. Clones are selected and then cell stocks are grown for assay purposes.

Growth media is replaced with serum free media (i.e., Eagle's minimum essential medium (EMEM) buffered with 10 mMHEPES (N-2-Hydroxyethylpiperazine-N'-2-ethanesulfonic acid)) 24 hours after plating of the cells. Per replicate, two plates are incubated for 10 minutes at room temperature and atmospheric CO2 and two plates are incubated for 10 minutes at 37C and 5% CO₂.

Each test compound is solvated using 100% dimethyl sulfoxide (DMSO). Multiple solutions of each compound are prepared at varying concentrations for testing in separate wells of each plate. The solutions are sonicated to increase solubility.

Each of the solutions of varying concentrations of the fifteen compounds is added to a well on each of the four plates (four plates are used per replicate). Two plates per replicate are incubated for 30 minutes at room temperature and atmospheric CO₂. The other two plates per replicate are incubated for 30 minutes at 37C and 5% CO₂.

Agonist is then added to each well. For each compound to be tested, 100 nM isoproterenol (0.4% weight/volume ascorbic acid) is added to one of the 37C plates and one of the RT plates. 100 nM arginine vasopressin is added to one of the 37C plates and one of the RT plates.

The assay is terminated using 1% paraformaldehyde containing 1 uM DRAQ5 DNA probe to fix the cells. The cells are analyzed using a line scanning, confocal imaging system to quantitate the localization of the arrestin-GFP conjugate for the cells in each well using the Amersham Biosciences granularity analysis GRNO algorithm. This algorithm finds the nucleus of cells and then dilates out a specified distance in which fluorescent spots of arrestin-GFP localization are identified based on size and fluorescent intensity. The average of the fluorescent intensity of the identified grains per cell in an acquired image is determined for each well on the plates.

Control wells are used on each plate to determine the basal level of fluorescent spots for the cells on the different plates as well as to determine the maximally stimulated level of fluorescent spots for the cells on the different plates. The cells in the control wells are subjected to the method described above, but no test compound or agonist is added to the wells. The cells in the "agonist" control wells are subjected to the method described above, including the addition of agonist, but no test compound is added to the wells.

Formulations in accordance with embodiments of the present disclosure are also useful as repellants against other biting anthropod vectors such as sand flies, mosquitoes and bugs that transmit deadly infections in both human and animals. Experimental hosts such as mice (for bugs) dogs (for sand flies) and human (mosquitoes) are well known in the art. Such host animals are treated with the formulations of the present disclosure and the ability of the arthropod vectors to feed on the host are evaluated. Appropriate dosages of the formulations are readily determined by methods such as those described above well known in the art.

## Claims

1. An antiparasitic composition comprising Para-Cymene, Thymol, α-Pinene and Linalool for use in treatment of a disease caused by a parasite selected from the group consisting of: Schistosoma mansoni, Opisthorchis sinensis, Cryptosporidium parvum, Trichinella, Enterobius vermicularis, Ascaris suum and Diplyidium caninum.

2. The antiparasitic composition for use according to claim 1, wherein said Para-Cymene is in the range of 29.5-30.5% by volume, Thymol (crystal) in the range of 34-36% by volume, α-Pinene in the range of 1-5% by volume and Linalool (Coeur) in the range of 5-10% by volume.

3. The antiparasitic composition for use according to any of the preceding claims, wherein said α-Pinene is in the range of 3.5-4.5% by volume.

4. The antiparasitic composition for use according to any of the preceding claims, wherein said Linalool (Coeur) is in the range of 6.5-7.5% by volume.

5. The antiparasitic composition for use according to any of the preceding claims, further comprising soy bean oil.

## Patentansprüche

1. Antiparasitäre Zusammensetzung, umfassend Para-Cymol, Thymol, α-Pinen und Linalool zur Verwendung bei der Behandlung einer Krankheit, die durch einen Parasiten verursacht wird, ausgewählt aus der Gruppe bestehend aus: Schistosoma mansoni, Opisthorchis sinensis, Cryptosporidium parvum, Trichinella, Enterobius vermicularis, Ascaris suum und Diplyidium caninum.

2. Antiparasitäre Zusammensetzung zur Verwendung nach Anspruch 1, wobei das Para-Cymol im Bereich von 29,5-30,5 Vol.-%, das Thymol (Kristall) im Bereich von 34-36 Vol.-%, das α-Pinen im Bereich von 1-5 Vol.-% und das Linalool (Coeur) im Bereich von 5-10 Vol.-% liegt.

3. Antiparasitäre Zusammensetzung zur Verwendung nach einem der vorhergehenden Ansprüche, wobei das α-Pinen im Bereich von 3,5-4,5 Vol.-% liegt.

4. Antiparasitäre Zusammensetzung zur Verwendung nach einem der vorhergehenden Ansprüche, wobei das Linalool (Coeur) im Bereich von 6,5-7,5 Vol.-% liegt.

5. Antiparasitäre Zusammensetzung zur Verwendung nach einem der vorhergehenden Ansprüche, ferner Sojabohnenöl umfassend.

## Revendications

1. Composition antiparasitaire comprenant du para-cymène, du thymol, de l'a-pinène et du linalol pour une utilisation dans le traitement d'une maladie causée par un parasite choisi dans le groupe constitué de : Schistosoma mansoni, Opisthorchis sinensis, Cryptosporidium parvum, Trichinella, Enterobius vermicularis, Ascaris suum et Diplyidium caninum.

2. Composition antiparasitaire pour une utilisation selon la revendication 1, dans laquelle ledit para-cymène est dans une plage de 29,5 à 30,5 % en volume, le thymol (cristal) dans une plage de 34 à 36 % en volume, l'a-pinène dans une plage de 1 à 5 % en volume et le linalol (Cœur) dans une plage de 5 à 10 % en volume.

3. Composition antiparasitaire pour une utilisation selon l'une quelconque des revendications précédentes, dans laquelle ledit α-pinène est dans une plage de 3,5 à 4,5 % en volume.

4. Composition antiparasitaire pour une utilisation selon l'une quelconque des revendications précédentes, dans laquelle ledit linalol (Cœur) est dans une plage de 6,5 à 7,5 % en volume.

5. Composition antiparasitaire pour une utilisation selon l'une quelconque des revendications précédentes, comprenant en outre de l'huile de soja.
